(19) Europäisches Patentamt
European Patent Office
Office européen des brevets

(11) EP 3 235 945 B1

(12) **EUROPEAN PATENT SPECIFICATION**

(45) Date of publication and mention of the grant of the patent:
**14.04.2021 Bulletin 2021/15**

(21) Application number: **15869998.3**

(22) Date of filing: **16.12.2015**

(51) Int Cl.:
***D06M 15/647*** *(2006.01)* ***D06M 13/02*** *(2006.01)*
***D06M 13/17*** *(2006.01)* ***D06M 13/184*** *(2006.01)*
***D06M 13/224*** *(2006.01)* ***D06M 13/292*** *(2006.01)*
***D06M 13/513*** *(2006.01)* ***D06M 15/53*** *(2006.01)*
***A61L 15/16*** *(2006.01)* ***A61F 13/15*** *(2006.01)*

(86) International application number:
**PCT/JP2015/085156**

(87) International publication number:
**WO 2016/098796 (23.06.2016 Gazette 2016/25)**

(54) **LIQUID FILM CLEAVAGE AGENT**

FLÜSSIGKEITSFILMSPALTUNGSMITTEL

AGENT DE CLIVAGE DE FILM LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2014 JP 2014255639**

(43) Date of publication of application:
**25.10.2017 Bulletin 2017/43**

(73) Proprietor: **Kao Corporation**
**Chuo-Ku**
**Tokyo 103-8210 (JP)**

(72) Inventors:
- **KABAYA, Yoshiaki**
  **Tochigi 321-3497 (JP)**
- **TANEICHI, Shoichi**
  **Tochigi 321-3497 (JP)**
- **YASUDA, Michio**
  **Tochigi 321-3497 (JP)**
- **SANGAWA, Yuta**
  **Tochigi 321-3497 (JP)**

(74) Representative: **Vossius & Partner**
**Patentanwälte Rechtsanwälte mbB**
**Siebertstrasse 3**
**81675 München (DE)**

(56) References cited:
**WO-A1-2012/086476**
**WO-A1-2012/086476**
**WO-A1-2012/133724**
**WO-A1-2013/065794**
**WO-A1-2013/088969**
**WO-A1-2014/050310**
**WO-A1-2014/050310**
**WO-A1-2014/171388**
**JP-A- H0 482 961**
**JP-A- H1 053 955**
**JP-A- H1 053 955**
**JP-A- H01 148 879**
**JP-A- H09 215 706**
**JP-A- H09 215 706**
**JP-A- 2006 175 689**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to a liquid film cleavage agent, a nonwoven fabric containing the liquid film cleavage agent, an absorbent article containing a nonwoven fabric containing the liquid film cleavage agent, and a method of producing a nonwoven fabric containing the liquid film cleavage agent.

BACKGROUND OF THE INVENTION

Proposals have been recently made on improving liquid-permeation function or liquid-absorption function of a nonwoven fabric.
For example, Patent Literature 1 describes a nonwoven fabric in which absorption time of water drops dropped from a predetermined height is adjusted to be in a predetermined range for the purpose of reducing liquid backflow in an absorbent article, in which a hydrophilic treatment agent such as polyoxyalkylene group-modified polysiloxane is used in order to achieve this absorption time.
Patent Literature 2 describes an art in which a polyoxypropylene glycol-based compound serving as a blood modifier is incorporated into a topsheet for the purpose of reducing residual liquid in an absorbent article, in which the blood modifier used is a moderate material whose affinity with menstrual blood is suppressed in comparison with a surfactant.
Moreover, Patent Literature 3 similarly describes as a topsheet a material formed of two layers having convex portions on a skin contact surface side, in which the convex portions are coated with a blood lubricant that has lower affinity with menstrual blood than a surfactant.
Patent Literature 4, 5 and 6 describe various surfactants which are soluble to some extent in water.

CITATION LIST

Patent Literatures

Patent Literature 1: JP-A-2004-256935 ("JP-A" means unexamined published Japanese patent application)
Patent Literature 2: JP-A-2013-179969
Patent Literature 3: JP-A-2014-68942
Patent Literature 4: JP-H01 148879 A
Patent Literature 5: JP-H09 215706 A
Patent Literature 6: JP-H10 53955 A

SUMMARY OF THE INVENTION

The present invention provides a liquid film cleavage agent having a spreading coefficient of 15 mN/m or more to a liquid having surface tension of 50 mN/m, and a water solubility of 0 g or more and 0.025 g or less; and a nonwoven fabric containing the liquid film cleavage agent.
The present invention also provides a liquid film cleavage agent having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m; and a nonwoven fabric containing the liquid film cleavage agent.
Other and further objects, features and advantages of the invention will appear more fully from the following description, appropriately referring to the accompanying drawings.

BRIEF DESCRIPTION OF DRAWINGS

{FIG. 1}
FIG. 1 is a schematic diagram showing a liquid film formed in an interstice between fibres of a nonwoven fabric.
{FIG. 2}
FIGs. 2(A1) to 2(A4) each are an explanatory drawing schematically showing, from a side, a state in which a liquid

film cleavage agent according to the present invention cleaves a liquid film, and FIGs. 2(B1) to 2(B4) each are an explanatory drawing schematically showing, from above, a state in which the liquid film cleavage agent according to the present invention cleaves the liquid film.

{FIG. 3}

FIG. 3 is a cross-sectional view of a nonwoven fabric, showing a preferable aspect (first aspect) of the nonwoven fabric according to the present invention.

{FIG. 4}

FIG. 4 is a perspective view schematically showing another preferable aspect (second aspect) of a nonwoven fabric by partially cutting surfaces according to the present invention.

{FIG. 5}

FIG. 5 is a perspective view schematically showing a further another preferable aspect (third aspect) of a nonwoven fabric by partially cutting surfaces according to the present invention, in which FIG. 5(A) shows a nonwoven fabric formed of one layer, and FIG. 5(B) shows a nonwoven fabric formed of two layers.

{FIG. 6}

FIG. 6 is a perspective view schematically showing another preferable aspect (fourth aspect) of a nonwoven fabric according to the present invention.

{FIG. 7}

FIG. 7 is a perspective view showing a modified example of the nonwoven fabric shown in FIG. 6.

{FIG. 8}

FIG. 8 is a perspective view schematically showing another preferable aspect (fifth aspect) of a nonwoven fabric according to the present invention.

{FIG. 9}

FIG. 9 is an explanatory drawing schematically showing a state in which constituent fibres of the nonwoven fabric shown in FIG. 18 are fixed with each other in thermally fusion bonded portions.

{FIG. 10}

FIG. 10 is a perspective view schematically showing another preferable aspect (sixth aspect) of a nonwoven fabric according to the present invention.

{FIG. 11}

FIG. 11 is a perspective view schematically showing another preferable aspect (seventh aspect) of a nonwoven fabric according to the present invention.

{FIG. 12}

FIG. 12 is a photograph substituted for drawing, showing a state in which a liquid film cleavage agent is localised in fibres of the nonwoven fabric sample in Example 1.

{FIG. 13}

FIG. 13 is a photograph substituted for drawing, showing a state in which a liquid film cleavage agent is localised in fibres of the nonwoven fabric sample in Example 29.

{FIG. 14}

FIG. 14 is a photograph substituted for drawing, showing a state in which a liquid film cleavage agent is localised in fibres of the nonwoven fabric sample in Example 30.

DESCRIPTION OF EMBODIMENTS

The present invention relates to a liquid film cleavage agent with which liquid films formed between fibres of a nonwoven fabric are reduced to realise a dry feeling at a higher level. Moreover, the present invention relates to a nonwoven fabric containing the liquid film cleavage agent preferable for an absorbent article which is excellent in dry feeling and also reduces concern about leakage, an absorbent article employing the nonwoven fabric, and a method for producing the nonwoven fabric containing the liquid film cleavage agent.

The nonwoven fabric according to the present invention contains a liquid film cleavage agent. "Liquid film cleavage agent" in the present invention means an agent which inhibits formation of liquid film by cleaving liquid film formed between fibres or on a surface of the fibres of the nonwoven fabric when a liquid, for example, an excreted liquid such as a high viscosity liquid including menstrual blood or urine comes into contact with the nonwoven fabric. Liquid film cleavage is achieved by effect of the liquid film cleavage agent which thrusts away part of a layer of the liquid film to destabilise the part. It is facilitated by this effect of the liquid film cleavage agent that liquid passes through the nonwoven fabric without retaining in a narrow interfibre region of the nonwoven fabric. That is, a nonwoven fabric excellent in liquid permeability is formed. Thus, even if the fibres which constitute the nonwoven fabric are narrowed to reduce the interfibre distance, both softness of texture and suppression of residual liquid are achieved. Such a nonwoven fabric can be used, for example, in the form of a topsheet of an absorbent article such as a sanitary towel, a baby nappy or an adult nappy.

Improvement in dry feeling has been indicated so far by nonwoven fabrics that use the treatment agent disclosed in Patent Literature 1, the blood modifier disclosed in Patent Literature 2 or the blood lubricant disclosed in Patent Literature 3. In particular, the blood modifier or the blood lubricant contributes to blood viscosity or lubricity, thereby reduces amount of liquid remaining in the topsheet formed of the nonwoven fabric. However, narrow interfibre regions exist in all nonwoven fabrics, and high interfibre meniscus force, high surface activity by plasma protein or high surface viscosity of the blood causes formation of a stable liquid film between the fibres resulting retention of the liquid in the region. Therefore, wetting is sensed on touch, and dry feeling has not been achievable even in a nonwoven fabric in which the conventional blood modifier or the blood lubricant has so far been applied. Further, in addition to the dry feeling, consumers have also recently shown a desire for good texture, which requires use of fine fibres. However, if fine fibres are used, the interfibre distance is further narrowed. Thus, because interfibre liquid film is even more easily formed and harder to cleave, liquid retention tends to increase.

Moreover, such a phenomenon is not limited to blood as the liquid targeted for absorption, and phospholipid also exhibits surface activity in urine by which liquid film is formed in a manner similar to that described above, so that dry feeling has not yet been satisfied.

Thus, an art for eliminating liquid film formed in a narrow interfibre part in a nonwoven fabric has been sought, but elimination has been difficult owing to the high stability of liquid film. Moreover, it is also conceivable to eliminate liquid film by applying a water-soluble surfactant to reduce liquid surface tension. However, if an attempt is made to enable removal of liquid film by using such a surfactant in the absorbent article, the liquid is liable to permeate through a liquid leak-proof backsheet.

To solve the above-described problem, formation of liquid film between the fibres of nonwoven fabric or the like is reduced by incorporating the liquid film cleavage agent according to the present invention into the nonwoven fabric to realise a higher level of dry feeling. Moreover, the nonwoven fabric containing the liquid film cleavage agent according to the present invention is excellent in dry feeling, and if this nonwoven fabric is used, an absorbent article that relieves concern about leakage can be provided.

The liquid film cleavage agent is applied on constituent fibres in at least some regions of the nonwoven fabric, and contained. At least some regions on which the agent is coated are preferably regions where a particularly large amount of liquid is received. For example, when the nonwoven fabric according to the present invention is processed into the topsheet of an absorbent article such as a sanitary towel, the coated region is one corresponding to a wearer's excretion region where an excreted liquid such as menstrual blood is received. Moreover, with regard to thickness direction of the nonwoven fabric according to the present invention, the cleavage agent is preferably contained at least into a surface on a liquid receiving side. In the topsheet of the above-described example, the liquid film cleavage agent is contained at least into a place on the skin-contact surface side in contact with the wearer's skin.

As termed with respect to the present invention, the expression "the nonwoven fabric contains or has the liquid film cleavage agent" means that the liquid film cleavage agent is mainly attached to the surface of the fibres. However, insofar as the liquid film cleavage agent remains present on the surface of the fibres, it is also acceptable for the liquid film cleavage agent to be present inside the fibres, or to be present inside the fibres by internally incorporating. As a method of attaching the liquid film cleavage agent to the surface of the fibres, any of various commonly utilised methods can be adopted without particular restriction. Specific examples thereof include coating by a sprayer, coating by a slot coater, coating by roll transfer, and immersion. Such treatment may be applied to the fibres before being formed into a web, or may be applied after the fibres are formed into a web by any of various methods. The fibres on the surface to which the liquid film cleavage agent is attached are dried, for example, by a dryer of a hot air blowing type at a temperature sufficiently lower than melting point of a fibre resin (for example, 120°C or lower). Moreover, when the liquid film cleavage agent is attached to the fibres using the above-described attaching method, the attachment is performed by using a solution containing the liquid film cleavage agent prepared by dissolving the liquid film cleavage agent into a solvent when necessary, or an emulsified liquid or a dispersion liquid of the liquid film cleavage agent.

In order for the liquid film cleavage agent according to the present invention to exhibit the liquid film cleavage effect explained later in the nonwoven fabric, the liquid film cleavage agent is required to be present in a fluid state when the liquid film cleavage agent touches a bodily fluid. Owing to this, melting point of the liquid film cleavage agent according to the present invention is preferably 40°C or lower, and further preferably 35°C or lower. Further, the melting point of the liquid film cleavage agent according to the present invention is preferably - 220°C or higher, and further preferably -180°C or higher.

Hereinafter, preferable embodiments of the liquid film cleavage agent and the nonwoven fabric containing the liquid film cleavage agent according to the present invention will be described.

In a liquid film cleavage agent in a first embodiment, a spreading coefficient thereof to a liquid having surface tension of 50 mN/m is 15 mN/m or more and a water solubility thereof is 0 g or more and 0.025 g or less. A nonwoven fabric in the first embodiment contains the liquid film cleavage agent.

The term "spreading coefficient to a liquid having surface tension of 50 mN/m" of the liquid film cleavage agent means the spreading coefficient in the case where the excreted liquid such as the menstrual blood and urine as described

above is assumed. The "spreading coefficient" means a value to be determined, based on Expression (1) and from a measured value obtained by the measuring method mentioned later in an environmental region of a temperature of 25°C and a relative humidity (RH) of 65%. Further, the liquid film in Expression (1) means a liquid phase of the the "liquid having surface tension of 50 mN/m," including both of the liquid in a state in which the film is formed between the fibres or on the surface of the fibres, and the liquid in a state before the film is formed, which is also referred to only as the liquid. Moreover, the surface tension in Expression (1) means interfacial tension on an interface of the liquid film and the liquid film cleavage agent respectively to a gas phase, and is differentiated from interfacial tension of the liquid film cleavage agent to the liquid film between liquid phases. The same rule of this differentiation also applies to other descriptions herein.

$$S = \gamma_w - \gamma_o - \gamma_{wo} \qquad (1)$$

$\gamma_w$: surface tension of a liquid film (liquid).
$\gamma_o$: surface tension of a liquid film cleavage agent.
$\gamma_{wo}$: interfacial tension of a liquid film cleavage agent to a liquid film.

As is known from Expression (1), the spreading coefficient (S) of the liquid film cleavage agent is increased as the surface tension ($\gamma_o$) of the liquid film cleavage agent is reduced, and as the interfacial tension ($\gamma_{wo}$) of the liquid film cleavage agent to the liquid film is reduced. When the spreading coefficient is 15 mN/m or more, the liquid film cleavage agent has high mobility, namely, high diffusivity, on the surface of the liquid film formed in a narrow interfibre region. From this viewpoint, the spreading coefficient of the liquid film cleavage agent is preferably 20 mN/m or more, more preferably 25 mN/m or more, and furhter preferably 30 mN/m or more. On the other hand, an upper limit thereof is not particularly limited, but from Expression (1), the surface tension of the liquid which forms the liquid film serves as the upper limit of the spreading coefficient of the liquid film cleavage agent, in such a manner that a value of the upper limit is 50 mN/m when a liquid having surface tension of 50 mN/m is used, a value of the upper limit is 60 mN/m when a liquid having surface tension of 60 mN/m is used, and a value of the upper limit is 70 mN/m when a liquid having surface tension of 70 mN/m is used. Therefore, from a viewpoint of using the liquid having surface tension of 50 mN/m in the present invention, the upper limit is 50 mN/m or less.

The term "water solubility" of the liquid film cleavage agent means mass of the liquid film cleavage agent which is dissolvable in 100 g of deionised water, and is a value to be measured in an environmental range of a temperature of 25°C and a relative humidity (RH) of 65% based on the measuring method described later. When this water solubility is 0 g or more and 0.025 g or less, the liquid film cleavage agent is hard to dissolve and forms the interface with the liquid film to make the above-described diffusivity more effective. From a similar viewpoint, the water solubility of the liquid film cleavage agent is preferably 0.0025 g or less, more preferably 0.0017 g or less, and further preferably less than 0.0001 g. Moreover, the water solubility is preferably smaller, and is 0 g or more, and from a viewpoint of the diffusivity on the liquid film, the water solubility is practically adjusted to $1.0 \times 10^{-9}$ g or more. Further, the water solubility is considered to be applied also to menstrual blood or urine which contains water as a main component.

The surface tension ($\gamma_w$) of the liquid film (a liquid having surface tension of 50 mN/m), the surface tension ($\gamma_o$) of the liquid film cleavage agent, the interfacial tension ($\gamma_{wo}$) of the liquid film cleavage agent to the liquid film, and the water solubility of the liquid film cleavage agent are measured by the following methods.

In addition, when a measurement object nonwoven fabric is a member (for example, the topsheet) assembled in the absorbent article such as a sanitary towel and a disposable nappy, the nonwoven fabric is taken as described below and measured. That is, an adhesive used for bonding between a measurement object member and other members in the absorbent article is weakened by a cooling means such as a cold spray, and then the measurement object member is carefully peeled off and obtained. This removal method is applied in measurement related to the nonwoven fabric according to the present invention, such as the measurement of an interfibre distance and fineness to be mentioned later.

Moreover, when the liquid film cleavage agent attached to the fibres is measured, the fibres to which the liquid film cleavage agent is attached are first washed with a washing liquid such as hexane, methanol and ethanol, then a solvent (washing solvent containing the liquid film cleavage agent) used for the washing is dried to isolate the liquid film cleavage agent. Mass of the isolated substance at this time is applied upon calculating a content proportion (OPU) of the liquid film cleavage agent to fibre mass. When an amount of the isolated substance is not enough for the measurement of the surface tension or the interfacial tension, a suitable column and a suitable solvent are selected according to components of the isolated substance, and then each component is fractionated by high performance liquid chromatography, and MS measurement, NMR spectroscopy or elementary analysis is further performed each fraction to identify a structure of each fraction. Moreover, when the liquid film cleavage agent contains a polymer compound, a technique such as gel permeation chromatography (GPC) is simultaneously used to further facilitate to perform identification of a

constituent. Then, a sufficient amount is obtained by procurement if the substance is a commercial item or by synthesis if the substance is not the commercial item, to measure surface tension or the interfacial tension. In particular, with regard to the measurement of the surface tension and the interfacial tension, when the liquid film cleavage agent obtained as described above is solid, the liquid film cleavage agent is heated to a temperature of a melting point of the liquid film cleavage agent plus 5°C to induce phase transition into liquid, and the measurement is performed with keeping the temperature conditions.

(Measuring method of surface tension ($\gamma_w$) of liquid film (liquid))

In an environmental range of a temperature of 25°C and a relative humidity (RH) of 65%, measurement can be performed using a platinum plate by a plate method (Wilhelmy method). As a measuring apparatus on the above occasion, an automatic surface tensiometer "CBVP-Z" (trade name, manufactured by Kyowa Interface Science Co., Ltd.) can be used. As the platinum plate, a plate having purity of 99.9%, and a size of 25 mm width and 10 mm length is used.

Further, the above-mentioned "liquid having surface tension of 50 mN/m" is a solution adjusted to be 50 $\pm$ 1 mN/m by adding a surfactant to deionised water by applying the above-described measuring method.

(Measuring method of surface tension ($\gamma_o$) of liquid film cleavage agent)

Measurement can be performed using the same apparatus by the plate method in the same manner with the measurement of the surface tension ($\gamma_w$) of the liquid film in the environmental range of the temperature of 25°C and the relative humidity (RH) of 65%. Upon this measurement, as mentioned above, when the obtained liquid film cleavage agent is solid, the liquid film cleavage agent is heated to the level of the melting point of the liquid film cleavage agent plus 5°C to induce phase transition into liquid, and the measurement is performed with keeping the temperature conditions.

(Measuring method of interfacial tension ($\gamma_{wo}$) of liquid film cleavage agent to liquid film)

In an environmental range of a temperature of 25°C and a relative humidity (RH) of 65%, measurement can be performed by a pendant drop method. As a measuring apparatus on the above occasion, Automatic Interface Viscoelasticity Measurement Apparatus (trade name "THE TRACKER," manufactured by TECLIS-IT CONCEPT) can be used. In the pendant drop method, adsorption of the surfactant contained in the liquid having surface tension of 50 mN/m starts simultaneously when a drop is formed, interfacial tension decreases with elapse of time. Therefore, the interfacial tension when the drop is formed (at 0 seconds) is read. Moreover, upon this measurement, as mentioned above, when the obtained liquid film cleavage agent is solid, the liquid film cleavage agent is heated to a temperature of a melting point of the liquid film cleavage agent plus 5°C to induce phase transition into liquid, and the measurement is performed with keeping the temperature conditions.

Moreover, upon measurement of the interfacial tension, when a density difference between the liquid film cleavage agent and the liquid having surface tension of 50 mN/m is significantly small, when viscosity is markedly high, or when an interfacial tension value is equal to or less than a measuring limit of the pendant drop, the measurement of the interfacial tension by the pendant drop method becomes difficult in several cases. On the above case, the measurement can be performed by a spinning drop method in the environmental range of the temperature of 25°C and the relative humidity (RH) of 65%. As a measuring apparatus on the above occasion, a spinning drop interfacial tensiometer (trade name "SITE100," manufactured by KURUSS) can be used. Moreover, also with regard to the measurement, the interfacial tension when a shape of a drop is stabilised is read, and when the obtained liquid film cleavage agent is solid, the liquid film cleavage agent is heated to a temperature of a melting point of the liquid film cleavage agent plus 5°C to induce phase transition into liquid, and the measurement is performed with keeping the temperature conditions.

In addition, when the interfacial tension can be measured by both measuring apparatuses, a smaller interfacial tension value is adopted as measurement results.

(Measuring method of the water solubility of liquid film cleavage agent)

In an environmental range of a temperature of 25°C and relative humidity (RH) of 65%, the obtained liquid film cleavage agent is gradually dissolved therein while 100 g of deionised water is stirred by a stirrer, and a dissolved amount at a time point of resulting in no dissolution (when suspension, precipitation, deposition or cloudiness is observed) is taken as the water solubility. Specifically, the agent is added for every 0.0001 g, and the measurement is performed. As a result, a sample in which the agent in an amount as small as 0.0001 g is observed to be not dissolved therein, the water solubility is taken as "less than 0.0001 g," and a sample in which the agent in an amount of 0.0001 g is observed to be dissolved therein, and the agent in an amount of 0.0002 g is observed to be not dissolved therein, the water solubility is taken as "0.0001 g." Further, when the liquid film cleavage agent is a surfactant, the term "dissolution" means

both monodisperse dissolution and micellar dispersion dissolution, and a dissolved amount at a time point of observation of suspension, precipitation, deposition or cloudiness is taken as the water solubility.

As the liquid film cleavage agent in the embodiment has the spreading coefficient and the water solubility as set out above, it can spread without being dissolved on the surface of the liquid film and can thrust away a layer of the liquid film nearly from the vicinity of a centre of the liquid film. Thus, the liquid film is destabilised and cleaved.

The above-described effect of the liquid film cleavage agent in the nonwoven fabric in the embodiment herein is specifically described referring to FIGs. 1 and 2.

As shown in FIG. 1, an excreted liquid such as a high viscosity liquid including menstrual blood, and urine, easily forms a liquid film 2 in a narrow interfibre region. In order to cope therewith, the liquid film cleavage agent destabilises and cleaves the liquid film, as described below, and inhibits formation of the liquid film to induce drainage from inside of the nonwoven fabric. First, as shown in FIGs. 2(A1) and 2(B1), a liquid film cleavage agent 3 contained in fibres 1 of the nonwoven fabric moves onto a surface of the liquid film 2 while keeping an interface with the liquid film 2. Next, as shown in FIGs. 2(A2) and 2(B2), the liquid film cleavage agent 3 thrusts away part of the liquid film 2 to intrude into the liquid film 2 in thickness direction, and as shown in FIGs. 2(A3) and 2(B3), the liquid film cleavage agent 3 gradually changes the liquid film 2 into a non-uniform and thin film. As a result, as shown in FIGs. 2(A4) and 2(B4), the liquid film 2 is perforated and cleaved in a bursting manner. The cleaved liquid, such as menstrual blood, is formed into a liquid drop to easily pass through an interfibre space of the nonwoven fabric, and residual liquid is reduced. Moreover, the effect of the liquid film cleavage agent on the liquid film is exhibited in a similar manner not only on liquid film between the fibres, but also on liquid film clinging on the surface of the fibres. That is, the liquid film cleavage agent can move onto the liquid film while clinging on the surface of the fibres, and thrust away part of the liquid film to cleave the liquid film. Moreover, in the case of liquid film clinging on the surface of the fibres, the liquid film cleavage agent can cleave the liquid film also by hydrophobic effect even without moving from the position where the agent is attached to the fibres, and can inhibit formation of the liquid film.

Thus, the liquid film cleavage agent according to the present invention induces drainage of the liquid from the inside of the nonwoven fabric, not by modifying a property of the liquid such as by reducing its surface tension, but by cleaving the liquid film per se formed between the fibres or on the surface of the fibres, while thrusting away and inhibiting formation of the liquid film. Thus, residual liquid in the nonwoven fabric can be reduced. Moreover, if such a nonwoven fabric is assembled into an absorbent article such as a topsheet, retention of the liquid between the fibres is suppressed, and a liquid permeation route to an absorbent body is ensured. Thus, the liquid permeability is improved, liquid flow on a surface of the sheet is suppressed, and absorption rate of the liquid is improved. In particular, the absorption rate of a liquid which easily retaines between the fibres, such as high viscosity menstrual blood, can be improved. As a result, a comfortable absorbent article of high reliability can be formed in which staining such as redness in the topsheet is inconspicuous and whose absorptive force is perceivable.

Further, in the embodiment, the interfacial tension of the liquid film cleavage agent to the liquid having surface tension of 50 mN/m is preferably 20 mN/m or less, which means that "interfacial tension ($\gamma_{wo}$) of the liquid film cleavage agent to the liquid film" being one variable for determining the value of the spreading coefficient (S) in Expression (1) mentioned above is 20 mN/m or less. The spreading coefficient of the liquid film cleavage agent is improved by suppressing "interfacial tension ($\gamma_{wo}$) of the liquid film cleavage agent to the liquid film," and the liquid film cleavage agent easily moves from the surface of the fibres to the vicinity of the centre of the liquid film, and the above-mentioned effect becomes clearer. From this viewpoint, "the interfacial tension to the liquid having surface tension of 50 mN/m" of the liquid film cleavage agent is more preferably 17 mN/m or less, further preferably 13 mN/m or less, still further preferably 10 mN/m or less, particularly preferably 9 mN/m or less, and especially preferably 1 mN/m or less. On the other hand, a lower limit thereof is not particularly limited, and only needs to be larger than 0 mN/m from a viewpoint of insolubility into the liquid film. Further, if the interfacial tension is 0 mN/m, that is, if the liquid film cleavage agent is soluble, the interface between the liquid film and the liquid film cleavage agent cannot be formed, and therefore Expression (1) does not hold, and spreading of the agent does not occur.

As is known from the expression, a numerical value of the spreading coefficient changes depending on the surface tension of a target liquid. For example, when the surface tension of the target liquid is 72 mN/m, the surface tension of the liquid film cleavage agent is 21 mN/m, and the interfacial tension thereof is 0.2 mN/m, the spreading coefficient becomes 50.8 mN/m.

Moreover, when the surface tension of the target liquid is 30 mN/m, the surface tension of the liquid film cleavage agent is 21 mN/m, and the interfacial tension thereof is 0.2 mN/m, the spreading coefficient becomes 8.8 mN/m.

In any cases, in an agent in which the spreading coefficient is larger, a liquid film cleavage effect becomes larger.

In this description, the numerical value in the surface tension of 50 mN/m is defined. However, even if the surface tension is different, there exists no change in a magnitude relationship of the numerical value of the spreading coefficient between substances. Therefore, even if the surface tension of the bodily fluid should be changed, depending on a daily physical condition, the agent, in which the spreading coefficient is larger, shows a superb liquid film cleavage effect.

Moreover, in the embodiment, the surface tension of the liquid film cleavage agent is preferably 32 mN/m or less, more preferably 30 mN/m or less, further preferably 25 mN/m or less, and particularly preferably 22 mN/m or less. Moreover, the surface tension is preferably smaller, and a lower limit thereof is not particularly limited. From a viewpoint of durability of the liquid film cleavage agent, the surface tension is practically 1 mN/m or more.

Next, a liquid film cleavage agent and a nonwoven fabric containing the liquid film cleavage agent in a second embodiment will be described.

In the liquid film cleavage agent in the second embodiment, a spreading coefficient thereof to a liquid having surface tension of 50 mN/m is larger than 0 mN/m, namely, a positive value, a water solubility thereof is 0 g or more and 0.025 g or less, and an interfacial tension thereof to the liquid having surface tension of 50 mN/m is 20 mN/m or less. The nonwoven fabric in the second embodiment contains the liquid film cleavage agent. To have the "interfacial tension thereof to the liquid having surface tension of 50 mN/m" of 20 mN/m or less, as mentioned above, means that the diffusivity of the liquid film cleavage agent on the liquid film is improved as mentioned above. Thus, even when the spreading coefficient is comparatively small as in the case where "spreading coefficient to the liquid having surface tension of 50 mN/m" is less than 15 mN/m, the diffusivity is high, and therefore a large amount of the liquid film cleavage agent is dispersed to the liquid film from the surface of the fibres, and the effect similar to the effect in the case of the first embodiment can be produced by thrusting away the liquid film in many positions.

Further, the "spreading coefficient to the liquid having surface tension of 50 mN/m," the "water solubility" and the "interfacial tension to the liquid having surface tension of 50 mN/m" with regard to the liquid film cleavage agent are defined in a manner same as definitions in the first embodiment, and the measuring methods thereof are the same.

In the embodiment, from a viewpoint of further effectively exhibiting the effect of the liquid film cleavage agent, the above-described "interfacial tension to the liquid having surface tension of 50 mN/m" is preferably 17 mN/m or less, more preferably 13 mN/m or less, further preferably 10 mN/m or less, still further preferably 9 mN/m or less, and particularly preferably 1 mN/m or less. A lower limit is not particular limited in a manner similar to the first embodiment, and from a viewpoint of insolubility in the liquid film (the liquid having surface tension of 50 mN/m), the interfacial tension is practically adjusted to be larger than 0 mN/m.

Moreover, from a viewpoint of further effectively exhibiting the effect of the liquid film cleavage agent, the "spreading coefficient to the liquid having surface tension of 50 mN/m" is preferably 9 mN/m or more, more preferably 10 mN/m or more, and further preferably 15 mN/m or more. An upper limit thereof is not particularly limited, but from a viewpoint in which the surface tension of the liquid which forms the liquid film serves as the upper limit from Expression (1), the spreading coefficient is substantially 50 mN/m or less.

Moreover, further preferable ranges of the surface tension and the water solubility of the liquid film cleavage agent are the same with the ranges in the first embodiment.

The nonwoven fabric in the first embodiment and the nonwoven fabric in the second embodiment each preferably further contain, in addition to the above-described respective liquid film cleavage agents, a phosphoric acid ester type anionic surfactant. Thus, hydrophilicity on the surface of the fibres is improved and wettability is improved to increase a contact area in which the liquid film and the liquid film cleavage agent are brought into contact. In addition, because blood and urine contain a surface active substance having a phosphoric acid group which originates in a living body, when the surfactant having the phosphoric acid group is used together with the liquid film cleavage agent, the surfactant shows a compatibility and a fine affinity for phospholipid contained in blood and urine. Thereby, the liquid film cleavage agent easily moves onto the liquid film, and cleavage of the liquid film is further induced. A content ratio of the liquid film cleavage agent to the phosphoric acid ester type anionic surfactant is preferably (1:1) to (19:1), more preferably (2:1) to (15:1), and further preferably (3:1) to (10:1) in terms of a mass ratio. In particular, the content ratio is preferably (5:1) to (19:1), further preferably (8:1) to (16:1), and still further preferably (11:1) to (13:1) in terms of a mass ratio.

The phosphoric acid ester type anionic surfactant can be used without particular restriction. Specific examples thereof include alkyl ether phosphoric acid ester, dialkyl phosphoric acid ester and alkyl phosphoric acid ester. Above all, alkyl phosphoric acid ester is preferable from a viewpoint of a function of improving affinity for the liquid film and providing processability of the nonwoven fabric.

As alkyl ether phosphoric acid ester, various kinds thereof can be used without particular restriction. Specific examples include alkyl ether phosphoric acid ester having a saturated carbon chain such as polyoxyalkylene stearyl ether phosphoric acid ester, polyoxyalkylene myristyl ether phosphoric acid ester, polyoxyalkylene lauryl ether phosphoric acid ester and polyoxyalkylene palmityl ether phosphoric acid ester; alkyl ether phosphoric acid ester having an unsaturated carbon chain such as polyoxyalkylene oleyl ether phosphoric acid ester and polyoxyalkylene palmitoleyl ether phosphoric acid ester; and alkyl ether phosphoric acid ester having a side chain in each carbon chain thereof. The alkyl ether phosphoric acid ester is further preferably a completely or partially neutralised salt of mono- or di-polyoxyalkylene alkyl ether phosphoric acid ester having a carbon chain of 16 to 18. Moreover, specific examples of polyoxyalkylene include polyoxyethylene, polyoxypropylene, polyoxybutylene and a material in which constituent monomers thereof are copolymerised. In addition, specific examples of a salt of alkyl ether phosphoric acid ester include a salt with alkali metal such as sodium and potassium, ammonia and various amines. As alkyl ether phosphoric acid ester, one kind can be

used alone, or two or more kinds can be mixed and used.

Specific examples of alkyl phosphoric acid ester include alkyl phosphoric acid ester having a saturated carbon chain such as stearyl phosphoric acid ester, myristyl phosphoric acid ester, lauryl phosphoric acid ester and palmityl phosphoric acid ester; alkyl phosphoric acid ester having an unsaturated carbon chain such as oleyl phosphoric acid ester and palmitoleyl phosphoric acid ester; and alkyl phosphoric acid ester having a side chain in each carbon chain thereof. Further preferably, the alkyl phosphoric acid ester is a completely or partially neutralised salt of monoalkyl phosphoric acid ester or dialkyl phosphoric acid ester having a carbon chain of 16 to 18. In addition, specific examples of a salt of alkyl phosphoric acid ester include a salt with alkali metal such as sodium and potassium, ammonia and various amines. As alkyl phosphoric acid ester, one kind can be used alone, or two or more kinds can be mixed and used.

In the first embodiment and the second embodiment, a contact angle of constituent fibres of the nonwoven fabric containing the liquid film cleavage agent, or the liquid film cleavage agent and the phosphoric acid ester type anionic surfactant as described above is preferably 90 degrees or less, more preferably 80 degrees or less, and further preferably 70 degrees or less. Thus, the surface of the fibres becomes hydrophilic, a wettable area is increased, and the liquid film cleavage agent easily moves onto the liquid film.

Measurement of the above-described contact angle can be performed by the following method.

That is, fibres are taken from a predetermined site of a nonwoven fabric, and a contact angle of water to the fibres is measured. As a measuring apparatus, Automatic Contact Angle Meter MCA-J manufactured by Kyowa Interface Science Co., Ltd. is used. Deionised water is used for measurement of the contact angle. Measurement is performed under measurement conditions of a temperature of 25°C and relative humidity (RH) of 65%. A liquid amount to be dropped from an inkjet system water droplet jet unit (Pulse Injector CTC-25 having a jet unit bore of 25 μm, manufactured by Cluster Technology Co., Ltd.) is set to 20 picoliters, and a water drop is dropped just above the fibres. An aspect of dropping is recorded on a high-speed recording device connected to a horisontally installed camera. From a viewpoint of performing image analysis later, a personal computer in which a high-speed capture device is assembled is preferable as a recording device. In the measurement, an image is recorded for every 17 msec. In the recorded video, a first image in which the water drop is dropped on the fibres taken from the nonwoven fabric is subjected to image analysis by using attached software FAMAS (a software version: 2.6.2, an analysis technique: liquid drop method, an analysis method: θ/2 method, an image processing algorithm: non-reflection, an image processing image mode: frame, a threshold level: 200, and no curvature correction), an angle between a surface of waterdrop in contact with air, and the fibres is calculated, and the calculated angle is taken as the contact angle. The fibres taken from the nonwoven fabric are cut to a fibre length of 1 mm, and the fibres are placed on a sample stage of the contact angle meter, and horisontally maintained. Contact angles in different two places are measured for one piece of the fibres. The contact angles in case of N = 5 pieces are measured to one decimal point, and a value obtained by averaging measurement values of ten places in total (rounded to one decimal place) is defined as the contact angle.

Next, specific examples of the liquid film cleavage agents in the first embodiment and the second embodiment will be described. These agents are in the above-mentioned specific numerical value range to have properties of being insoluble in water or hardly soluble in water, and exhibit the above-described liquid film cleavage effect. In contrast, the surfactant to be used as the conventional fibre treating agent is basically a water-soluble agent which is practically dissolved in water and used, and is not the liquid film cleavage agent according to the present invention.

As the liquid film cleavage agent in the first embodiment and the second embodiment, a compound having a mass average molecular weight of 500 or more is preferable. The mass average molecular weight greatly influences viscosity of the liquid film cleavage agent. If the viscosity is excessively low, move of the liquid film cleavage agent from the fibres to the liquid film is enhanced to cause running down of the liquid film cleavage agent when the liquid passes through the nonwoven fabric, so that sustainability of the liquid film cleavage effect is reduced. From a viewpoint of adjusting the viscosity to a level at which the liquid film cleavage effect is sufficiently sustained, the mass average molecular weight of the liquid film cleavage agent is more preferably 1000 or more, further preferably 1,500 or more, and particularly preferably 2,000 or more. On the other hand, if the viscosity is excessively high, diffusivity is reduced in several cases, and from a viewpoint of adjusting the viscosity to a level at which this diffusivity is held, the mass average molecular weight is preferably 50,000 or less, more preferably 20,000 or less, and further preferably 10,000 or less. Measurement of the average molecular weight is performed by using gel permeation chromatograph (GPC) "CCPD" (trade name, manufactured by TOSOH CORPORATION). Measurement conditions are as described below. Moreover, calculation of equivalent molecular weight is performed by using polystyrene.

Separation column: GMHHR-H+GMHHR-H (cation)
Eluent: L FAMIN DM20/CHCl3
Solvent flow rate: 1.0 ml/min
Separation column temperature: 40°C

Moreover, as the liquid film cleavage agent in the first embodiment, as mentioned below, a compound having at least one kind structure selected from the group consisting of the following structures X, X-Y and Y-X-Y is preferable.

The structure X designates a siloxane chain having a structure in which any of basic structures of >C(A)- (C

designates a carbon atom, moreover, <, > and - each designate a bonding, hereinafter, the same applies.), $-C(A)_2-$, $-C(A)(B)-$, $>C(A)-C(R^1)<$, $>C(R^1)-$, $-C(R^1)(R^2)-$, $-C(R^1)_2-$, $>C<$, $-Si(R^1)_2O-$ and $-Si(R^1)(R^2)O$ is repeated, or two or more kinds thereof are combined; or a mixed chain thereof. The structure X has, in an end of the structure X, a hydrogen atom or at least one kind of group selected from the group consisting of $-C(A)_3$, $-C(A)_2B$, $-C(A)(B)_2$, $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ and $-Si(R^1)_2(R^2)$.

The above-described $R^1$ and $R^2$ each independently designate various substituents such as a hydrogen atom, an alkyl group (the number of carbon atoms is preferably 1 to 20, for example, a methyl group, an ethyl group or a propyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.) and a halogen atom (for example, a fluorine atom is preferable.). A and B each independently designate a substituent including an oxygen atom or a nitrogen atom, such as a hydroxyl group, a carboxylic acid group, an amino group, an amide group, an imino group and a phenol group. When a plurality of $R^1$, $R^2$, A and B exist for each in the structure X, these may be identical to or different from each other. Moreover, a continuous inter-C (carbon atoms) or inter-Si bonding is ordinarily a single bond, but may include a double bond or a triple bond, and the inter-C or inter-Si bonding may include a linking group such as an ether group (-O-), an amide group ($--CONR^A-$: $R^A$ is a hydrogen atom or a monovalent group), an ester group (-COO-), a carbonyl group (-CO-) or a carbonate group (-OCOO-). The number of bonding of one C and one Si with any other C or Si is 1 to 4, and a long-chain silicone chain (siloxane chain) or a mixed chain may be branched or may have a radial structure.

Y designates a hydrophilic group having hydrophilicity, the group containing an atom selected from a hydrogen atom, a carbon atom, an oxygen atom, a nitrogen atom, a phosphorus atom or a sulphur atom. Specific examples include a hydrophilic group alone, such as a hydroxyl group, a carboxylic acid group, an amino group, an amide group, an imino group, a phenol group, a polyoxyalkylene group (the number of carbon atoms of an oxyalkylene group is preferably 1 to 4, for example, a polyoxyethylene (POE) group and a polyoxypropylene (POP) group are preferable.), a sulphonic acid group, a sulphate group, a phosphoric acid group, a sulphobetaine group, a carbobetaine group, a phosphobetaine group (the betaine group means a betaine residual group formed by removing one hydrogen atom from each betaine compound.) and a quaternary ammonium group; or a hydrophilic group formed from a combination thereof. In addition thereto, specific examples also include a group and a functional group listed in $M^1$ as mentioned below. In addition, when a plurality of Y exists, these groups may be identical to or different from each other.

In the structures X-Y and Y-X-Y, Y is bonded with X or a group at an end of X. When Y is bonded with the group at the end of X, for example, the group at the end X is bonded with Y after hydrogen atoms in the number identical with the number of bonding with Y are eliminated.

In this structure, the above-mentioned spreading coefficient, water solubility and interfacial tension can be satisfied by selecting the hydrophilic groups Y, A and B from the groups specifically described. Thus, an objective liquid film cleavage effect is developed.

In the above-described liquid film cleavage agent, a compound in which the structure X has a siloxane structure is preferable. Further, as specific examples of the above-described structures X, X-Y, Y-X-Y in the liquid film cleavage agent, a compound comprising a siloxane chain in which structures represented by any one of the following formulas (1) to (11) are arbitrarily combined is preferable. Further, from a viewpoint of the liquid film cleavage effect, it is preferable that the compound has a mass average molecular weight in the range mentioned above.

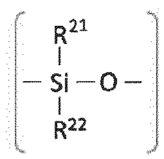 ••• (1)

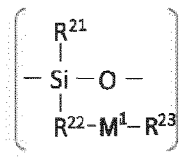 ••• (5)

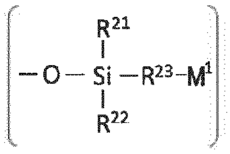 ••• (9)

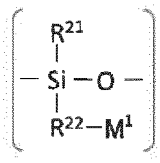 ••• (2)

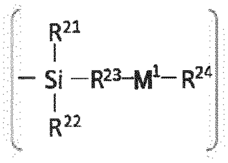 ••• (6)

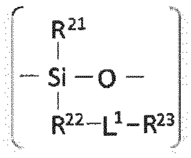 ••• (10)

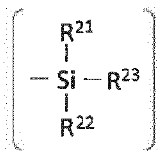 ••• (3) 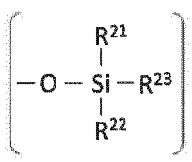 ••• (7) 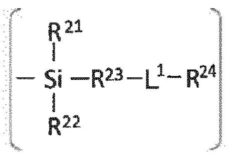 ••• (11)

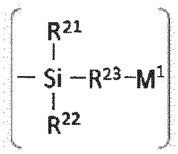 ••• (4) 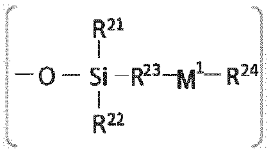 ••• (8)

In Formulas (1) to (11), $M^1$, $L^1$, $R^{21}$ and $R^{22}$ designate the following monovalent or polyvalent (divalent or more valent) group. $R^{23}$ and $R^{24}$ designate the following monovalent or polyvalent (divalent or more valent) group or a single bond.

$M^1$ designates a polyoxyethylene group, a polyoxypropylene group, a polyoxybutylene group, a group having a polyoxyalkylene group in combination therewith, an erythritol group, a xylitol group, a sorbitol group, a hydrophilic group having a plurality of hydroxyl groups such as a glycerol group or an ethylene glycol group (a hydrophilic group formed by removing one hydrogen atom from the above-described compound having a plurality of hydroxyl groups such as erythritol), a hydroxyl group, a carboxylic acid group, a mercapto group, an alkoxy group (the number of carbon atoms is preferably 1 to 20, for example, a methoxy group is preferable.), an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith. In addition, when $M^1$ is a polyvalent group, $M^1$ designates a group formed by further removing one or more hydrogen atoms from each of the groups or the functional group as mentioned above.

$L^1$ designates a linking group of an ether group, an amino group (the amino group adoptable as $L^1$ is represented by >$NR^C$ ($R^C$ is a hydrogen atom or a monovalent group).), an amide group, an ester group, a carbonyl group or a carbonate group.

$R^{21}$, $R^{22}$, $R^{23}$ and $R^{24}$ each independently designate an alkyl group (the number of carbon atoms is preferably 1 to 20, for example, a methyl group, an ethyl group, a propyl group, an isopropyl group, a butyl group, a pentyl group, a hexyl group, a heptyl group, 2-ethylhexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, an aralkyl group, a hydrocarbon group in combination therewith, or a halogen atom (for example, a fluorine atom is preferable.). In addition, when $R^{22}$ and $R^{23}$ are a polyvalent group, a polyvalent hydrocarbon group formed by further removing one or more hydrogen atoms or fluorine atoms from the above-described hydrocarbon group is represented.

Moreover, when $R^{22}$ or $R^{23}$ is bonded with $M^1$, specific examples of a group adoptable as $R^{22}$ or $R^{23}$ include, in addition to each of the groups, the hydrocarbon group or the halogen atom described above, an imino group adoptable as $R^{32}$.

Above all, the liquid film cleavage agent is preferably a compound having the structure represented by any of Formulas (1), (2), (5) and (10) as X, and having the structure represented by any of the above-described formulas other than these formulas as a group formed of an end of X or formed of the end of X and Y. Further, the liquid film cleavage agent is preferably a compound comprising a siloxane chain having at least one structure represented by any of the above-described formulas (2) (4), (5), (6), (8) and (9) as a group formed of X or formed of the end of X and Y.

Specific examples of the above-described compound include organic-modified silicone (polysiloxane) of a silicone-based surfactant. Specific examples of organic-modified silicone being modified with a reactive organic group include amino-modified silicone, epoxy-modified silicone, carboxy-modified silicone, diol-modified silicone, carbinol-modified silicone, (meth) acrylic-modified silicone, mercapto-modified silicone and phenol-modified silicone. Moreover, specific examples of organic-modified silicone being modified with a nonreactive organic group include polyether-modified silicone (including polyoxyalkylene-modified silicone), methylstyryl-modified silicone, long-chain alkyl-modified silicone, higher fatty acid ester-modified silicone, higher alkoxy-modified silicone, higher fatty acid-modified silicone and fluorine-modified silicone. The spreading coefficient at which the above-described liquid film cleavage effect is produced can be obtained by appropriately changing a molecular weight of a silicone chain, a modification ratio, the addition number of moles of a modifying group, in corresponding to kinds of the organic-modified silicone, for example. The term "long chain" herein means a material in which the number of carbon atoms is 12 or more, and preferably 12 to 20. Moreover, the term "higher" means a material in which the number of carbon atoms is 6 or more, and preferably 6 to 20.

Above all, modified silicone having a structure in which the liquid film cleavage agent being the modified silicone has at least one oxygen atom in a modifying group, such as polyoxyalkylene-modified silicone, epoxy-modified silicone, carbinol-modified silicone and diol-modified silicone, is preferable, and polyoxyalkylene modified-silicone is particularly preferable. Polyoxyalkylene-modified silicone is hard to be permeated into the fibres, and easy to remain on the surface thereof because polyoxyalkylene-modified silicone has a polysiloxane chain. Moreover, with the respect to polyoxyalkylene-modified silicone, the affinity with water is improved, and the interfacial tension is small by comprising a hydrophilic polyoxyalkylene chain, and therefore move onto a surface of the liquid film as mentioned above is easily induced, and such a case is preferable. Moreover, even if thermal fusion processing such as embossing is applied, polyoxyalkylene-modified silicone easily remains on the surface of the fibres in the part, and the liquid film cleavage effect is hardly reduced. The liquid film cleavage effect is sufficiently developed particularly in an embossed part in which the liquid is easy to accumulate, and therefore such a case is preferable.

Specific examples of the polyoxyalkylene-modified silicone include compounds represented by the following formulas [I] to [IV]. Further, the polyoxyalkylene-modified silicone preferably has the mass average molecular weight in the above-mentioned range from a viewpoint of the liquid film cleavage effect.

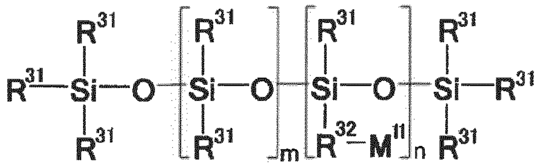

[I]

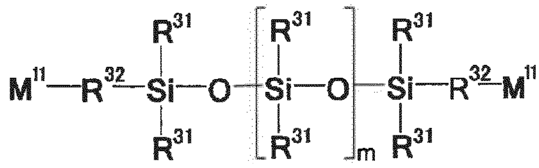

[II]

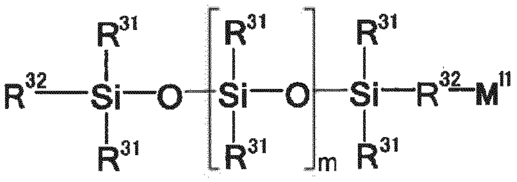

[III]

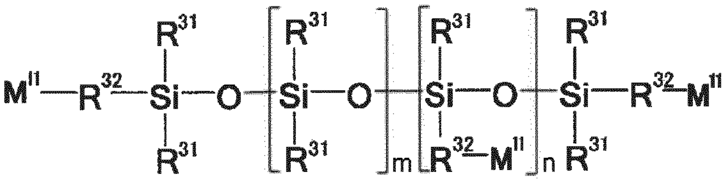

[IV]

In the formulas, $R^{31}$ designates an alkyl group (the number of carbon atoms is preferably 1 to 20, for example, a methyl group, an ethyl group, a propyl group, an isopropyl group, a butyl group, a pentyl group, a hexyl group, a heptyl group, a 2-ethyl-hexyl group, a nonyl group or a decyl group is preferable.). $R^{32}$ designates a single bond or an alkylene group (the number of carbon atoms is preferably 1 to 20, for example, a methylene group, an ethylene group, a propylene group or a butylene group is preferable.), and preferably designates the alkylene group. A plurality of $R^{31}$ and a plurality of $R^{32}$ may be each identical to or different from each other. $M^{11}$ designates a group having a polyoxyalkylene group, and the polyoxyalkylene group is preferable. Specific examples of the above-described polyoxyalkylene group include a polyoxyethylene group, a polyoxypropylene group, a polyoxybutylene group or a material in which constituent monomers thereof are copolymerised. Then, m and n are each independently an integer of 1 or more. In addition, signs of these repeating units are separately determined in each of Formulas (I) to (IV), and do not always represent an identical integer, and may be different from each other.

Moreover, polyoxyalkylene-modified silicone may have either or both of modifying groups of polyoxyethylene modification and polyoxypropylene modification. Moreover, the modified silicone preferably has a methyl group in $R^{31}$ as an alkyl group of a silicone chain in order to have insolubility in water and low interfacial tension. A material having this modifying group or the silicone chain is not particularly limited, but materials described in the paragraphs {0006}

and {0012} in JP-A-2002-161474 are exemplified. Further specific examples include polyoxyethylene (POE) polyoxypropylene (POP)-modified silicone, polyoxyethylene (POE)-modified silicone and polyoxypropylene (POP)-modified silicone. Specific examples of POE-modified silicone include POE (3)-modified dimethyl silicone to which 3 moles of POE are added. Specific examples of POP-modified silicone include POP (10)-modified dimethyl silicone, POP (12)-modified dimethyl silicone and POP (24)-modified dimethyl silicone, to which 10 moles of POP, 12 moles of POP and 24 moles of POP are added, respectively.

The spreading coefficient and the water solubility in the above-mentioned first embodiment can be adjusted in predetermined ranges, for example, in the polyoxyalkylene-modified silicone, by the addition number of moles of polyoxyalkylene groups (the number of bonding oxyalkylene groups which form a polyoxyalkylene group based on 1 mole of polyoxyalkylene modified silicone), the following modification ratio. In this liquid film cleavage agent, the surface tension and the interfacial tension can be adjusted to predetermined ranges in a similar manner, respectively.

From the above-described viewpoint, the addition number of moles of the polyoxyalkylene groups is preferably 1 or more. At the number less than 1, the interfacial tension is increased for the above-described liquid film cleavage effect to cause reduction of the spreading coefficient, and therefore the liquid film cleavage effect is weakened. From this viewpoint, the addition number of moles is more preferably 3 or more, and further preferably 5 or more. On the other hand, if the addition number of moles is excessively large, the liquid film cleavage agent becomes hydrophilic, and the water solubility is increased. From this viewpoint, the addition number of moles is preferably 30 or less, more preferably 20 or less, and further preferably 10 or less.

If the modification ratio of modified silicone is excessively small, the hydrophilicity is impaired, and therefore the modification ratio is preferably 5% or more, more preferably 10% or more, and further preferably 20% or more. Moreover, if the modification ratio is excessively large, the liquid film cleavage agent is dissolved in water, and therefore the modification ratio is preferably 95% or less, more preferably 70% or less, and further preferably 40% or less. In addition, the modification ratio of the modified silicone means a proportion of the number of repeating units of a modified siloxane bonding portion based on the total number of repeating units of a siloxane bonding portion in one molecule of the modified silicone. For example, the modification ratio is expressed by the expression: $(n/m + n) \times 100\%$ in Formulas [I] and [IV], the expression: $(2/m) \times 100\%$ in Formula [II], and the expression: $(1/m) \times 100\%$ in Formula [III].

Moreover, the spreading coefficient and the water solubility mentioned above each can be set in a predetermined range, in addition to the material described above, for example, in polyoxyalkylene-modified silicone, by simultaneously using a water-soluble polyoxyethylene group, and a water-insoluble polyoxypropylene group and a water-insoluble polyoxybutylene group as a modifying group, by changing a molecular weight of a water-insoluble silicone chain, or introducing an amino group, an epoxy group, a carboxy group, a hydroxyl group, a carbinol group thereinto in addition to polyoxyalkylene modification as the modifying group.

Polyalkylene modified silicone used as the liquid film cleavage agent is preferably contained in 0.02 mass% or more and 5.0 mass% or less in terms of a content proportion (Oil Per Unit) to fibre mass. If the content proportion of the polyalkylene modified silicone is excessively large, a surface material becomes sticky, and therefore such a case is not preferable. From this viewpoint, the content proportion (OPU) is more preferably 1.0 mass% or less, and further preferably 0.40 mass% or less. Moreover, if the content proportion of the polyalkylene modified silicone is excessively small, the liquid film cleavage effect becomes insufficient. From this viewpoint, the content proportion (OPU) is more preferably 0.04 mass% or more, and further preferably 0.10 mass% or more.

As the liquid film cleavage agent in the second embodiment, as mentioned later, a compound having at least one kind structure selected from the group consisting of the following structures Z, Z-Y and Y-Z-Y is preferable.

The structure Z designates a hydrocarbon chain having a structure in which any of basic structures of >C(A)- (C: carbon atom), $-C(A)_2-$, -C(A)(B)-, $>C(A)-C(R^3)<$, $>C(R^3)-$, $-C(R^3)(R^4)-$, $-C(R^3)_2-$ and >C< is repeated, or two or more kinds thereof are combined. The structure Z has, at an end thereof, a hydrogen atom or at least one kind of group selected from the group consisting of $-C(A)_3$, $-C(A)_2B$, $-C(A)(B)_2$, $-C(A)_2-C(R^3)_3$, $-C(R^3)_2A$ and $-C(R^3)_3$.

The above-described $R^3$ and $R^4$ each independently designate various kinds of substituents such as a hydrogen atom, an alkyl group (the number of carbon atoms is preferably 1 to 20, for example, a methyl group, an ethyl group, a propyl group, an isopropyl group, a butyl group, a pentyl group, a hexyl group, a heptyl group, a 2-ethyl-hexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, or an aralkyl group, or a hydrocarbon group in combination therewith, or a fluorine atom. A and B each independently designates a substituent containing an oxygen atom or a nitrogen atom, such as a hydroxyl group, a carboxylic acid group, an amino group, an amide group, an imino group or a phenol group. When a plurality of $R^3$, $R^4$, A or B are each included in the structure X, these may be identical to or different from each other. Moreover, a continuous inter-C (carbon atoms) bonding is ordinarily a single bond, but may include a double bond or a triple bond, and the inter-C bonding may include a linking group such as an ether group, an amide group, an ester group, a carbonyl group or a carbonate group. The number of bonding of one C with any other C is 1 to 4, and a long-chain hydrocarbon chain may have a branched structure or may have a radial structure.

Y designates a hydrophilic group having hydrophilicity, the hydrophilic group containing an atom selected from a hydrogen atom, a carbon atom, an oxygen atom, a nitrogen atom, a phosphorus atom and a sulphur atom. Specific examples include: a hydroxyl group, a carboxylic acid group, an amino group, an amide group, an imino group and a phenol group; or a polyoxyalkylene group (the number of carbon atoms of an oxyalkylene group is preferably 1 to 4, for example, a polyoxyethylene group, a polyoxypropylene group, a polyoxybutylene group, or a polyoxyalkylene group in combination therewith is preferable.); or a hydrophilic group having a plurality of hydroxyl groups, such as an erythritol group, a xylitol group, a sorbitol group, a glycerol group and an ethylene glycol group; or a hydrophilic group alone, such as a sulphonic acid group, a sulphate group, a phosphoric acid group, a sulphobetaine group, a carbobetaine group, a phosphobetaine group, a quaternary ammonium group, an imidazolium betaine group, an epoxy group, a carbinol group and a methacrylic group; or a hydrophilic group formed of a combination thereof. In addition, when Y is plural, the plurality may be identical to or different from each other.

In the structures Z-Y and Y-Z-Y, Y is bonded with Z or a group at an end of Z. When Y is bonded with the group at the end of Z, the group at the end of Z is bonded with Y, for example, after hydrogen atoms in the number identical with the number of bonding with Y are eliminated.

In this structure, the spreading coefficient, the water solubility and the interfacial tension mentioned above can be satisfied by selecting the hydrophilic groups Y, A and B from the groups specifically described. Thus, an objective liquid film cleavage effect is developed.

The liquid film cleavage agent is preferably a compound obtained by arbitrarily combining structures represented by the following formulas (12) to (25) as specific examples of the structures Z, Z-Y and Y-Z-Y. Further, from a viewpoint of the liquid film cleavage effect, it is preferable that this compound has a mass average molecular weight in the above-mentioned range.

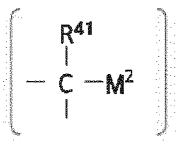 ••• (12)
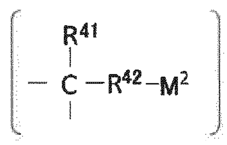 ••• (13)
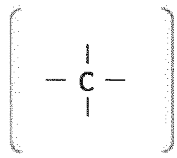 ••• (14)

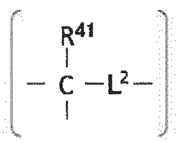 ••• (15)
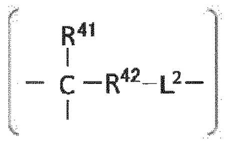 ••• (16)
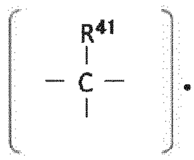 ••• (17)

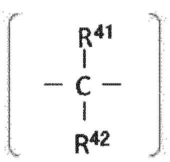 ••• (18)
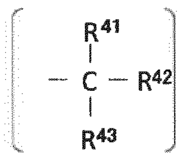 ••• (19)
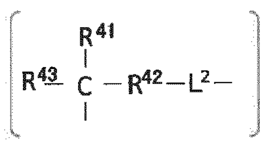 ••• (20)

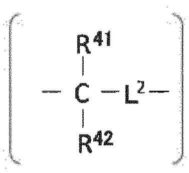 ••• (21)
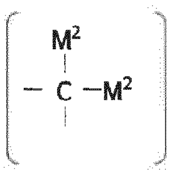 ••• (22)
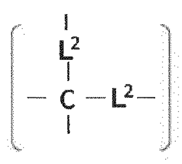 ••• (23)

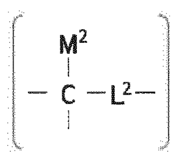 ••• (24)
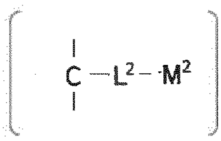 ••• (25)

In Formulas (12) to (25), $M^2$, $L^2$, $R^{41}$, $R^{42}$ and $R^{43}$ designate the following monovalent or polyvalent (divalent or more valent) group.

$M^2$ designates a polyoxyethylene group, a polyoxypropylene group, a polyoxybutylene group, a group having a polyoxyalkylene group in combination therewith, an erythritol group, a xylitol group, a sorbitol group, a hydrophilic

group having a plurality of hydroxyl groups such as a glycerol group or an ethylene glycol group, a hydroxyl group, a carboxylic acid group, a mercapto group, an alkoxy group (the number of carbon atoms is preferably 1 to 20, for example, a methoxy group is preferable.), an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith.

$L^2$ designates a linking group such as an ether group, an amino group, an amide group, an ester group, a carbonyl group, a carbonate group, a polyoxyethylene group, a polyoxypropylene group, or a polyoxybutylene group, or a polyoxyalkylene group in combination therewith.

$R^{41}$, $R^{42}$ and $R^{43}$ each independently designate various substituents such as a hydrogen atom, an alkyl group (the number of carbon atoms is preferably 1 to 20, for example, a methyl group, an ethyl group, a propyl group, an isopropyl group, a butyl group, a pentyl group, a hexyl group, a heptyl group, 2-ethylhexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, an aralkyl group, a hydrocarbon group in combination therewith, or a halogen atom (for example, a fluorine atom is preferable.).

When $R^{42}$ is a polyvalent group, $R^{42}$ designates a group formed by further removing one or more hydrogen atoms from the above-described each substituent.

In addition, at an end of the bonding described in each structure, any other structure may be arbitrarily linked, or a hydrogen atom may be introduced.

Further, specific examples of the above-described compounds include the following compounds, but are not limited thereto.

First, examples thereof include a polyether compound and a nonionic surfactant. Specific examples thereof include polyoxyalkylene alkyl (POA) ether represented by any of formulas in Formula [V]; and polyoxyalkylene glycol which is represented by any of formulas in Formula [VI] and has a mass average molecular weight of 1000 or more, Steareth, Beheneth, PPG myristyl ether, PPG stearyl ether and PPG behenyl ether. As polyoxyalkylene alkyl ether, lauryl ether to which POP is added in 3 moles or more and 24 moles or less, and preferably in 5 moles, is preferable. As a polyether compound, polypropylene glycol having a mass average molecular weight of 1000 to 10000 and preferably 3000 to which polypropylene glycol is added in 17 moles or more and 180 moles or less, and preferably in about 50 moles is preferable. In addition, the measurement of the mass average molecular weight can be performed by the above-mentioned measuring method.

The polyether compound or nonionic surfactant is preferably contained in 0.10 mass% or more and 5.0 mass% or less in terms of a content proportion (Oil Per Unit) to fibre mass. If the content proportion of the polyether compound or the nonionic surfactant is excessively large, a surface material becomes sticky, and therefore such a case is not preferable. From this viewpoint, the content proportion (OPU) is more preferably 1.0 mass% or less, and further preferably 0.40 mass% or less. Moreover, if the content proportion of the polyether compound or the nonionic surfactant is excessively small, the liquid film cleavage effect becomes insufficient. From this viewpoint, the content proportion (OPU) is more preferably 0.15 mass% or more, and further preferably 0.20 mass% or more.

$$C_mH_n-L^{21}-\left(C_aH_bO\right)_m-L^{21}-R^{51}$$

or

$$C_mH_n-L^{21}-\left(C_aH_bO\right)_m-R^{51} \qquad [V]$$

or

$$C_mH_n-L^{21}-\left(C_aH_bO\right)_m-H$$

$$R^{51}-\left(C_aH_bO\right)_m-R^{51} \qquad [VI]$$

In the Formulas, $L^{21}$ designates a linking group such as an ether group, an amino group, an amide group, an ester group, a carbonyl group, a carbonate group, a polyoxyethylene group, a polyoxypropylene group, or a polyoxybutylene group, or a polyoxyalkylene group in combination therewith. $R^{51}$ designate various substituents such as a hydrogen atom, a methyl group, an ethyl group, a propyl group, an isopropyl group, a butyl group, a pentyl group, a hexyl group, a heptyl group, 2-ethylhexyl group, a nonyl group, a decyl group, a methoxy group, an ethoxy group, a phenyl group, a fluoroalkyl group, an aralkyl group, a hydrocarbon group in combination therewith, or a fluorine atom. a, b, m and n each are independently an integer of 1 or more. $C_mH_n$ herein designates an alkyl group (n = 2m + 1), and $C_aH_b$ designates an alkylene group (a = 2b). In addition, the number of carbon atoms and the number of hydrogen atoms are each independently determined in each of Formulas (V) and (VI), and do not always represent an identical integer, and may be different from each other. Hereinafter, a same rule applies also to m, m', m", n, n' and n" in formulas (VII) to (XV). In addition, "m" in $-(C_aH_bO)_m-$ is an integer of 1 or more. Values of the repeating units are each independently determined in each of Formulas (V) and (VI), and do not always represent an identical integer, and may be different from each other.

The spreading coefficient, the surface tension and the water solubility described above in the second embodiment each can be set in a predetermined range, in the polyether compound or the nonionic surfactant, for example, by the number of moles of a polyoxyalkylene group. From this viewpoint, the number of mole of the polyoxyalkylene group is preferably 1 or more and 70 or less. At the number less than 1, the interfacial tension is high, and the above-described liquid film cleavage effect is weakened. From this viewpoint, the number of moles is more preferably 5 or more, and further preferably 7 or more. On the other hand, if the number of moles is excessively large, entanglement of molecular chains is strong, and therefore diffusivity in the liquid film is deteriorated, and such a case is not preferable. From this viewpoint, the addition number of moles is preferably 70 or less, more preferably 60 or less, and further preferably 50 or less.

Moreover, the spreading coefficient, the surface tension, interface tension and the water solubility described above each can be set in a predetermined range, in the polyether compound or the nonionic surfactant, for example, by simultaneously using a water-soluble polyoxyethylene group and a water-insoluble polyoxypropylene group and a water-insoluble polyoxybutylene group, by changing a chain length of a hydrocarbon chain, by using a material having a branched chain in a hydrocarbon chain, by using a material having a double bond in a hydrocarbon chain, by using a material having a benzene ring or a naphthalene ring in a hydrocarbon chain, or by appropriately combining the above.

Second, examples include a hydrocarbon compound having 5 or more carbon atoms. From a viewpoint in which spreading on the surface of the liquid film is further enhanced in a state of fluid, the number of carbon atoms is preferably 100 or less, and more preferably 50 or less. The hydrocarbon compound, excluding polyorganosiloxane, is not limited to a straight chain, and may have a branched chain, in which the chain is not particularly limited to a saturated chain or an unsaturated chain. Moreover, the hydrocarbon compound may have a substituent such as ester and ether in an intermediate and an end thereof. Above all, the hydrocarbon compound in fluid at ordinary temperature is preferable and used alone. The hydrocarbon compound is preferably contained in 0.10 mass% or more and 5.0 mass% or less in terms of a content proportion (Oil Per Unit) to fibre mass. If the content proportion of the hydrocarbon compound is excessively large, the nonwoven fabric becomes sticky, and therefore the above-mentioned liquid film cleavage effect becomes hard to exhibit, and therefore such a case is not preferable. From this viewpoint, the content proportion (OPU) is preferably 1.0 mass% or less, more preferably 0.99 mass% or less, and further preferably 0.40 mass% or less. Moreover, if the content proportion of the hydrocarbon compound is excessively small, the liquid film cleavage effect becomes insufficient. From this viewpoint, the content proportion (OPU) is more preferably 0.15 mass% or more, and further preferably 0.20 mass% or more.

Examples of the hydrocarbon compound include oil or fat, such as natural oil or natural fat. Specific examples include palm oil, camellia oil, castor oil, coconut oil, corn oil, olive oil, sunflower oil, tall oil, and a mixture thereof.

Moreover, specific examples include fatty acid as represented by Formula (VII), such as caprylic acid, capric acid, oleic acid, lauric acid, palmitic acid, stearic acid, myristic acid, behenic acid, and a mixture thereof.

$$\mathbf{C_mH_n}\text{ - COOH} \qquad [VII]$$

In Formula [VII], m and n each independently are an integer of 1 or more. $C_mH_n$ herein designates a hydrocarbon group of the above-described fatty acid.

Examples of straight-chain or branched-chain, saturated or unsaturated, or substituted or unsubstituted polyhydric alcohol fatty acid ester or a mixture of polyhydric alcohol fatty acid ester include glycerol fatty acid ester or pentaerythritol fatty acid ester as represented by Formula (VIII-I) or (VIII-II), and specific examples include glyceryl tricaprylate, glyceryl tripalmitate and a mixture thereof. In addition, a certain amount of monoester, diester and trimester is typically included in the mixture of glycerol fatty acid ester or pentaerythritol fatty acid ester. Specific preferable examples of the glycerol fatty acid ester include a mixture of glyceryl tricaprylate and glyceryl tricapryate. Moreover, from a viewpoint of reducing the interfacial tension to obtain a higher spreading coefficient, polyhydric alcohol fatty acid

ester into which a polyoxyalkylene group is introduced in a degree at which water insolubility can be maintained may be used.

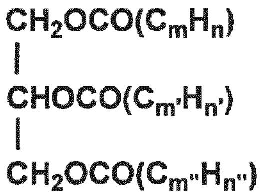

[VIII-I]

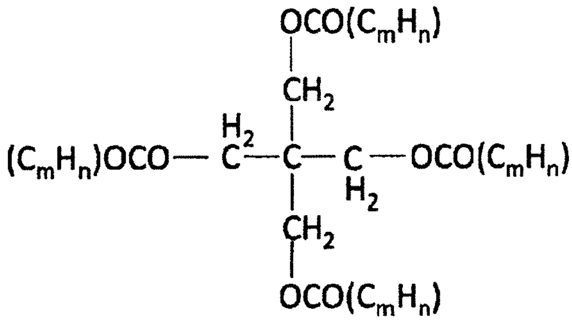

[VIII-II]

In Formulas [VIII-I] and [VIII-II], m, m', m", n, n' and n" each are independently an integer of 1 or more. A plurality of m or a plurality of n each may be identical to or different from each other. $C_mH_n$, $C_m'H_n'$ and $C_m''H_n''$ each herein designate a hydrocarbon group of the above-described fatty acid.

Examples of fatty acid or a fatty acid mixture in which straight-chain or branched-chain, saturated or unsaturated fatty acid forms polyol and ester with polyol having a large number of hydroxyl groups, and part of hydroxyl groups remain without being esterified, include a partially esterified product of glycerol fatty acid ester, sorbitan fatty acid ester or pentaerythritol fatty acid ester as represented by any of formulas in Formula (IX), any of formulas in Formula (X) or any of formulas in Formula (XI). Specific examples thereof include ethylene glycol monomyristate, ethylene glycol dimyristate, ethylene glycol palmitate, ethylene glycol dipalmitate, glyceryl dimyristate, glyceryl dipalmitate, glyceryl monooleate, sorbitan monooleate, sorbitan monostearate, sorbitan dioleate, sorbitan tristearyl, pentaerythritol monostearate, pentaerythritol dilaurate, pentaerythritol tristearate, and a mixture thereof. In addition, a certain quantity of a completely esterified compound is typically included in the mixture formed of the partially esterified product of glycerol fatty acid ester, sorbitan fatty acid ester or pentaerythritol fatty acid ester.

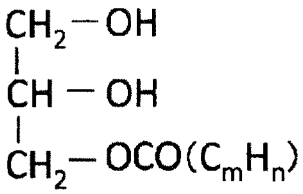

or

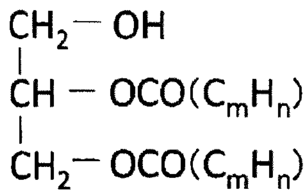

[IX]

In Formula [IX], m and n each are independently an integer of 1 or more. A plurality of m or a plurality of n each may be identical to or different from each other. $C_mH_n$ herein designates a hydrocarbon group of the above-described fatty acid.

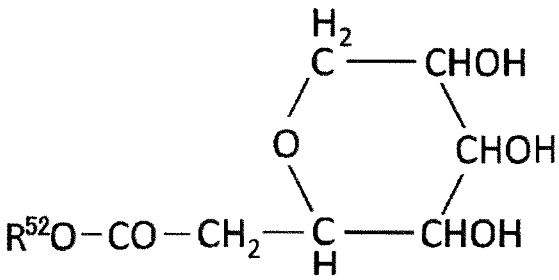

or

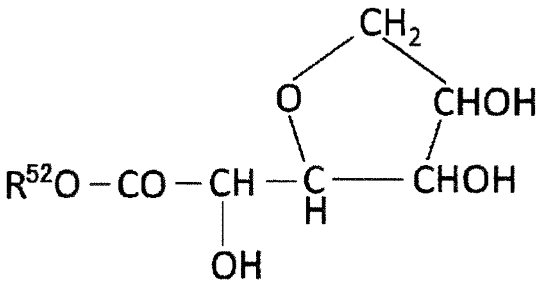

[X]

In the formula [X], $R^{52}$ designates a straight-chain or branched-chain, or saturated or unsaturated hydrocarbon group (alkyl group, alkenyl group, alkynyl group) having 2 or more and 22 or less carbon atoms. Specific examples include a 2-ethylhexyl group, a lauryl group, a myristyl group, a palmityl group, a stearyl group, a behenyl group, an oleyl group and a linoleic group.

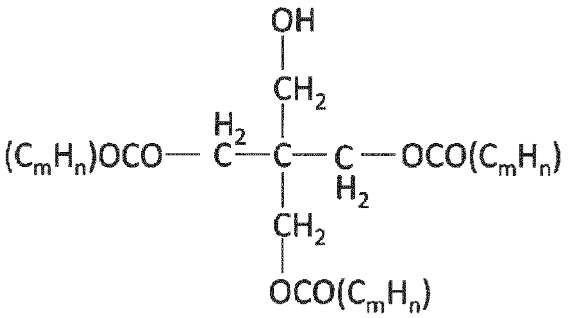

[XI]

or

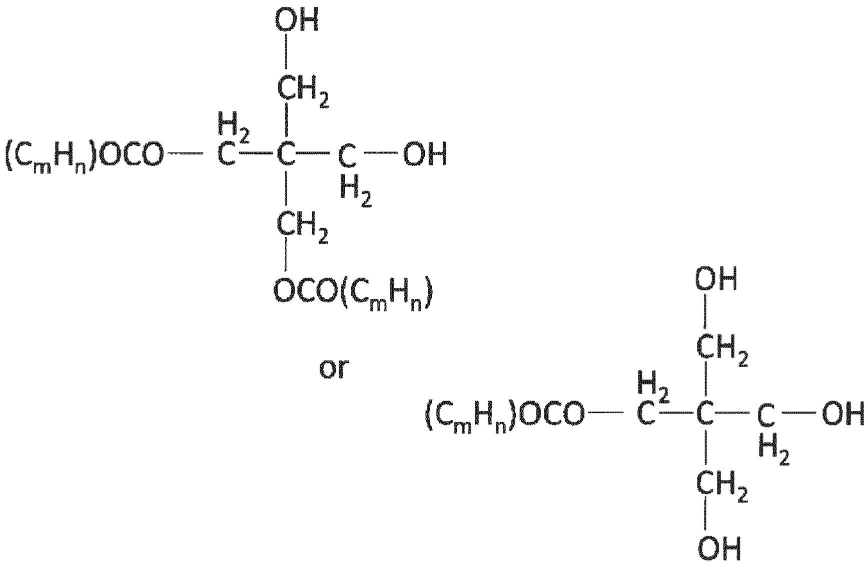

or

In Formula [XI], m and n each are independently an integer of 1 or more. A plurality of m or a plurality of n each may be identical to or different from each other. $C_mH_n$ herein designates a hydrocarbon group of the above-described

fatty acid.

Moreover, examples include sterol, phytosterol and a sterol derivative. Specific examples include cholesterol, sitosterol, stigmasterol and ergosterol, and a mixture thereof, each having a sterol structure of Formula (XII).

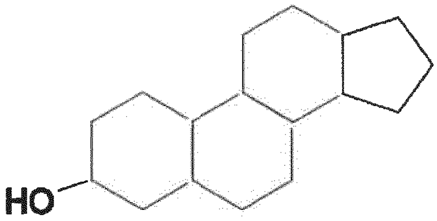

[XII]

Specific examples of alcohol include lauryl alcohol, myristyl alcohol, cetyl alcohol, stearyl alcohol, cetostearyl alcohol, behenyl alcohol and a mixture thereof, as represented by Formula (XIII).

$$C_mH_n - OH \qquad [XIII]$$

In Formula [XIII], m and n each are independently an integer of 1 or more. $C_mH_n$ herein designates a hydrocarbon group of the above-described alcohol.

Specific examples of fatty acid ester include isopropyl myristate, isopropyl palmitate, cetyl ethylhexanoate, triethylhexanoin, octyldodecyl myristate, ethylhexyl palmitate, ethylhexyl stearate, butyl stearate, myristyl myristate, stearyl stearate, cholesteryl isostearate and a mixture thereof, as represented by Formula (XIV).

$$C_mH_n - COO - C_mH_n \qquad [XIV]$$

In Formula [XIV], m and n each are independently an integer of 1 or more. Two pieces of $C_mH_n$ herein may be identical to or different from each other. $C_mH_n$ in $C_mH_n$-COO- designates a hydrocarbon group of each fatty acid described above. $C_mH_n$ in -COO$C_mH_n$ designates a hydrocarbon group derived from alcohol which forms ester.

Moreover, specific examples of wax include ceresin, paraffin, vaseline, mineral oil and liquid isoparaffin, as represented by Formula (XV).

$$C_mH_n \qquad \textbf{[XV]}$$

In Formula [XV], m and n each are independently an integer of 1 or more.

The spreading coefficient, the surface tension, the water solubility and the interfacial tension each mentioned above in the second embodiment can be set in a predetermined range, in the above-described hydrocarbon compound having the number of carbon atoms of 5 or more, for example, by introducing a small amount of hydrophilicity polyoxyethylene group thereinto at a degree at which water insolubility can be maintained, by introducing a polyoxypropylene group or polyoxybutylene group which is hydrophobic but can reduce the interfacial tension, by changing a chain length of a hydrocarbon chain, by using a material having a branched chain in a hydrocarbon chain, by using a material having a double bond in a hydrocarbon chain, by using a material having a benzene ring or a naphthalene ring in a hydrocarbon chain.

In the nonwoven fabric according to the present invention, in addition to the above-mentioned liquid film cleavage agent, other components may be contained thereinto, when necessary. Moreover, the liquid film cleavage agent in the first embodiment and the liquid film cleavage agent in the second embodiment may be used in combination in addition to an aspect of separate using. In this regard, the same rule applies also to the first compound and the second compound in the liquid film cleavage agent in the second embodiment.

Further, in the nonwoven fabric according to the present invention, when the liquid film cleavage agent or the phosphoric acid ester type anionic surfactant being contained thereinto is identified, the identifying method described in the above-described measuring method for the surface tension (γw) of the liquid film (the liquid having surface tension of 50 mN/m) can be applied.

Moreover, when a component of the liquid film cleavage agent is a compound in which a main chain has a siloxane chain, or a hydrocarbon compound in which the number of carbon atoms is 1 or more and 20 or less, a content proportion thereof (OPU) to fibre mass can be determined by dividing the content proportion of the liquid film cleavage agent by the fibre mass based on mass of a substance obtained by the above-mentioned analytical method.

The nonwoven fabric according to the present invention is formed into a product having high liquid permeability, irrespective of a thickness of the fibres or the interfibre distance. However, the nonwoven fabric according to the present

invention is particularly effective when fine fibres are used. If the fine fibres are used in order to form the nonwoven fabric having softer texture than usual, the interfibre distance is reduced, and a narrow interfibre region is increased. For example, ordinarily, in the case of the nonwoven fabric (fineness: 2.4 dtex) generally used, the interfibre distance is 120 μm, and a liquid film area proportion to be formed becomes about 2.6%. However, if the fineness is reduced to 1.2 dtex, the interfibre distance is 85 μm, and the liquid film area proportion is increased to about 7.8% at a degree as high as about 3 times the proportion in an ordinary nonwoven fabric. To the contrary, the liquid film cleavage agent according to the present invention positively cleaves frequently formed liquid films to reduce liquid remains. As mentioned later, the liquid film area proportion is expressed in terms of a liquid film area proportion to be calculated by image analysis from the surface of the nonwoven fabric, and has a strong correlation with a state of the liquid remains on an outermost surface of a surface material. Therefore, if the liquid film area proportion is reduced, the liquid in the vicinity of skin is eliminated, a comfort level after excretion is improved and thus the absorbent article which is comfortable to wear even after excretion is obtained. On the other hand, an amount of the liquid remains mentioned later means an amount of the liquid retained in the nonwoven fabric as a whole. If the liquid film area proportion is minimised, the liquid remains are reduced, although the reduction is not unconditionally proportional. Moreover, whiteness of the surface is expressed in terms of an L value mentioned later. With regard to the L value, a numerical value tends to increase by reduction of the liquid remains as induced by cleaving of the liquid film on the surface, in which the whiteness easily becomes visually conspicuous. In the nonwoven fabric containing the liquid film cleavage agent according to the present invention, even if the fibres are thinned, the liquid film area proportion and the amount of the liquid remains are reduced, and the L value can be increased, and therefore both a dry feeling and soft texture given by thinning the fibres can be satisfied at a high level. Moreover, the nonwoven fabric according to the present invention is used as the constituent member such as the surface material of the absorbent article to achieve a high dry feeling in the part in contact with skin, and inconspicuousness of dirt with the bodily liquid by visual whiteness, and therefore the absorbent article can be provided in which a worry about leakage can also be suppressed and the significant comfort to wear is realised.

In the nonwoven fabric containing such the liquid film cleavage agent, from a viewpoint of improving softness of texture, the interfibre distance in the nonwoven fabric is preferably 150 μm or less, and more preferably 90 μm or less. Moreover, from a viewpoint of suppressing liquid permeability from being adversely affected as caused by an excessively narrowed interfibre distance, a lower limit thereof is preferably 50 μm or more, and more preferably 70 μm or more. Specifically, the interfibre distance is preferably 50 μm or more and 150 μm or less, and more preferably 70 μm or more and 90 μm or less.

The fineness of the fibres in this case is preferably 3.3 dtex or less, and more preferably 2.4 dtex or less. Moreover, a lower limit thereof is preferably 0.5 dtex or more, and more preferably 1.0 dtex or more. Specifically, the fineness is preferably 0.5 dtex or more and 3.3 dtex or less, and more preferably 1.0 dtex or more and 2.4 dtex or less.

(Measuring method for interfibre distance)

An interfibre distance is determined by measuring a thickness of a measuring object nonwoven fabric and then applying a measured value to Expression (2).

First, a nonwoven fabric as a measuring object is cut to a piece of 50 mm in a longitudinal direction × 50 mm in a crosswise direction to prepare a cut piece of the nonwoven fabric.

A thickness of the cut piece is measured under 49 Pa pressure. A measurement environment is a temperature of 20±2°C and a relative humidity of 65±5%, and a microscope (VHX-1000, manufactured by KEYENCE Corporation) is used as a measuring instrument. First, an enlarged photograph of the cross section of the above-described nonwoven fabric is obtained. In the enlarged photograph, a piece having a known dimension is simultaneously photographed. A scale is aligned on the enlarged photograph of the cross section of the above-mentioned nonwoven fabric to measure the thickness of the nonwoven fabric. The operation described above is performed 3 times, and a mean value of 3 times measurement is taken as the thickness (mm) of the nonwoven fabric in a dry state. Further, in the case of a laminated product, a boundary is distinguished from a fibre diameter to calculate the thickness.

Next, the interfibre distance in the fibres which constitute the measuring object nonwoven fabric is determined by the formula based on assumption by Wrotnowski shown below. The formula based on the assumption by Wrotnowski is generally used upon determining the interfibre distance in the fibres which constitute the nonwoven fabric. According to the formula based on the presumption by Wrotnowski, an interfibre distance A (μm) is determined by the following Expression (2) by using a thickness h (mm) of a nonwoven fabric, a basis weight e ($g/m^2$) thereof, a fibre diameter d (μm) of fibres which constitute the nonwoven fabric and a fibre density p ($g/cm^3$) thereof. Further, when the nonwoven fabric has concavity and convexity, the interfibre distance is calculated by using a nonwoven fabric thickness h (mm) in a convex portion as a representative value.

With regard to the fibre diameter d (μm), 10 pieces of fibre cross sections of cut fibres are measured by using a scanning electron microscope (DSC6200, manufactured by Seiko Instruments Inc.), and a mean value thereof is taken as the fibre diameter.

The fibre density p (g/cm$^3$) is measured by using a density gradient tube in accordance with the measuring method of the density gradient tube method described in JIS L1015 Test methods for chemical staple fibres.

With regard to the basis weight e (g/m$^2$), the measuring object nonwoven fabric is cut into a piece having a predetermined size (0.12 m × 0.06 m), and after mass measurement, the basis weight is determined by calculation in accordance with a Expression "mass / area determined from the predetermined size = basis weight (g/m$^2$)."

Interfibre distance

$$A = \frac{d\sqrt{\pi \rho h \times 10^3}}{2\sqrt{e}} - d \qquad (\mu m) \qquad (2)$$

(Measuring method for fineness of constituent fibres)

Fineness is calculated by measuring a cross-sectional shape of a fibre by an electron microscope to measure a cross-sectional area of the fibre (the cross-sectional area of each resin component in case of a fibre formed of a plurality of resins), and simultaneously specifying a kind of the resin (also an approximate component ratio in the case of a plurality of resins) by DSC (differential scanning calorimeter) to identify specific gravity. For example, if a staple constituted of only PET is used, a cross section is first observed to calculate a cross-sectional area. Then, the fibre is measured by DSC to identify that the fibre is constituted of a single component from a melting point or peak shape, and the component is a PET core. Then, the fineness is calculated by calculating the mass of the fibre by using the density of the PET resin and the cross-sectional area.

As the fibres which constitute the nonwoven fabric according to the present invention, the fibres ordinarily used in this kind of article can be adopted without particular restriction. Specific examples include various fibres such as thermally fusible sheath-core type composite fibres, thermally extensible fibres, thermally non-extensible fibres, thermally shrinkable fibres, thermally non-shrinkable fibres, three-dimensionally crimped fibres, potentially crimpable fibres and hollow fibres. In particular, the fibres preferably have a thermoplastic resin. Moreover, the thermally non-extensible fibres and the thermally non-shrinkable fibres are preferably thermally fusible. The sheath-core type composite fibres may be of a concentric sheath-core type, an eccentric sheath-core type, a side-by-side type or a deformed type, and are preferably of a concentric sheath-core type. In production of the fibres and the nonwoven fabric, the liquid film cleavage agent, or the liquid film cleavage agent and the phosphoric acid ester type anionic surfactant may be incorporated in the fibres in any step. For example, the liquid film cleavage agent, or a mixture of the liquid film cleavage agent and the phosphoric acid ester type anionic surfactant may be blended in a spinning oil for the fibres to be ordinarily used during spinning of the fibres, and the resultant mixture may be applied onto the fibres, or the liquid film cleavage agent, or the mixture of the liquid film cleavage agent and the phosphoric acid ester type anionic surfactant may be blended in a finishing oil for the fibres before or after stretching the fibres, and the resultant mixture may be applied onto the fibres. Moreover, the liquid film cleavage agent or the phosphoric acid ester type anionic surfactant may be blended in a fibre treating agent to be ordinarily used for production of the nonwoven fabric, and the resultant mixture may be coated on the fibres, or the fibres after forming the nonwoven fabric.

The nonwoven fabric according to the present invention contains the liquid film cleavage agent, or the phosphoric acid ester type anionic surfactant further therewith, and therefore is excellent in suppressing the liquid remains in corresponding to various fibre structures. Therefore, even if a large amount of liquid is poured on the nonwoven fabric, a permeation passage of the liquid between the fibres is ensured at all times, and the nonwoven fabric is excellent in the liquid permeability. Thus, various functions can be provided for the nonwoven fabric without being restricted by problems of the interfibre distance and liquid film formation. For example, the nonwoven fabric may be formed of one layer or a plurality of layers of two or more layers. Moreover, the nonwoven fabric may have a flat shape, a concavo-convex shape on one side or both sides, or a shape having a variety with the respect to the fibre basis weight or the fibre density. Further, a width of options is extended also with regard to a combination with an absorbent body. Moreover, when the nonwoven fabric is formed of the plurality of layers, the liquid film cleavage agent may be contained in all the layers, or in part of the layers. The liquid film cleavage agent is preferably contained at least in the layer on a side in which the liquid is directly received. For example, when the nonwoven fabric according to the present invention is incorporated as the topsheet in the absorbent article, the liquid film cleavage agent is preferably contained at least in a layer on a skin-contact surface side.

In the nonwoven fabric according to the present invention, the liquid film cleavage agent is preferably localised at least around part of the points that fibres are entangled to each other or the points that fibres are fusion bonded to each other. The term "localisation" of the liquid film cleavage agent herein mean a state in which the liquid film cleavage agent is not evenly attached to the whole surface of the fibres which constitute the nonwoven fabric, but the liquid film cleavage agent is locally attached thereto in the vicinity of the points that fibres are entangled to each other or the points

that fibres are fusion bonded to each other rather than each surface of the fibres. Specifically, the term can be defined in which a concentration of the liquid film cleavage agent in the vicinity of the points that fibres are entangled to each other or the points that fibres are fusion bonded to each other is higher in comparison with the surface of the fibres (surface of the fibres between the entangled points or between fusion bonded points). On the occasion, the liquid film cleavage agent existing in the vicinity of the points that fibres are entangled to each other or the points that fibres are fusion bonded to each other may be attached thereto so as to partially cover an interfibre space centring on the fibre entangled points or the fibre fusion bonded points. As the concentration of the liquid film cleavage agent in the vicinity of the points that fibres are entangled to each other or the points that fibres are fusion bonded to each other is preferably as higher as possible. The concentration is changed depending on a kind of the liquid film cleavage agent to be used, a kind of the fibres to be used, or an effective component proportion when the agent is mixed with other agents, and therefore cannot be unambiguously defined, but from a viewpoint of exhibiting the above-mentioned liquid film cleavage effect, the concentration can be appropriately determined.

Localisation of the liquid film cleavage agent facilitates to further develop the liquid film cleavage effect. That is, the area in the vicinity of the points that fibres are entangled to each other or the points that fibres are fusion bonded to each other is a place in which the liquid film is particularly easily formed, and therefore a larger amount of the liquid film cleavage agent exists in the place to easily directly interact with the liquid film.

Such localisation of the liquid film cleavage agent exists preferably in 30% or more, more preferably in 40% or more, and further preferably in 50% or more of the places in the vicinity of the points that fibres are entangled to each other or the points that fibres are fusion bonded to each other of the whole nonwoven fabric. In the nonwoven fabric, in a place in which a distance between the fibre entangled points or a distance between fibre fusion bonded points is comparatively short, the interfibre space is small, and the liquid film is particularly easily formed therein. Therefore, if the liquid film cleavage agent is selectively localised in the vicinity of the points that fibres are entangled to each other or the points that fibres are fusion bonded to each other in a place in which the interfibre space is small, the liquid film cleavage effect is particularly effectively developed, and such a case is preferable. Moreover, in the case of the selective localisation as described above, it is preferable to adjust a covering ratio of the liquid film cleavage agent, in such a manner that the covering ratio of the liquid film cleavage agent to a comparatively small interfibre space is increased, and the covering ratio of the liquid film cleavage agent to a comparatively large interfibre space is reduced. Thus, while the liquid permeability is retained in the nonwoven fabric, the cleavage effect can be effectively developed in a part in which capillary force is high and the liquid film is easily formed, and an effect on reducing the residual liquid in the whole nonwoven fabric is improved. The term "comparatively small interfibre space" herein means an interfibre space having the interfibre distance of 1/2 or less relative to the interfibre distance determined in (Measuring method for interfibre distance) mentioned above.

(Confirmation method for localised state of liquid film cleavage agent)

The above-described localised state of the liquid film cleavage agent can be confirmed by the following method.

First, a nonwoven fabric is cut into a piece of 5 mm × 5 mm, and the piece is attached on a sample stage by using a carbon tape. The sample stage is placed in a scanning electron microscope (S4300SE/N, manufactured by Hitachi, Ltd.) in a state of no vapour deposition and is made into a low vacuum state or a vacuum state. Localisation is detected by using an annular reflection electron detector (attachment). As the atomic number is larger, a reflection electron is further easily emitted. Thus, a part coated with the liquid film cleavage agent containing a large number of oxygen atoms or silicon atoms appears white, in which the oxygen atoms or the silicon atoms each have the larger atomic number in comparison with carbon atoms or hydrogen atoms which mainly constitute polyethylene (PE), polypropylene (PP) or polyester (PET). Therefore, the localised state can be confirmed by whiteness. Further, the whiteness is increased as the atomic number is larger or an attached amount is larger.

Moreover, upon producing the nonwoven fabric according to the present invention, a method ordinarily applied to this kind of article can be adopted. For example, as a method of forming a fibre web, a carding method, an air-laid method or a spunbond method can be applied. As a method of processing the fibre web into the nonwoven fabric, various methods of forming the nonwoven fabrics to be ordinarily applied can be adopted, such as spunlacing, needle punching, chemical bonding and embossing in a dot form. Above all, from a viewpoint of texture, the nonwoven fabric is preferably an air-through nonwoven fabric or a spunbond nonwoven fabric. "Air-through nonwoven fabric" herein means a nonwoven fabric which is produced through a step of blowing a fluid at 50°C or higher, for example, a gas and a water vapour onto the web or the nonwoven fabric (air-through processing step). Moreover, "spunbond nonwoven fabric" means a laminated nonwoven fabric which is produced by the spunbond method. The nonwoven fabric includes not only one which is produced only in the above step, but also one which is produced by adding the above step to a nonwoven fabric which is produced by any other method, or one which is produced by performing a step of some kind after the above step. Moreover, the nonwoven fabric according to the present invention is not limited to one formed of the air-through nonwoven fabric solely or the spunbond nonwoven fabric solely, but also includes one formed as a composite of the air-through

nonwoven fabric or the spunbond nonwoven fabric with a fibre sheet or a film material such as other nonwoven fabrics.

In the method for producing the nonwoven fabric according to the present invention, when the liquid film cleavage agent is applied thereon after the nonwoven fabric is formed as mentioned above, specific examples include a method in which a raw material nonwoven fabric is immersed into a solution containing a liquid film cleavage agent. Specific examples of the solution include a solution in which a liquid film cleavage agent is diluted with a solvent (hereinafter, this solution is also referred to as a liquid film cleavage agent solution.). Moreover, specific examples of another method include a method in which a liquid film cleavage agent alone or a solution containing the liquid film cleavage agent is coated onto a raw material nonwoven fabric. Further, a phosphoric acid ester type anionic surfactant may be mixed with the solution containing the liquid film cleavage agent. In this case, a content proportion of the liquid film cleavage agent to the phosphoric acid ester type anionic surfactant is preferably as mentioned above. As the solvent, a solvent in which the liquid film cleavage agent having a significantly small water solubility can be moderately dissolved, or dispersed and emulsified so as to easily coat the agent onto the nonwoven fabric can be used without particular restriction. Specific examples of the solvent which dissolves the agent include an organic solvent such as ethanol, methanol, acetone and hexane, or when the agent is converted into an emulsified liquid, water can also be obviously used as the solvent or a dispersion medium, and specific examples of an emulsifying agent used upon emulsifying the agent include various surfactants including alkyl phosphoric acid ester, fatty acid amide, alkyl betaine and alkyl sodium sulphosuccinate. Further, the raw material nonwoven fabric means a nonwoven fabric before the liquid film cleavage agent is applied thereon, and as a production method thereof, the production method to be ordinarily applied as mentioned above can be applied without particular restriction.

As the method of coating the agent to the raw material nonwoven fabric, the method applied in this production method for the nonwoven fabric can be adopted without particular restriction. Specific examples include coating by a spray, coating by a slot coater, coating by roll transfer such as a gravure system, a flexographic system and a gate roll system, and coating by a dipping system.

From a viewpoint of the above-mentioned localisation of the liquid film cleavage agent in the vicinity of the points that fibres are entangled to each other or the points that fibres are fusion bonded to each other, the liquid film cleavage agent is preferably applied on the raw material nonwoven fabric after the nonwoven fabric is formed, and a method of coating the liquid film cleavage agent to the raw material nonwoven fabric, and not by immersion, is further preferable. Among the coating methods, a coating method by the flexographic system is particularly preferable from a viewpoint of further clearly achieving localisation of the liquid film cleavage agent.

Moreover, as the raw material nonwoven fabric, various nonwoven fabrics can be used without particular restriction. In particular, from a viewpoint of keeping localisation of the liquid film cleavage agent, a nonwoven fabric in which the fibre entangled points are thermally fusion bonded or thermally compressed is preferable, and use of the nonwoven fabric prepared by thermally bonding the fibres with each other by the above-mentioned air-through processing or thermal embossing is further preferable.

The liquid film cleavage agent, upon attaching the liquid film cleavage agent to the raw material nonwoven fabric or the fibres, is preferably used in the form of the solution in which the liquid film cleavage agent is diluted with the solvent as mentioned above. The solution containing the liquid film cleavage agent can also be prepared separately as a single solution as a fibre treating agent. "Fibre treating agent" described herein means an agent in which an oily liquid film cleavage agent having a significantly small water solubility is formed into a state in which applying processing is facilitated on the raw material nonwoven fabric or the fibres. In the fibre treating agent for applying the liquid film cleavage agent thereon, a content proportion of the liquid film cleavage agent is preferably 50 mass% or less based on mass of the fibre treating agent. Thus, the fibre treating agent can be formed into a state in which the liquid film cleavage agent as an oily component is stably emulsified in the solvent. From a viewpoint of stable emulsification, a content proportion of the liquid film cleavage agent is more preferably 40 mass% or less, and further preferably 30 mass% or less, based on mass of the fibre treating agent. Moreover, from a viewpoint of realising the above-mentioned localisation of the liquid film cleavage agent in the nonwoven fabric by locomotion of the liquid film cleavage agent on the fibres at moderate viscosity after applying, the proportion is preferably adjusted to the above-described content proportion. From a viewpoint of developing a sufficient liquid film cleavage effect, a content proportion of the liquid film cleavage agent is preferably 5 mass% or more, more preferably 15 mass% or more, and further preferably 25 mass% or more, based on mass of the fibre treating agent. In addition, the fibre treating agent containing the liquid film cleavage agent may also contain other agents within the range in which effect of the liquid film cleavage agent is not adversely affected. For example, the fibre treating agent may contain the above-mentioned phosphoric acid ester type anionic surfactant. A content proportion of the liquid film cleavage agent to the phosphoric acid ester type anionic surfactant in this case is preferably as mentioned above. In addition thereto, the fibre treating agent may contain an antistatic agent or an antifriction agent used upon processing the fibres, a hydrophilising agent for providing the nonwoven fabric with moderate hydrophilicity, an emulsifying agent for emulsification stability.

Specific examples of the nonwoven fabric according to the present invention include a concavo-convex nonwoven fabric which is constituted with including thermoplastic fibres and has a first surface and a second surface

positioned on a side opposite thereto, and, at least on the first surface, has the concavo-convex shape having a plurality of convex portions projecting on a side of the first surface, and concave portions positioned between the convex portions.

In the following, a specific example of the nonwoven fabric having a concavo-convex shape will be described.

Specific examples include a nonwoven fabric shown in FIG. 3 in which thermally shrinkable fibres are utilised (first aspect). A nonwoven fabric 10 shown in FIG. 3 comprises two layers: an upper layer 11 on a side of a top surface 1A (skin-contact surface upon applying as the topsheet); and a lower layer 12 on a side of a bottom surface 1B (skin non-contact surface upon applying as the topsheet). Moreover, embossing (compression) is applied thereto from the top surface 1A in a thickness direction, and the two layers are bonded (a part subjected to embossing is referred to as an embossed concave portion (concave bonding portion) 13). The lower layer 12 is a layer in which thermal shrinkage of the thermally shrinkable fibres is developed. The upper layer 11 is a layer containing thermally non-shrinkable fibres, and the thermally non-shrinkable fibres are partially bonded in the concave bonding portion 13. The thermally non-shrinkable fibres include, without limiting to fibres that are not shrunk at all by heating, fibres that are shrunk at a degree at which thermal shrinkage of the thermally shrinkable fibres in the lower layer 12 is not adversely affected. From a viewpoint of forming the nonwoven fabric by heat, as the thermally non-shrinkable fibres, thermally non-shrinkable and thermally fusible fibres are preferable.

For example, the nonwoven fabric 10 can be produced by the raw material and the production method described in the paragraphs {0032} to {0048} of JP-A-2002-187228. For example, in this production, embossing applied to a laminate between the upper layer 11 and the lower layer 12 from a side of the upper layer 11, and then the thermally shrinkable fibres are thermally shrunk by heat treatment. At this time, adjacent embossed parts are pulled to each other by shrinking of the fibres, and an interval with each other is shortened. Owing to this deformation, the fibres in the upper layer 11 rise on a side of the top surface 1A with the embossed concave portion 13 as a base point to form a convex portion 14. Alternatively, the upper layer is laminated on the lower layer 12 in a state of extending the lower layer 12 in which thermal shrinkage is developed, and the above-described embossing is applied thereto. Then, when an extended state of the lower layer 12 is released, the side of the upper layer 11 rises on the side of the top surface 1A to form the convex portion 14. The embossing can be performed by a method to be ordinarily applied, such as heat embossing and ultrasonic embossing. Moreover, with regard to bonding of both layers, a bonding method using an adhesive may be applied.

In the thus produced nonwoven fabric 10, the upper layer 11 is compressed and bonded to a place on the side of the lower layer 12 in the embossed concave portion (concave bonding portion) 13. The embossed concave portion 13 is formed on the nonwoven fabric 10 in a plane direction in a scattered dot manner, and a part surrounded by the embossed concave portion 13 is the above-mentioned convex portion 14 formed by rising of the upper layer 11. The convex portion 14 has a three-dimensional solid shape, and forms a dome shape, for example. The convex portion 14 formed by the production method as described above is in a state in which the fibre density is lower than the fibre density in the lower layer 12. An inside of the convex portion 14 may be filled with the fibres as shown in FIG. 3, or may have a hollow portion formed in which the upper layer 11 and the lower layer 12 are separated. Arrangement of the embossed concave portion 13 and the convex portion 14 can be arbitrarily formed, for example, formed into lattice arrangement. Specific examples of the lattice arrangement include arrangement in which a plurality of rows formed of a plurality of embossed concave portions 13 are aligned, and an interval of the embossed concave portions 13 in each row is deviated by a half pitch between the adjacent rows. Moreover, a plane shape of the embossed concave portion 13 may be formed, when the shape is formed in a dot form, in a circle, an elliptical form, a triangular form, a rectangular form or any other polygonal form, and can be appropriately arbitrarily set. Moreover, the embossed concave portion 13 may be formed in a linear form in addition to the dot form.

The nonwoven fabric 10 has a concavo-convex surface having the convex portion 14 and the embossed concave portion 13 on the side of the top surface 1A, and therefore is excellent in shape recoverability when the nonwoven fabric 10 is extended in the plane direction, and compressive deformability when the nonwoven fabric 10 is compressed in the thickness direction. Moreover, the nonwoven fabric 10 is formed into a comparatively bulky nonwoven fabric by rising of the fibres in the upper layer 11 as described above. Thus, a user in touching the nonwoven fabric 10 can feel soft and gentle texture. Moreover, in an absorbent article comprising the nonwoven fabric 10 as a topsheet in which a top surface 10A is applied as the skin-contact surface and the bottom surface 1B is applied as the skin non-contact surface, the skin-contact surface side becomes excellent in air-permeability by the concavo-convex shape having the convex portions 14 and the embossed concave portions 13.

Moreover, liquid remains are reduced in the nonwoven fabric 10 by the effect of the liquid film cleavage agent, or cooperative effect of the liquid film cleavage agent and the phosphoric acid ester type anionic surfactant as mentioned above. Thus, the liquid permeability produced by utilising the concavo-convex surface and a dense embossed part can be further improved.

In addition, the nonwoven fabric 10 may have further other layers without limiting to the double-layered structure of the upper layer 11 and the lower layer 12. For example, a monolayer or a plurality of layers may be arranged between the upper layer 11 and the lower layer 12, or the monolayer or the plurality of layers may be arranged on the side of the

top surface 10A or the side of the bottom surface 10B in the nonwoven fabric 10. The monolayer or the plurality of layers may be a layer having the thermally shrinkable fibres, or a layer having the thermally non-shrinkable fibres.

As other specific examples of the nonwoven fabric according to the present invention having the concavo-convex shape, nonwoven fabrics 20, 30, 40, 50, 60 and 70 (second to seventh aspects) are shown below.

First, as shown in FIG. 4, a nonwoven fabric 20 in the second aspect has a double-layered structure having a hollow portion 21. All layers contain thermoplastic fibres. The nonwoven fabric 20 has a bonding portion 22 in which a first nonwoven fabric 20A and a second nonwoven fabric 20B are partially thermally fusion bonded. A non-bonding portion 24 surrounded by the bonding portions 22 has a large number of convex portions 23 in which the first nonwoven fabric 20A is projected in a direction apart from the second nonwoven fabric 20B, with the hollow portion 21 inside thereof. The bonding portion 22 is a concave portion positioned between the adjacent convex portions 23 and 23 to constitute concavity and convexity of a first surface 1A in cooperation with the convex portions 23. The nonwoven fabric 20 can be formed by a method to be ordinarily applied. For example, the first nonwoven fabric 20A is imparted to a concavo-convex shape by engagement of two concavo-convex rolls, and then the second nonwoven fabric is laminated thereon to obtain the nonwoven fabric 20. From a viewpoint of shaping the nonwoven fabric by engagement of the concavo-convex rolls, both of the first nonwoven fabric 20A and the second nonwoven fabric 20B preferably contain thermally non-extensible and thermally non-shrinkable and thermally fusible fibres.

For example, when the nonwoven fabric 20 is laminated on an absorbent body as a topsheet in which the first surface 1A is directed toward a skin-contact surface side, and used, the nonwoven fabric 20 is excellent in liquid permeability from a side of the first surface 1A to a side of the second surface 2B. Specifically, liquid permeation through the hollow portion 21 is excellent. Moreover, a wearer's body pressure is applied to the convex portions 23, and the liquid in the convex portion 23 directly migrates to a second nonwoven fabric 3. Thus, liquid remains on the side of the first surface 1A are small. Such effect may be sustainably exhibited at a higher level by the effect of the liquid film cleavage agent, or cooperative effect of the liquid film cleavage agent and the phosphoric acid ester type anionic surfactant as mentioned above. That is, even if long-time use or a large amount of excretion is experienced, a permeation passage of the liquid is ensured by liquid film cleaving, and therefore the liquid permeability as described above can be sufficiently exhibited.

Next, as shown in FIGs. 5(A) and 5(B), a nonwoven fabric 30 in the third aspect contains thermoplastic fibres, and has a first fibre layer 301 having a concavo-convex shape for both sides. FIG. 5(A) shows a nonwoven fabric 30A having a monolayer structure consisting of the first fibre layer 301. FIG. 5(B) shows a nonwoven fabric 30B having a double-layered structure having a first fibre layer 310 and a second fibre layer 302 bonded along a side of a second surface 1B of the first fibre layer 301. In the following, each nonwoven fabric will be specifically described.

In the nonwoven fabric 30A (first fibre layer 301) shown in FIG. 5(A), a first projecting portion 31 projecting to a first surface 1A and a second projecting portion 32 projecting on a side of a second surface 1B are alternately continuously arranged in different intersecting directions in a planar view of the nonwoven fabric 30A. The first projecting portion 31 and the second projecting portion 32 each have an internal space opened on a side of each opposite surface, and the parts form concave portions 33 and 34 on the surface. Thus, the first surface 1A has a concavo-convex shape of the first projecting portion 31 and the concave portion 34. Moreover, the second surface 1B has a concavo-convex shape of the second projecting portion 32 and the concave portion 33. Moreover, the nonwoven fabric 30A has a wall portion 35 which connects the first projecting portion 31 with the second projecting portion 32. The wall portion 35 forms a wall to each internal space in the first projecting portion 31 and the second projecting portion 32, and has annular structure in a plane direction. Fibres which constitute the wall portion 35 has fibre orientation properties in all points of the annular structure in a direction connecting the first projecting portion 31 and the second projecting portion 32. Thus, resilience is generated in the wall portion. As a result, the nonwoven fabric 30A has moderate cushioning properties, and even if a pressure is applied thereto, the nonwoven fabric 30A is excellent in recoverability, and crush in each internal space can be avoided. Moreover, dispersibility to a body pressure is high and a contact area is also suppressed by projections on both surfaces, and therefore the nonwoven fabric 30A is excellent in soft texture and liquid-backflow prevention properties. The nonwoven fabric 30A can be adopted as a topsheet of an absorbent article with any one of the surfaces as a skin-contact surface side, and can provide the absorbent article with the moderate cushioning properties, the soft texture and the excellent liquid-backflow prevention properties.

In the nonwoven fabric 30B shown in FIG. 5(B), the second fibre layer 302 is arranged and bonded along the concavo-convex shape on a side of the second surface 1B of the above-mentioned first fibre layer 301. In the nonwoven fabric 30B, the first surface 1A is typically used as a skin-contact surface. On a side of the first surface 1A of the nonwoven fabric 30B, the concavo-convex shape of the first projecting portion 31 and the concave portion 34 of the first fibre layer 301 is spread, and the wall portion 35 having the annular structure between the first projecting portion 31 and the concave portion 32 is arranged. Accordingly, the nonwoven fabric 30B also has the fibre orientation properties of the above-mentioned first fibre layer 301, and thus resilience is generated in the wall portion, and the nonwoven fabric 30B is excellent in recoverability of the concavo-convex shape.

In addition thereto, in the nonwoven fabric 30B, shaping of a fibre web, formation of the nonwoven fabric, and

bonding of both layers are performed by hot-air processing in an air-through step, and therefore the nonwoven fabric 30B is formed to be bulky and low in a basis weight as a whole. In particular, bonding of both the fibre layers 301 and 302 is performed by thermal fusion of the fibres with each other by hot air, and therefore a gap is formed between the fibres in the bonded part between the fibre layers, and a liquid passing rate is high even in the concave portion 32 serving as a bonding portion. Moreover, the nonwoven fabric 30B has, on the side of the second surface 1B in a top portion of the first projecting portion 31 in the first fibre layer 301, a part 36 in which a fibre density in the second fibre layer 302 is lower than a fibre density in the first fibre layer 301 and in other parts of the second fibre layer 302. The existence of the part 36 having a lower fibre density facilitate denting of the first projecting portion 31 in the first fibre layer 301 even at a low load, and therefore the cushioning properties of the nonwoven fabric 30B is improved. When the nonwoven fabric 30B is adopted as the topsheet of the absorbent article, the side of the first surface 1A (namely, the side of the first fibre layer 301) is preferably applied as the skin-contact surface side.

Also in the nonwoven fabric 30 (30A and 30B), a permeation passage of the liquid is ensured at all times by the effect of the liquid film cleavage agent, or cooperative effect of the liquid film cleavage agent and the phosphoric acid ester type anionic surfactant as mentioned above. Thus, a width of design with regard to the fibre diameter and the fibre density is extended.

In production of the nonwoven fabric 30 (30A and 30B), for example, air-through processing can be adopted in which multi-stage hot-air processing is applied to the fibre web while a hot-air temperature and an air speed are controlled. For example, in the nonwoven fabric 30A (first fibre layer 301), the production method described in the paragraphs {0031} and {0032} of JP-A-2012-136790 can be applied. Moreover, as a support on which the web is imparted to a concavo-convex shape, the support preferably has a solid projecting portion and an opening portion. For example, the supports shown in FIGs. 1 and 2 of JP-A-2012-149370 or the supports shown in FIGs. 1 and 2 of JP-A-2012-149371 can be used. Moreover, the nonwoven fabric 30B (laminated nonwoven fabric of the first fibre layer 301 and the second fibre layer 302) can be produced by laminating the fibre web serving as the second fibre layer 302 in the air-through step of the first fibre layer 301. For example, the production method described in the paragraphs {0042} to {0064} of JP-A-2013-124428 can be applied. From a viewpoint of shaping the nonwoven fabrics 30A and 30B by air-through processing, both of the first fibre layer 301 and the second fibre layer 302 are preferably thermally non-extensible and thermally non-shrinkable and thermally fusible fibres.

Next, as shown in FIG. 6, a nonwoven fabric 40 in the fourth aspect is formed of one layer containing thermoplastic fibres, and has a shape in which a plurality of hemicylindrical convex portions 41 and a plurality of concave portions 42 arranged along side edges of the convex portions 41 are alternately arranged on a side of a first surface 1A. Concave bottom portions 43 formed of fibres of the nonwoven fabric are arranged on a lower side of the concave portions 42. A fibre density of the concave bottom portions 43 is smaller than that of the convex portions 41. In the nonwoven fabric 30, another fibre layer 45 may be partially laminated on the convex portion 41 (see FIG. 7). If the nonwoven fabric 40 is assembled into an absorbent article as a topsheet in which a side of the first surface 1A is applied as a skin-contact surface side, a liquid received by the convex portion 41 easily migrates to the concave portion 42, and easily migrates to a side of the second surface 1B in the concave portion 43. Thus, liquid remains are small, and stickiness on skin is suppressed.

Also in the nonwoven fabric 40, a permeation passage of the liquid is ensured at all times by the above-mentioned effect of the liquid film cleavage agent, or the liquid film cleavage agent and the phosphoric acid ester type anionic surfactant. Thus, a width of design with regard to a fibre diameter and the fibre density is extended.

Such a nonwoven fabric 40 can be formed by moving fibres by spraying a fluid such as hot air to a part to be formed into the concave portion 42 relative to a fibre web. Thus, the fibre density in the concave bottom portion 43 can be reduced in comparison with a periphery thereof.

Next, as shown in FIG. 8, a nonwoven fabric 50 in the fifth aspect has a concavo-convex structure in which stripe-shaped protruded portions 51 and recessed portions 52 which are extended in one direction (Y direction) are alternately arranged. Moreover, the concavo-convex structure can be divided, in a thickness direction of the nonwoven fabric sheet 50, equally into three of a top region 50A, a bottom portion region 50B, and a side region 50C positioned therebetween.

The nonwoven fabric 50 has a plurality of thermally fusion bonded portions 55 in intersections among constituent fibres 54. As shown in FIG. 9, focusing on one constituent fibre 54, the constituent fibre 54 has, between adjacent fusion bonded portions 55, a large diameter portion 57 interposed between two small diameter portions 56 each having a small fibre diameter. Thus, flexibility of the nonwoven fabric 50 is improved, and texture thereof becomes satisfactory. Moreover, a contact area with skin is reduced in a fibre unit, and a better dry feeling is obtained. Moreover, from a viewpoint of flexibility, a change point 58 from the small diameter portion 56 to the large diameter portion 57 is preferably within the range of 1/3 of an interval T between adjacent fusion bonded portion 55 and 55, in which the change point 58 is close to the fusion bonded portion 55 (range of T1 and T3 in FIG. 9). Further, a plurality of combinations of the small diameter portion 56 and the large diameter portion 57 interposed therebetween may exist in the interval T. A configuration of the small diameter portion 56 and the large diameter portion 57 in such a constituent fibre is formed by the fibres being

stretched upon gullet stretching processing for forming the protruded portion 51 and the recessed portion 52. As the fibres used on the above occasion, the fibres having a high stretching degree are preferable. Specific examples include thermally extensible fibres in which a crystalline state of a resin is changed by heating and a length is elongated, as obtained through the processing step described in the paragraph {0033} of JP-A-2010-168715.

Further, from a viewpoint of liquid permeability, the nonwoven fabric 50 is preferably formed in such a manner that a hydrophilic degree of the small diameter portion is smaller than a hydrophilic degree of the large diameter portion. This difference with the respect to the hydrophilic degree can be formed by incorporating a stretchable component (hydrophobic component) into the fibre treating agent which is attached to the fibres. In particular, the stretchable component and a hydrophilic component are preferably contained therein. Specifically, when the fibres are stretched by the above-described gullet stretching processing, the stretchable component is spread in the small diameter portion 35 being formed by stretching to generate a difference in the hydrophilic degree between the small diameter portion and the large diameter portion. In the large diameter portion, the hydrophilic component which is hard to spread stays therein, in which the hydrophilic degree becomes higher in comparison with the small diameter portion. Specific examples of the stretchable component include a silicone resin having a low glass transition point and flexibility in molecular chains, and as the silicone resin, polyorganosiloxane having a Si-O-Si chain as a main chain is preferably used.

In addition thereto, from a viewpoint of the above-described liquid permeability, in the nonwoven fabric 50, a fibre density in a side wall region 30C is preferably lower than a fibre density in a top region 30A and a bottom portion region 30B.

Also in the nonwoven fabric 50, a permeation passage of the liquid is ensured at all times by the above-mentioned effect of the liquid film cleavage agent, or the liquid film cleavage agent and the phosphoric acid ester type anionic surfactant. Thus, a width of design with regard to the fibre diameter and the fibre density is extended.

The nonwoven fabric 50 may be used alone, may be bonded with a flat fibre layer into a laminated nonwoven fabric, or may be laminated on a fibre layer having a concavo-convex shape into a laminated nonwoven fabric unified along the concavo-convex shape. For example, the nonwoven fabric 50 may be laminated on the second nonwoven fabric in the nonwoven fabric 20 in the second aspect (FIG. 4), or laminated on the nonwoven fabric 30A in the third aspect (FIG. 5(A)) or the nonwoven fabric 40 in the fourth aspect (FIG. 6 or FIG. 7).

Next, a nonwoven fabric 60 in the sixth aspect has a concavo-convex shape with thermally extensible fibres. As shown in FIG. 10, the concavo-convex shape is formed on a side of a first surface 1A. On the other hand, a shape on a side of a second surface 1B is flat, or has a significantly smaller degree of a concavo-convex shape in comparison with the side of the first surface 1A. Specifically, the concavo-convex shape on side of the first surface 1A has a plurality of convex portions 61 and linear concave portions 62 surrounding the convex portions 61. The concave portion 62 has a compression adhesion portion in which constituent fibres of the nonwoven fabric 60 are subjected to compression bonding or adhesion, and the thermally extensible fibres are in a non-extended state. The convex portion 62 is a part in which the thermally extensible fibres is thermally extended and rises on the side of the first surface 1A. Accordingly, the convex portion 62 is formed into a bulky part in which a fibre density is lower in comparison with the concave portion 62. Moreover, the linear concave portions 62 are arranged in a lattice form, and the convex portions 61 are arranged in each region partitioned by the lattice in a scattered manner. Thus, in the nonwoven fabric 60, a contact area with wearer's skin is suppressed, in which a stuffiness and a rash are effectively prevented. Moreover, the convex portion 61 in contact with the skin is bulky by thermal extension of the thermally extensible fibres into soft texture. Further, the nonwoven fabric 60 may have a monolayer structure, or a multi-layered structure of two or more layers. For example, when the nonwoven fabric 60 has a double-layered structure, a layer on the side of the second surface 1B preferably contains no thermally extensible fibres, or has a smaller content of the thermally extensible fibres than a layer on the side of the first surface 1A having the concavo-convex shape. Moreover, the both layers are preferably bonded in the compression adhesion portion of the concave portion 62.

Also in the nonwoven fabric 60, a permeation passage of the liquid is ensured at all times by the above-mentioned effect of the liquid film cleavage agent, or the liquid film cleavage agent and the phosphoric acid ester type anionic surfactant. Thus, a width of design with regard to the fibre diameter and the fibre density is extended.

Such a nonwoven fabric 60 can be produced by the following method. First, the linear concave portion 62 is formed by heat embossing to a fibre web. At this time, in the concave portion 62, the thermally extensible fibres are subjected to compression bonding or fusion without being thermally extended, and fixed. Next, the thermally extensible fibres existing in parts other than the concave portion 61 are extended by air-through processing, and the convex portion 61 is formed into the nonwoven fabric 60. Moreover, the constituent fibres of the nonwoven fabric 60 may be mixed fibres of the above-described thermally extensible fibres and thermally non-extensible and thermally fusible fibres. As these constituent fibres, for example, the fibres described in the paragraphs {0013}, and {0037} to {0040} of JP-A-2005-350836, the fibres described in the paragraphs {0012}, and {0024} to {0046} of JP-A-2011-1277258, and so forth can be used.

Next, as shown in FIG. 11, a nonwoven fabric 70 in the seventh aspect is a laminated nonwoven fabric formed of an upper layer 71 and a lower layer 72 each containing thermoplastic fibres. In the upper layer 71, convex-shaped

portions 73 and concave-shaped portions 74 are alternately arranged, and the concave-shaped portions 74 have openings. A fibre density in the concave-shaped portion 74 is low in comparison with a fibre density in the convex-shaped portion 73. A region in which the convex-shaped portions 73 and the concave-shaped portions 74 are alternately and repeatedly arranged may exist partially or wholly in the upper layer 71. When the region in which the convex-shaped portions 73 and the concave-shaped portions 74 are alternately and repeatedly arranged exists partially in the upper layer, the region preferably exists in a part serving as a liquid receiving region (region corresponding to an excretion portion) upon employing the nonwoven fabric 70 as a topsheet of an absorbent article. On the other hand, the lower layer 72 has a substantially uniform fibre density. The lower layer 72 is laminated at least in corresponding to the region in which the convex-shaped portions 73 and the concave-shaped portions 74 in the upper layer 71 are alternately and repeatedly arranged. Thus, the nonwoven fabric 70 has bulky cushioning properties owing to a high fibre density in the convex-shaped portion 73, and if the nonwoven fabric 70 is employed as the topsheet of the absorbent article, liquid backflow becomes hard to occur. Moreover, the nonwoven fabric 70 is low in the fibre density in the concave-shaped portion 74, and in an opened state, and therefore the nonwoven fabric 70 is excellent in liquid permeability, particularly, permeability to a liquid with high viscosity.

Also in the nonwoven fabric 70, a permeation passage of the liquid is ensured at all times by the above-mentioned effect of the liquid film cleavage agent, or the liquid film cleavage agent and the phosphoric acid ester type anionic surfactant. Thus, a width of design with regard to the fibre diameter and the fibre density is extended.

Such a nonwoven fabric 70 can be produced by the method described in, for example, the line 12 in the left lower column on page 6 to the line 19 in the right upper column on page 8 in JP-A-H4-24263.

The liquid film cleavage agent and the nonwoven fabric containing the liquid film cleavage agent according to the present invention can be applied in various fields by taking advantage of the soft texture and reduction of the liquid remains. For example, such a material is preferably used as a topsheet, a second sheet (a sheet arranged between the topsheet and an absorbent body), an absorbent body, a covering sheet for wrapping the absorbent body, or a leakage preventive sheet in an absorbent article used for absorption of a fluid excreted from a body, such as a sanitary towel, a panty liner, a disposable nappy, an incontinence pad; a wiping sheet for a person; a sheet for skin care or, further, a wiper for an object. When the nonwoven fabric according to the present invention is employed as the topsheet or the second sheet of the absorbent article, the side of the first layer of the nonwoven fabric is preferably used as a side of skin-facing surface. Further, the liquid film cleavage agent according to the present invention can be applied to various fibre materials such as a woven fabric without limiting to the nonwoven fabric, as long as the liquid film cleavage agent has effect of cleaving the liquid film.

With regard to the basis weight of the web used for production of the nonwoven fabric according to the present invention, a suitable range is selected in corresponding to a specific application of an objective nonwoven fabric. The basis weight of the nonwoven fabric to be finally obtained is preferably 10 $g/m^2$ or more and 100 $g/m^2$ or less, and particularly preferably 15 $g/m^2$ or more and 80 $g/m^2$ or less.

The absorbent article used for absorption of the fluid excreted from the body is typically provided with the topsheet, the backsheet, and a liquid-retainable absorbent body interposed between both sheets. As the absorbent body and the backsheet when the nonwoven fabric according to the present invention is used as the topsheet, a material to be ordinarily used in the technical field can be used without particular restriction. For example, as the absorbent body, such a material can be used as prepared by covering, with a covering sheet such as tissue paper and the nonwoven fabric, a fibre aggregate formed of a fibre material such as pulp fibres, or the fibre aggregate with a superabsorbent polymer therein. As the backsheet, a film of a thermoplastic resin, or a liquid-impermeable or water-repellent sheet such as a laminate between the film and the nonwoven fabric can be used. The backsheet may have water-vapour permeability. The absorbent article may be further provided with various members in corresponding to the specific application of the absorbent article. Such a member is known to those skilled in the art. For example, when the absorbent article is applied to the disposable nappy or the sanitary towel, one pair or two or more pairs three-dimensional guards can be arranged in both right-left side portions on the topsheet.

Exemplary embodiments

Embodiment 1 is a liquid film cleavage agent having a spreading coefficient of 15 mN/m or more to a liquid having surface tension of 50 mN/m, and a water solubility of 0 g or more and 0.025 g or less, comprising a compound having at least one kind structure selected from the group consisting of the following structures X, X-Y and Y-X-Y:

wherein the structure X designates a siloxane chain having a structure in which any of basic structures of >C(A)- (C designates a carbon atom, moreover, <, > and - each designates a bonding, hereinafter, the same applies.), $-C(A)_2-$, -C(A)(B)-, $>C(A)-C(R^1)<$, $>C(R^1)-$, $-C(R^1)(R^2)-$, $-C(R^1)_2-$, >C<, $-Si(R^1)_2O-$ and $-Si(R^1)(R^2)O$ is repeated,

or two or more kinds thereof are combined; or a mixed chain thereof; the structure X has, in an end of the structure X, a hydrogen atom or at least one kind of group selected from the group consisting of $-C(A)_3$, $-C(A)_2B$, $-C(A)(B)_2$, $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ and $-Si(R^1)_2(R^2)$;
wherein $R^1$ and $R^2$ each independently designate a hydrogen atom, an alkyl group, an alkoxy group, an aryl group, or a halogen atom; A and B each independently designate a substituent having an oxygen atom or a nitrogen atom; when a plurality of $R^1$, $R^2$, A and B exist for each in the structure X, these may be identical to or different from each other; and
wherein Y designates a hydrophilic group having hydrophilicity, the group containing an atom selected from a hydrogen atom, a carbon atom, an oxygen atom, a nitrogen atom, a phosphorus atom or a sulphur atom; and when a plurality of Y exists, these groups may be identical to or different from each other, which compound is a polyoxyalkylene modified-silicone, and wherein the addition number of moles of the polyoxyalkylene groups of the polyoxyalkylene modified-silicone is 1 or more and 10 or less.

Embodiment 2 is the liquid film cleavage agent of embodiment 1, wherein the spreading coefficient is more preferably 20 mN/m or more, further preferably 25 mN/m or more, and particularly preferably 30 mN/m or more.

Embodiment 3 is the liquid film cleavage agent of embodiment 1 or 2,
wherein an interfacial tension to the liquid having surface tension of 50 mN/m is preferably 20 mN/m or less, more preferably 17 mN/m or less, further preferably 13 mN/m or less, more further preferably 10 mN/m or less, particularly preferably 9 mN/m or less, and especially preferably 1 mN/m or less; and more than 0 mN/m.

Embodiment 4 is the liquid film cleavage agent of any one of embodiments 1 through 3, wherein A and B each independently designate a hydroxyl group, a carboxylic acid group, an amino group, an amido group, an imino group, or a phenol group. Embodiment 5 is the liquid film cleavage agent of any one of embodiments 1 through 4, comprising a compound composed of a siloxane chain in which structures represented by any one of the following formulas (1) to (11) are arbitrarily combined:

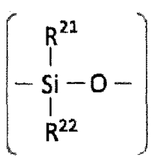 ••• (1)

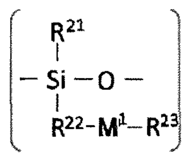 ••• (5)

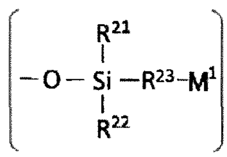 ••• (9)

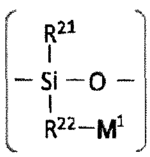 ••• (2)

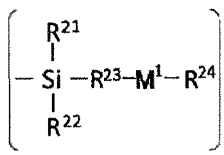 ••• (6)

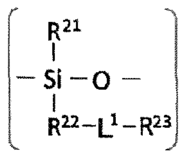 ••• (10)

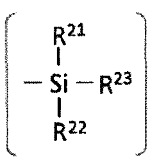 ••• (3)

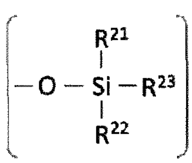 ••• (7)

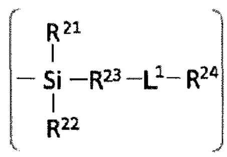 ••• (11)

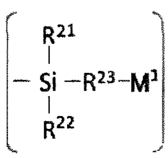 ••• (4)

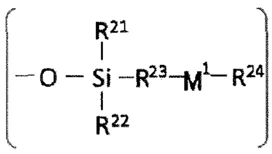 ••• (8)

wherein, in Formulas (1) to (11), $M^1$, $L^1$, $R^{21}$ and $R^{22}$ designate the following monovalent or polyvalent (divalent or more valent) group; $R^{23}$ and $R^{24}$ designate the following monovalent or polyvalent (divalent or more valent) group or a single bond;
$M^1$ designates a polyoxyethylene group, a polyoxypropylene group, a polyoxybutylene group, a group having

a polyoxyalkylene group in combination therewith, an erythritol group, a xylitol group, a sorbitol group, a glycerol group or an ethylene glycol group, a hydroxyl group, a carboxylic acid group, a mercapto group, an alkoxy group, an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith; when $M^1$ is a polyvalent group, $M^1$ designates a group formed by further removing one or more hydrogen atoms from each of the groups or the functional group;

$L^1$ designates a linking group of an ether group, an amino group (the amino group adoptable as $L^1$ is represented by $>NR^C$ ($R^C$ is a hydrogen atom or a monovalent group).), an amide group, an ester group, a carbonyl group or a carbonate group; and

$R^{21}$, $R^{22}$, $R^{23}$ and $R^{24}$ each independently designate an alkyl group, an alkoxy group, an aryl group, a fluoroalkyl group, an aralkyl group, a hydrocarbon group in combination therewith, or a halogen atom.

Embodiment 6 is the liquid film cleavage agent of any one of embodiments 1 through 5, wherein the polyoxyalkylene modified-silicone is represented by any one of Formulas [I] to [IV]:

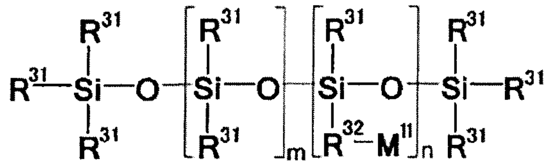

[I]

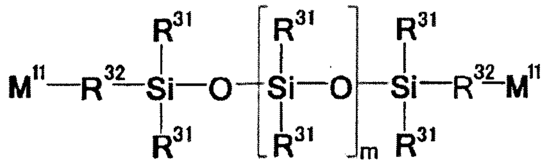

[II]

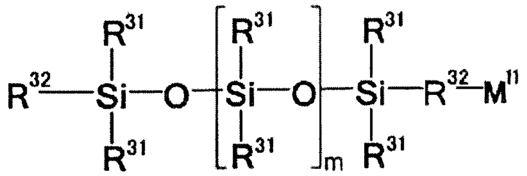

[III]

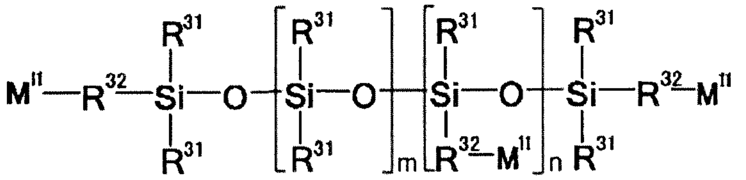

[IV]

wherein $R^{31}$ designates an alkyl group; $R^{32}$ designates a single bond or an alkylene group; a plurality of $R^{31}$ and a plurality of $R^{32}$ may be each identical to or different from each other; $M^{11}$ designates a group having a polyoxyalkylene group; as the polyoxyalkylene group, a polyoxyethylene group, a polyoxypropylene group, a polyoxybutylene group and a material in which constituent monomers thereof are copolymerised or the like are taken; and m and n each are independently an integer of 1 or more. Embodiment 7 is a liquid film cleavage agent having a structure represented by any one of Formulas [I] to [IV], and a water solubility of 0 g or more and 0.025 g or less:

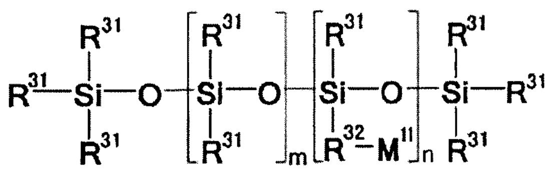

[I]

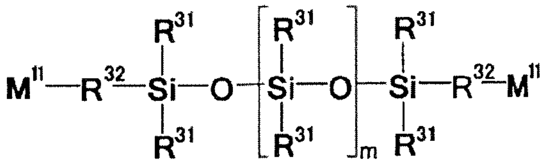

[II]

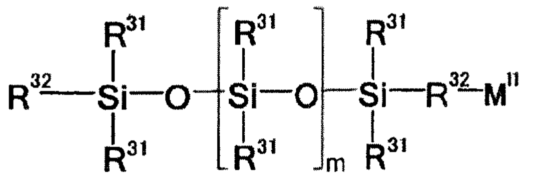

[III]

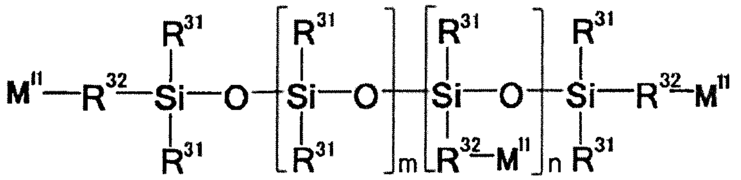

[IV]

wherein $R^{31}$ designates an alkyl group; $R^{32}$ designates a single bond or an alkylene group; a plurality of $R^{31}$ and a plurality of $R^{32}$ may be each identical to or different from each other; $M^{11}$ designates a group having a polyoxyalkylene group; as the polyoxyalkylene group, a polyoxyethylene group, a polyoxypropylene group, a polyoxybutylene group and a material in which constituent monomers thereof are copolymerised or the like are taken; and m and n each are independently an integer of 1 or more.

Embodiment 8 is the liquid film cleavage agent of any one of embodiments 1 through 7, wherein the polyoxyalkylene-modified silicone is any of polyoxyethylene (POE) polyoxypropylene (POP)-modified silicone, polyoxyethylene (POE)-modified silicone and polyoxypropylene (POP)-modified silicone. Embodiment 9 is the liquid film cleavage agent of any one of embodiments 1 through 8, wherein the addition number of moles of the polyoxyalkylene groups of the polyoxyalkylene-modified silicone is preferably 3 or more, and further preferably 5 or more; and 10 or less.

Embodiment 10 is a liquid film cleavage agent having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m, and comprising a compound having at least one kind structure selected from the group consisting of the following structures Z, Z-Y and Y-Z-Y:

wherein the structure Z designates a hydrocarbon chain having a structure in which any of basic structures of >C(A)- (C: carbon atom), $-C(A)_2-$, - C(A)(B)-, >C(A)-C($R^3$)<, >C($R^3$)-, -C($R^3$)($R^4$)-, $-C(R^3)_2-$and >C< is repeated, or two or more kinds thereof are combined; the structure Z has, at an end thereof, a hydrogen atom or at least one kind of group selected from the group consisting of $-C(A)_3$, $-C(A)_2B$, $-C(A)(B)_2$, $-C(A)_2-C(R^3)_3$, $-C(R^3)_2A$ and $-C(R^3)_3$;

the $R^3$ and $R^4$ each independently designate a hydrogen atom, an alkyl group, an alkoxy group, an aryl group, a fluoroalkyl group, or an aralkyl group, or a hydrocarbon group in combination therewith, or a fluorine atom; A and B each independently designates a substituent containing an oxygen atom or a nitrogen atom;

Y designates a hydrophilic group having hydrophilicity, the hydrophilic group containing an atom selected from a hydrogen atom, a carbon atom, an oxygen atom, a nitrogen atom, a phosphorus atom and a sulphur atom; and when Y is plural, the plurality may be identical to or different from each other.

Embodiment 11 is the liquid film cleavage agent of embodiment 10, wherein the interfacial tension to the liquid having surface tension of 50 mN/m is preferably 17 mN/m or less, more preferably 13 mN/m or less, further preferably 10 mN/m or less, particulary preferably 9 mN/m or less, and especially preferably 1 mN/m or less; and more than 0 mN/m. Embodiment 12 is the liquid film cleavage agent of embodiment 10 or 11, wherein the spreading coefficient to the liquid having surface tension of 50 mN/m is preferably 9 mN/m or more, more preferably 10 mN/m or more, and further preferably 15 mN/m or more; and 50 mN/m or less.

Embodiment 13 is the liquid film cleavage agent of any one of embodiments 10 through 12, wherein Y is any of a

hydroxyl group, a carboxylic acid group, an amino group, an amide group, an imino group and a phenol group; or a polyoxyalkylene group; or any of an erythritol group, a xylitol group, a sorbitol group, a glycerol group and an ethylene glycol group; or any of a sulphonic acid group, a sulphate group, a phosphoric acid group, a sulphobetaine group, a carbobetaine group, a phosphobetaine group, a quaternary ammonium group, an imidazolium betaine group, an epoxy group, a carbinol group and a methacrylic group; or a hydrophilic group formed of a combination thereof. Embodiment 14 is the liquid film cleavage agent of any one of embodiments 10 through 13, comprising polyoxyalkylene alkyl ether, or a hydrocarbon compound having 5 or more carbon atoms. Embodiment 15 is the liquid film cleavage agent of any one of embodiments 10 through 14, comprising polyoxyalkylene alkyl (POA) ether represented by any of formulas in Formula [V]; or any of polyoxyalkylene glycol, Steareth, Beheneth, PPG myristyl ether, PPG stearyl ether and PPG behenyl ether, which are represented by any of formulas in Formula [VI] and have a mass average molecular weight of 1000 or more:

$$C_mH_n-L^{21}\left(C_aH_bO\right)_m L^{21}-R^{51}$$

or

$$C_mH_n-L^{21}\left(C_aH_bO\right)_m R^{51} \qquad [V]$$

or

$$C_mH_n-L^{21}\left(C_aH_bO\right)_m H$$

$$R^{51}-\left(C_aH_bO\right)_m R^{51} \qquad [VI]$$

wherein $L^{21}$ designates an ether group, an amino group, an amide group, an ester group, a carbonyl group, a carbonate group, a polyoxyethylene group, a polyoxypropylene group, a polyoxybutylene group, or a polyoxyalkylene group in combination therewith; $R^{51}$ designate a substitute of a hydrogen atom, a methyl group, an ethyl group, a propyl group, an isopropyl group, a butyl group, a pentyl group, a hexyl group, a heptyl group, 2-ethylhexyl group, a nonyl group, a decyl group, a methoxy group, an ethoxy group, a phenyl group, a fluoroalkyl group, an aralkyl group, a hydrocarbon group in combination therewith, or a fluorine atom; a, b, m and n each independently are an integer of 1 or more; $C_mH_n$ herein designates an alkyl group (n = 2m + 1), and $C_aH_b$ designates an alkylene group (a = 2b); the number of carbon atoms and the number of hydrogen atoms are each independently determined in each of Formulas (V) and (VI), and do not always represent an identical integer, and may be different from each other; "m" in $-(C_aH_bO)_m-$ is an integer of 1 or more; and values of the repeating units are each independently determined in each of Formulas (V) and (VI), and do not always represent an identical integer, and may be different from each other.

Embodiment 16 is the liquid film cleavage agent of any one of embodiments 10 through 15, comprising a compound having a polyoxyalkylene group, wherein the number of mole of the polyoxyalkylene group is 1 or more and 70 or less, more preferably 5 or more, further preferably 7 or more, and preferably 70 or less, more preferably 60 or less, and further preferably 50 or less.

Embodiment 17 is the liquid film cleavage agent of any one of embodiments 10 through 13, comprising a hydrocarbon compound having 5 or more and preferably 100 or less, more preferably 50 or less carbon atoms. Embodiment 18 is the liquid film cleavage agent of embodiment 17, wherein the hydrocarbon compound excludes polyorganosiloxane. Embodiment 19 is the liquid film cleavage agent of embodiment 17 or 18, wherein the hydrocarbon compound is represented by any one of Formulas [VII] to [XV]:

$C_mH_n$ - COOH [VII]

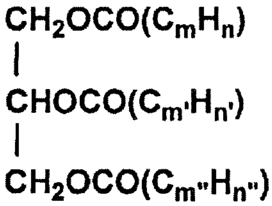

[VIII-I]

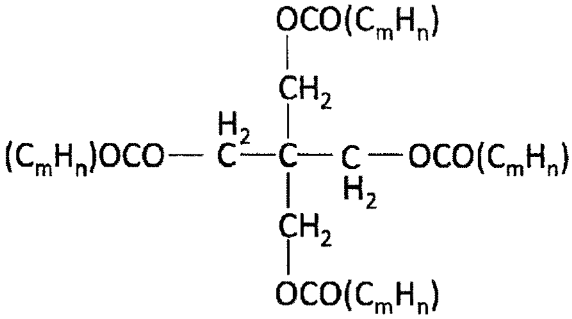

[VIII-II]

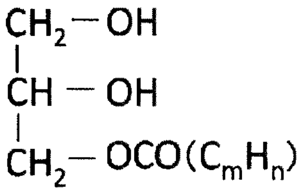

or

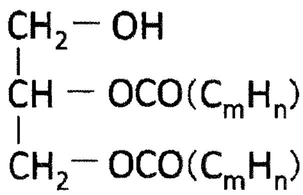

[IX]

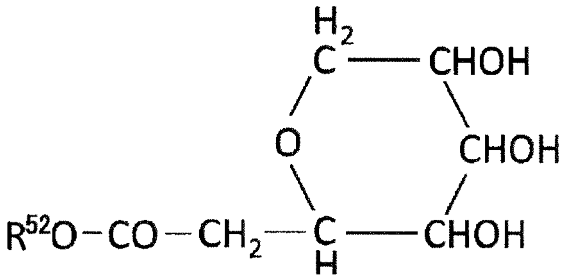

or

[X]

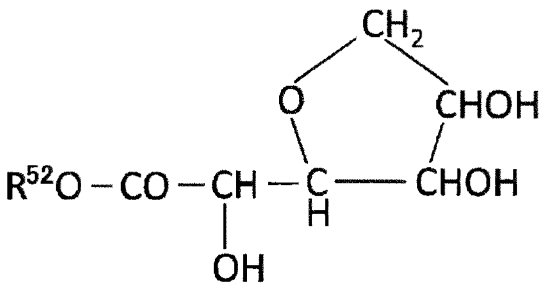

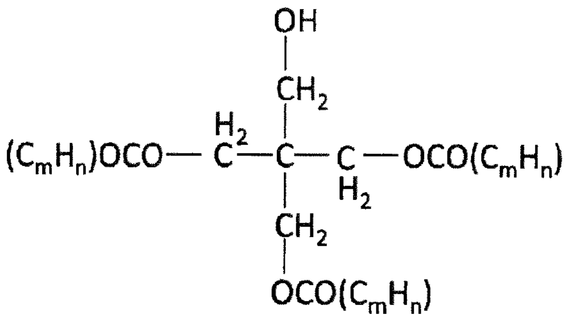

[XI]

or

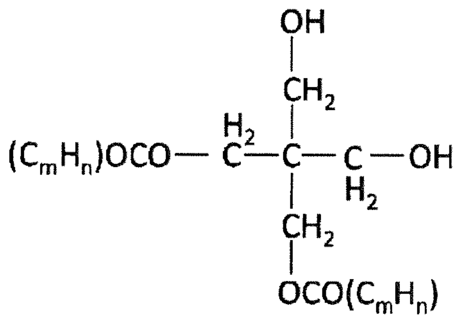

or

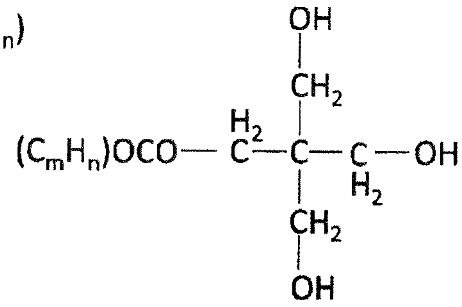

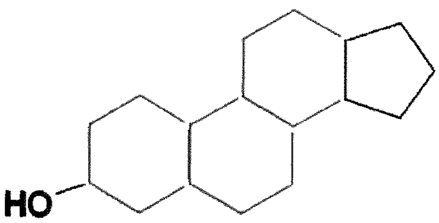

[XII]

$C_mH_n$ - OH [XIII]

$C_mH_n$ - COO - $C_mH_n$ [XIV]

$C_mH_n$ [XV]

wherein, in Formulas [VII] to [XV], m, m', m", n, n' and n" each independently are an integer of 1 or more; a plurality of m or a plurality of n each may be identical to or different from each other; and in Formula [X], $R^{52}$ designates a straight-chain or branched-chain, or saturated or unsaturated hydrocarbon group having 2 or more and 22 or less carbon atoms.

Embodiment 20 is the liquid film cleavage agent of any one of embodiments 10 through 19,
wherein the spreading coefficient to the liquid having surface tension of 50 mN/m is 9 mN/m or more, the water solubility is 0 g or more and 0.025 g or less, the interfacial tension to the liquid having surface tension of 50 mN/m is 9 mN/m or less, and the surface tension is 32 mN/m or less.

Embodiment 21 is the liquid film cleavage agent of any one of embodiments 1 through 20,

wherein the water solubility is preferably 0.0025 g or less, more preferably 0.0017 g or less, further preferably less than 0.0001 g, and $1.0 \times 10^{-9}$ g or more. Embodiment 22 is the liquid film cleavage agent of any one of embodiments 1 through 21, wherein a surface tension of the liquid film cleavage agent is preferably 32 mN/m or less, more preferably 30 mN/m or less, further preferably 25 mN/m or less, and particularly preferably 22 mN/m or less; and preferably 1mN/m or more.

Embodiment 23 is the liquid film cleavage agent of any one of embodiments 1 through 22 which has a mass average molecular of 500 or more, more preferably 1,000 or more, further preferably 1,500 or more, and particularly preferably 2,000 or more; and preferably 50,000 or less, more preferably 20,000 or less, and further preferably 10,000 or less. Embodiment 24 is the liquid film cleavage agent of any one of embodiments 1 through 23 which has a melting point of preferably 40°C or less, and more preferably 35°C or less; and -220°C or more, and more preferably -180°C or more. Embodiment 25 is a fibre treating agent containing the liquid film cleavage agent of any one of embodiments 1 through 24 in an amount of 5 mass% or more and 50 mass% or less. Embodiment 26 is the fibre treating agent of embodiment 25 further containing a phosphoric acid ester type anionic surfactant. Embodiment 27 is the fibre treating agent of embodiment 26, wherein a content ratio of the liquid film cleavage agent to the phosphoric acid ester type anionic surfactant is preferably (1:1) to (19:1), more preferably (2:1) to (15:1), and further preferably (3:1) to (10:1) in terms of a mass ratio.

Embodiment 28 is the fibre treating agent of embodiments 26 or 27, wherein the phosphoric acid ester type anionic surfactant is any one of alkyl ether phosphoric acid ester, dialkyl phosphoric acid ester and alkyl phoshoric acid ester.

Embodiment 29 is the fibre treating agent of embodiment 28, wherein the alkyl phosphoric acid ester is any one of: alkyl phosphoric acid ester having a saturated carbon chain, such as stearyl phosphoric acid ester, myristyl phosphoric acid ester, lauryl phosphoric acid ester and palmityl phosphoric acid ester; alkyl phosphoric acid ester having an unsaturated carbon chain such as oleyl phosphoric acid ester and palmitoleyl phosphoric acid ester; and alkyl phosphoric acid ester having a side chain in each carbon chain thereof.

Embodiment 30 is a nonwoven fabric containing the liquid film cleavage agent of any one of embodiments 1 through 24. Embodiment 31 is the nonwoven fabric of embodiment 30, wherein the liquid film cleavage agent is localised at least around part of the points that fibres are entangled to each other or the points that fibres are fusion bonded to each other. Embodiment 32 is the nonwoven fabric of embodiment 30 or 31 further containing a phosphoric acid ester type anionic surfactant. Embodiment 33 is the nonwoven fabric of embodiment 32, wherein a content ratio of the liquid film cleavage agent to the phosphoric acid ester type anionic surfactant is preferably (1:1) to (19:1), more preferably (2:1) to (15:1), and further preferably (3:1) to (10:1) in terms of a mass ratio.

Embodiment 34 is the nonwoven fabric of embodiment 32 or 33, wherein the phosphoric acid ester type anionic surfactant is any one of alkyl ether phosphoric acid ester, dialkyl phosphoric acid ester and alkyl phosphoric acid ester.

Embodiment 35 is the nonwoven fabric of embodiment 34 wherein the alkyl phosphoric acid ester is any one of: alkyl phosphoric acid ester having a saturated carbon chain, such as stearyl phosphoric acid ester, myristyl phosphoric acid ester, lauryl phosphoric acid ester and palmityl phosphoric acid ester; alkyl phosphoric acid ester having an unsaturated carbon chain such as oleyl phosphoric acid ester and palmitoleyl phosphoric acid ester; and alkyl phosphoric acid ester having a side chain in each carbon chain thereof.

Embodiment 36 is the nonwoven fabric of any one of embodiments 30 through 35
wherein the contact angle of constituent fibres of the nonwoven fabric is preferably 90 degrees or less, more preferably 80 degrees or less, and further preferably 70 degrees or less.

Embodiment 37 is the nonwoven fabric of any one of embodiments 30 through 36
wherein the interfibre distance in the nonwoven fabric is preferably 150 μm or less, and more preferably 90 μm or less; and preferably 50 μm or more, and more preferably 70 μm or more. Embodiment 38 is the nonwoven fabric of any one of embodiments 30 through 37, wherein the fineness of the fibres in the nonwoven fabric is preferably 3.3 dtex or less, and more preferably 2.4 dtex or less; and preferably 0.5 dtex or more, and more preferably 1.0 dtex or more.

Embodiment 39 is the nonwoven fabric of any one of embodiments 30 through 38,
comprising thermoplastic fibres, wherein the nonwoven fabric has a first surface and a second surface positioned on the side opposite thereto, and at least in the first surface, has a concavo-convex shape having a plurality of

convex portions projecting on a side of the first surface and concave portions positioned among the convex portions to be a concavo-convex nonwoven fabric.

Embodiment 40 is a topsheet for an absorbent article in which the nonwoven fabric of any one of embodiments 30 through 38 is used,

wherein the topsheet has at least two layers;
the topsheet has a plurality of concave bonding portions in which the layers are bonded to each other with the compression from a skin-contact surface side in a thickness direction;
a layer on a skin non-contact surface side of the topsheet is a layer formed in which thermally shrinkable fibres are being thermally shrunk; and
a layer on the skin-contact surface side of the topsheet has thermally non-shrinkable fibres partially bonded at the bonding portion, and has convex portions projecting on the skin-contact surface side in a region among the concave bonding portions to form a concavo-convex surface of the nonwoven fabric. Embodiment 41 is a topsheet for an absorbent article in which the nonwoven fabric of any one of embodiments 30 through 38 is used,
wherein the topsheet has a double-layered structure having a hollow portion and formed of a first nonwoven fabric on a skin-contact surface side and a second nonwoven fabric on a skin non-contact surface side, and both layers contain thermoplastic fibres; and
the topsheet has a bonding portion in which the first nonwoven fabric and the second nonwoven fabric are partially thermally fusion bonded, and in a non-bonding portion surrounded by the bonding portions, the first nonwoven fabric has a large number of convex portions projecting in a direction away from the second nonwoven fabric with the hollow portions inside thereof, and the bonding portion is a concave portion positioned between the adjacent convex portions and constitutes a concavo-convex shape on the skin-contact surface side in cooperation with the convex portions. Embodiment 42 is a topsheet for an absorbent article in which the nonwoven fabric of any one of embodiments 30 through 38 is used,
wherein the topsheet comprises one layer containing thermoplastic fibres and has a concavo-convex shape for both sides;
a first projecting portion projecting on a side of a first surface and a second projecting portion projecting on a side of a second surface are alternately continuously arranged in different intersecting directions in a planar view of the nonwoven fabric; and
the first projecting portion and the second projecting portion each have an internal space opened on a side of each opposite surface, which forms concave portions on the each surface. Embodiment 43 is a topsheet for an absorbent article in which the nonwoven fabric of any one of embodiments 30 through 38 is used,
wherein the topsheet comprises two layers containing thermoplastic fibres;
a first projecting portion projecting on a side of a first surface and a second projecting portion projecting on side of a second surface are alternately continuously arranged in different intersecting directions in a planar view of the nonwoven fabric, and the first projecting portion and the second projecting portion each have an internal space opened on a side of each opposite surface; and
a first fibre layer has a concave portion formed in the internal space on the each surface, and a second fibre layer is arranged along a concavo-convex shape on the side of the second surface of the first fibre layer and bonded with the first fibre layer, and a side of the first fibre layer is applied as a skin-contact surface side. Embodiment 44 is a topsheet for an absorbent article in which the nonwoven fabric of any one of embodiments 30 through 38 is used,
wherein the topsheet comprises one layer containing thermoplastic fibres, and has a shape in which a plurality of hemicylindrical convex portions and a plurality of concave portions arranged along side edges of the convex portions are alternately arranged on a skin-contact surface side; and
concave bottom portions comprising fibres of the nonwoven fabric are arranged on a lower side of the concave portions, and a fibre density of the concave bottom portions are smaller than a fibre density of the convex portions. Embodiment 45 is an absorbent article, empoying the nonwoven fabric of any one of embodiments 30 through 39. Embodiment 46 is an absorbent article, employing the nonwoven fabric of any one of embodiments 30 through 39 as a topsheet, or containing the topsheet of any one of embodiments 40 through 44. Embodiment 47 is the absorbent article of embodiments 45 or 46, wherein the absorbent article is a sanitary towel.

Embodiment 48 is a method for producing the nonwoven fabric of any one of embodiments 30 through 39 comprising a step of immersing, into a solution containing a liquid film cleavage agent, a raw material nonwoven fabric prepared by thermally fusing fibres to each other by air-through processing or heat embossing. Embodiment 49 is a method for producing the nonwoven fabric of any one of embodiments 30 through 39 comprising a step of coating a liquid film cleavage agent alone or a solution containing the liquid film cleavage agent to a raw material nonwoven fabric

prepared by thermally fusing fibres to each other by air-through processing or heat embossing.

Embodiment 50 is the method for producing the nonwoven fabric of embodiment 49 wherein the coating method employs a flexographic system. Embodiment 51 is a use of a compound having a spreading coefficient of 15 mN/m or more to a liquid having surface tension of 50 mN/m, and a water solubility of 0 g or more and 0.025 g or less, as a liquid film cleavage agent. Embodiment 52 is a use of a compound having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m, as a liquid film cleavage agent.

EXAMPLES

Hereinafter, the present invention will be described more in detail with reference to Examples, but the present invention is not limited thereto. Further, both terms "part" and "%" in the Example are based on mass unless otherwise noted. Moreover, as mentioned above, a spreading coefficient, interfacial tension, a surface tension and a water solubility were measured in an environmental range of a temperature of 25°C and a relative humidity (RH) of 65%. A surface tension, a water solubility and an interfacial tension of a liquid film cleavage agent in the Examples described below were measured by the above-mentioned measuring method.

(Example 1)

A concavo-convex shaped raw material nonwoven fabric shown in FIG. 3 was prepared by the above-mentioned method. Thermally non-shrinkable and thermally fusible fibres having fineness of 1.2 dtex were used in an upper layer (layer on a side of a first surface 1A), and thermally shrinkable fibres having fineness of 2.3 dtex were used in a lower layer (layer on a side of a second surface 1B). An interfibre distance in the upper layer at this time was 80 μm, and an interfibre distance in the lower layer was 60 μm. Moreover, a basis weight of the nonwoven fabric was 74 g/m$^2$.

Before the above-described preparation, a diluted solution having 0.06 mass% of effective component of a liquid film cleavage agent was prepared by dissolving, into ethanol, the liquid film cleavage agent being polyoxyethylene (POE)-modified dimethyl silicone (KF-6015, manufactured by Shin-Etsu Chemical Co., Ltd.), in which X in a structure X-Y was formed of a dimethyl silicone chain composed of $-Si(CH_3)_2O-$, Y was formed of a POE chain composed of $-(C_2H_4O)-$, an end group of the POE chain was a methyl group ($CH_3$), a modification ratio was 20%, the addition number of moles of polyoxyethylene was 3, and a mass average molecular weight was 4000. A nonwoven fabric sample in Example 1 was prepared by immersing the above-described raw material nonwoven fabric into the diluted solution and drying the resultant material. A content proportion (OPU) of the polyoxyethylene (POE)-modified dimethyl silicone as the liquid film cleavage agent to fibre mass was 0.1 mass%.

With regard to the polyoxyethylene (POE) modified dimethyl silicone, a surface tension was 21.0 mN/m, and a water solubility was less than 0.0001 g. Moreover, a spreading coefficient of the polyoxyethylene (POE)-modified dimethyl silicone to the liquid having surface tension of 50 mN/m was 28.8 mN/m, and an interfacial tension thereof to the liquid having surface tension of 50 mN/m was 0.2 mN/m. These numerical values were measured by the above-mentioned measuring method. On the occasion, as a "liquid having surface tension of 50 mN/m," a solution was used, in which the solution was prepared by adding, to 100 g of deionised water, 3.75 μL of polyoxyethylene sorbitan monolaurate (trade name "Leodol Super TW-L120," manufactured by Kao Corporation) being a nonionic surface active substance by using a micropipette (ACURA 825, manufactured by Socorex Isba SA), and adjusting surface tension to 50±1 mN/m. Moreover, the water solubility was measured by adding the agent for every 0.0001 g. As a result, a sample which was observed to be not dissolved even in 0.0001 g was taken as "less than 0.0001 g," and a sample which was observed to be dissolved in 0.0001 g but not dissolved in 0.0002 g was taken as "0.0001g." The numerical values other than the above were measured by the same methods.

(Example 2)

A nonwoven fabric sample in Example 2 was prepared in the same manner with Example 1 except that the material described below is used as the liquid film cleavage agent.

<Liquid film cleavage agent>

Polyoxypropylene (POP)-modified dimethyl silicone (obtained by performing a hydrosilylation reaction between silicone oil and a hydrocarbon compound), in which X in a structure X-Y is formed of a dimethyl silicone chain composed of $- Si(CH_3)_2O-$, Y is formed of a POP chain composed of $-(C_3H_6O)-$, an end group of the POP chain is a methyl group ($CH_3$), a modification ratio is 20%, the addition number of moles of polyoxypropylene is 3, and a mass average molecular

weight is 4150.
A surface tension: 21.0 mN/m
A water solubility: less than 0.0001 g (< 0.0001 g)
A spreading coefficient to the liquid having surface tension of 50 mN/m: 25.4 mN/m
An interfacial tension to the liquid having surface tension of 50 mN/m: 3.6 mN/m (The four numerical values were measured by the method same as in Example 1.)

(Example 3)

A nonwoven fabric was prepared in the same manner with Example 1 except for: using, as a liquid film cleavage agent, polyoxypropylene (POP)-modified dimethyl silicone (obtained by performing a hydrosilylation reaction between silicone oil and a hydrocarbon compound), in which X in a structure X-Y was formed of a dimethyl silicone chain composed of $-Si(CH_3)_2O-$, Y was formed of a POP chain composed of $-(C_3H_6O)-$, an end group of the POP chain was a methyl group ($CH_3$), a modification ratio was 20%, the addition number of moles of polyoxypropylene was 10, and a mass average molecular weight was 7000; and adjusting a content proportion (OPU) of the liquid film cleavage agent to fibre mass to 0.1 mass%. The obtained nonwoven fabric was taken as a sample of Example 3.

With regard to the polyoxypropylene (POP)-modified dimethyl silicone, a surface tension was 21.5 mN/m, and a water solubility was less than 0.0001 g. Moreover, a spreading coefficient of the polyoxypropylene (POP)-modified dimethyl silicone to the liquid having surface tension of 50 mN/m was 28.0 mN/m, and an interfacial tension thereof to the liquid having surface tension of 50 mN/m was 0.5 mN/m. The numerical values were measured by the method same as in Example 1.

(Example 4)

A nonwoven fabric sample in Example 4 was prepared in the same manner with Example 1 except that the material described below is used as the liquid film cleavage agent.

<Liquid film cleavage agent>

Polyoxypropylene (POP)-modified dimethyl silicone (obtained by performing a hydrosilylation reaction between silicone oil and a hydrocarbon compound), in which X in a structure X-Y is formed of a dimethyl silicone chain composed of $- Si(CH_3)_2O-$, Y is formed of a POP chain composed of $-(C_3H_6O)-$, an end group of the POP chain is a methyl group ($CH_3$), a modification ratio is 15%, the addition number of moles of polyoxypropylene is 10, and a mass average molecular weight is 5160.
A surface tension: 21.5 mN/m
A water solubility: 0.0001 g
A spreading coefficient to the liquid having surface tension of 50 mN/m: 27.5 mN/m
An interfacial tension to the liquid having surface tension of 50 mN/m: 1.0 mN/m (The four numerical values were measured by the method same as in Example 1.)

(Example 5)

A nonwoven fabric sample in Example 5 was prepared in the same manner with Example 1 except that the material described below is used as the liquid film cleavage agent.

<Liquid film cleavage agent>

Polyoxypropylene (POP)-modified dimethyl silicone (obtained by performing a hydrosilylation reaction between silicone oil and a hydrocarbon compound), in which X in a structure X-Y is formed of a dimethyl silicone chain composed of $- Si(CH_3)_2O-$, Y is formed of a POP chain composed of $-(C_3H_6O)-$, an end group of the POP chain is a methyl group ($CH_3$), a modification ratio is 10%, the addition number of moles of polyoxypropylene is 10, and a mass average molecular weight is 4340.
A surface tension: 21.5 mN/m
A water solubility: 0.0002 g
A spreading coefficient to the liquid having surface tension of 50 mN/m: 26.9 mN/m
An interfacial tension to the liquid having surface tension of 50 mN/m: 1.6 mN/m (The four numerical values were measured by the method same as in Example 1.)

(Example 6)

A nonwoven fabric was prepared in the same manner with Example 1 except for: using, as a liquid film cleavage agent, polyoxypropylene (POP)-modified dimethyl silicone (obtained by performing a hydrosilylation reaction between silicone oil and a hydrocarbon compound), in which X in a structure X-Y was formed of a dimethyl silicone chain composed of $-Si(CH_3)_2O-$, Y was formed of a POP chain composed of $-(C_3H_6O)-$, an end group of the POP chain was a methyl group ($CH_3$), a modification ratio was 20%, the addition number of moles of polyoxypropylene was 24, and a mass average molecular weight was 12500; and adjusting a content proportion (OPU) of the liquid film cleavage agent to fibre mass to 0.1 mass%. The obtained nonwoven fabric was taken as a sample of Example 6.

With regard to the polyoxypropylene (POP)-modified dimethyl silicone, a surface tension was 20.8 mN/m, and a water solubility was 0.0001 g. Moreover, a spreading coefficient of the polyoxypropylene (POP)-modified dimethyl silicone to the liquid having surface tension of 50 mN/m was 28.9 mN/m, and interfacial tension thereof to the liquid having surface tension of 50 mN/m was 0.3 mN/m. The numerical values were measured by the method same as in Example 1.

(Example 7)

A nonwoven fabric was prepared in the same manner with Example 1 except for: adjusting an effective component of a liquid film cleavage agent to 3.0 mass% by dissolving, into ethanol, polypropylene glycol (DEFOAMER No. 1, manufactured by Kao Corporation) as the film cleavage agent, in which X in a structure X was formed of a POP chain, the mole number of a polyoxypropylene group was 52, and a mass average molecular weight was 3000; and adjusting a content proportion (OPU) of the liquid film cleavage agent to fibre mass to 5.0 mass%. The obtained nonwoven fabric was taken as a sample in Example 7.

With regard to the polypropylene glycol, a surface tension was 32.7 mN/m, and a water solubility was less than 0.0001 g. Moreover, a spreading coefficient of the polypropylene glycol to the liquid having surface tension of 50 mN/m was 16.3 mN/m, and an interfacial tension thereof to the liquid having surface tension of 50 mN/m was 1.0 mN/m. The numerical values were measured by the method same as in Example 1.

(Example 8)

A nonwoven fabric was prepared in the same manner with Example 1 except for: using, as a liquid film cleavage agent, epoxy-modified dimethyl silicone (manufactured by Shin-Etsu Chemical Co., Ltd., KF-101), in which X in a structure X-Y was formed of a dimethyl silicone chain composed of $-Si(CH_3)_2O-$, Y was formed of an epoxy group composed of $-(RC_2H_3O)-$, a modification ratio was 32%, and a mass average molecular weight was 35800; and adjusting a content proportion (OPU) of the liquid film cleavage agent to fibre mass to 0.1 mass%. The obtained nonwoven fabric was taken as a sample of Example 8.

With regard to the epoxy-modified dimethyl silicone, a surface tension was 21.0 mN/m, and a water solubility was less than 0.0001 g. Moreover, a spreading coefficient of the epoxy-modified dimethyl silicone to the liquid having surface tension of 50 mN/m was 26.0 mN/m, and an interfacial tension thereof to the liquid having surface tension of 50 mN/m was 3.0 mN/m. The numerical values were measured by the method same as in Example 1.

(Example 9)

A nonwoven fabric was prepared in the same manner with Example 1 except for: using, as a liquid film cleavage agent, carbinol-modified dimethyl silicone (manufactured by Shin-Etsu Chemical Co., Ltd., X-22-4015), in which X in a structure X-Y was formed of a dimethyl silicone chain composed of $- Si(CH_3)_2O-$, Y was formed of a carbinol group composed of -(ROH)-, a modification ratio was 4%, and a mass average molecular weight was 9630; and adjusting a content proportion (OPU) of the liquid film cleavage agent to fibre mass to 0.1 mass%. The obtained nonwoven fabric was taken as a sample of Example 9.

With regard to the carbinol-modified dimethyl silicone, a surface tension was 21.0 mN/m, and a water solubility was less than 0.0001 g. Moreover, a spreading coefficient of the carbinol-modified dimethyl silicone to the liquid having surface tension of 50 mN/m was 27.3 mN/m, and an interfacial tension thereof to the liquid having surface tension of 50 mN/m was 1.7 mN/m. The numerical values were measured by the method same as in Example 1.

(Example 10)

A nonwoven fabric was prepared in the same manner with Example 1 except for: using, as the liquid film cleavage agent, diol-modified dimethyl silicone at one terminal (X-22-176DX, manufactured by Shin-Etsu Chemical Co.,

Ltd.), in which X in a structure X-Y was a dimethyl silicone chain composed of - $Si(CH_3)_2O$-, and Y was diol having a plurality of hydroxyl groups composed of - (OH)-, a modification ratio was 1.2%, and a mass average molecular weight was 6190; and adjusting a content proportion (OPU) of the liquid film cleavage agent to fibre mass to 0.1 mass%. The obtained nonwoven fabric was taken as a sample of Example 10.

With regard to the diol-modified dimethyl silicone, a surface tension was 21.0 mN/m, and a water solubility was less than 0.0001 g. Moreover, a spreading coefficient of the diol-modified dimethyl silicone to the liquid having surface tension of 50 mN/m was 27.1 mN/m, and an interfacial tension thereof to the liquid having surface tension of 50 mN/m was 1.9 mN/m. The numerical values were measured by the method same as in Example 1.

(Example 11)

A diluted solution to be applied to a nonwoven fabric was prepared in a manner similar to Example 1 with mixing polyoxyethylene (POE)-modified silicone used in Example 1 and stearyl (C18) phosphoric acid ester potassium salt (neutralised product with potassium hydroxide, GRIPPER 4131, manufactured by Kao Corporation). A content proportion of polyoxyethylene (POE)-modified silicone to stearyl (C18) phosphoric acid ester potassium salt in the diluted solution was adjusted to 1.67:1.

A nonwoven fabric sample in Example 11 was prepared in the same manner with Example 1 except for: using a raw material nonwoven fabric formed of an upper layer in which an interfibre distance was adjusted to 85 μm by using thermally non-shrinkable and thermally fusible fibres having fineness of 1.2 dtex, and a lower layer in which an interfibre distance was adjusted to 60 μm by using thermally shrinkable fibres having fineness of 2.3 dtex; and using the above-described diluted solution. A basis weight of the nonwoven fabric was adjusted to 74 $g/m^2$.

In the nonwoven fabric sample, a content proportion (OPU) of polyoxyethylene (POE)-modified silicone as the liquid film cleavage agent to fibre mass was adjusted to 0.1 mass%, and a content proportion (OPU) of stearyl phosphoric acid ester potassium salt to fibre mass was adjusted to 0.06 mass%.

(Example 12)

A diluted solution to be applied to a nonwoven fabric was prepared in a manner similar to Example 1 with mixing polyoxypropylene (POP)-modified silicone used in Example 3 and stearyl (C18) phosphoric acid ester potassium salt (neutralised product with potassium hydroxide, GRIPPER4131, manufactured by Kao Corporation). A content proportion of polyoxypropylene (POP)-modified silicone to stearyl (C18) phosphoric acid ester potassium salt in the diluted solution was adjusted to 1.67:1. A spreading coefficient, a water solubility and an interfacial tension of the polyoxypropylene (POP)-modified silicone as the liquid film cleavage agent were measured in the same manner with Example 3.

A nonwoven fabric sample in Example 12 was prepared in the same manner with Example 1 except for: using a raw material nonwoven fabric formed of an upper layer in which an interfibre distance was adjusted to 85 μm by using thermally non-shrinkable and thermally fusible fibres having fineness of 1.2 dtex, and a lower layer in which an interfibre distance was adjusted to 60 μm by using thermally shrinkable fibres having fineness of 2.3 dtex; and using the above-described diluted solution. A basis weight of the nonwoven fabric was adjusted to 74 $g/m^2$.

In the nonwoven fabric sample, a content proportion (OPU) of polyoxypropylene (POP)-modified silicone as the liquid film cleavage agent to fibre mass was adjusted to 0.1 mass%, and a content proportion (OPU) of alkly phosphoric acid ester potassium salt to fibre mass was adjusted to 0.06 mass%.

(Example 13)

A nonwoven fabric was prepared in the same manner with Example 1 except for: adjusting an effective component of a liquid film cleavage agent to 3.0 mass% by dissolving, into ethanol, polyoxypropylene (POP) alkyl ether (DEFOAMER No. 8, manufactured by Kao Corporation) as the liquid film cleavage agent which was contained in fibres, in which Z in a structure Z-Y was formed of a hydrocarbon chain composed of $-CH_2-$, Y was formed of a POP chain composed of $-(C_3H_6O)-$, the addition number of moles of polyoxypropylene was 5, and a mass average molecular weight was 500; and adjusting a content proportion (OPU) of the liquid film cleavage agent to fibre mass was adjusted to 5.0 mass%. The obtained nonwoven fabric was taken as a sample of Example 13.

With regard to the polyoxypropylene (POP) alkyl ether, a surface tension was 30.4 mN/m, and a water solubility was less than 0.0001 g. Moreover, a spreading coefficient of the polyoxypropylene (POP) alkyl ether to the liquid having surface tension of 50 mN/m was 13.7 mN/m, and an interfacial tension thereof to the liquid having surface tension of 50 mN/m was 5.9 mN/m. The numerical values were measured by the method same as in Example 1.

(Example 14)

A nonwoven fabric was prepared in the same manner with Example 1 except for: adjusting an effective component of a liquid film cleavage agent to 3.0 mass% by dissolving, into ethanol, caprylic/capric triglyceride (COCONARD MT, manufactured by Kao Corporation) as the liquid film cleavage agent, in which Z in a structure Z-Y was *-O-CH($CH_2$O-*)$_2$ (* represents a bonding portion.), Y was formed of a hydrocarbon chain of $C_8H_{15}$O- or $C_{10}H,_9$O-, a fatty acid composition was composed of 82% of caprylic acid and 18% of capric acid, and a mass average molecular weight was 550; and adjusting a content proportion (OPU) of the liquid film cleavage agent to fibre mass to 5.0 mass%. The obtained nonwoven fabric was taken as a sample of Example 14.

With regard to the caprylic/capric triglyceride, a surface tension was 28.9 mN/m, and a water solubility was less than 0.0001 g. Moreover, a spreading coefficient of the caprylic/capric triglyceride to the liquid having surface tension of 50 mN/m was 8.8 mN/m, and an interfacial tension thereof to the liquid having surface tension of 50 mN/m was 12.3 mN/m. The numerical values were measured by the method same as in Example 1.

(Example 15)

A nonwoven fabric sample in Example 15 was prepared in the same manner with Example 1 except for: adjusting an effective component of a liquid film cleavage agent to 3.0 mass% by using, as the liquid film cleavage agent, the material described below, and dissolving the material into diethyl ether; and adjusting a content proportion (OPU) of the liquid film cleavage agent to fibre mass to 5.0 mass%.

<Liquid film cleavage agent>

Liquid paraffin (manufactured by KISHIDA CHEMICAL Co., Ltd.), having a mass average molecular weight of 300
A surface tension: 30.6 mN/m
A water solubility: less than 0.0001 g
A spreading coefficient to the liquid having surface tension of 50 mN/m: 9.9 mN/m
An interfacial tension to the liquid having surface tension of 50 mN/m: 9.5 mN/m (The four numerical values were measured by the method same as in Example 1.)

(Example 16)

A nonwoven fabric sample in Example 16 was prepared in the same manner with Example 1 except for: adjusting an effective component of a liquid film cleavage agent to 3.0 mass% by using, as the liquid film cleavage agent, the material described below, and dissolving the material into ethanol; and adjusting a content proportion (OPU) of the liquid film cleavage agent to fibre mass was adjusted to 5.0 mass%.

<Liquid film cleavage agent>

Polyoxypropylene (POP) alkyl ether (UNILUBE MS-70K, manufactured by NOF CORPORATION), in which a structure Z is formed of a hydrocarbon chain composed of -$CH_2$-, Y is formed of a POP chain composed of -($C_3H_6$O)-, the mole number of a polyoxypropylene group is 15, and a mass average molecular weight is 1140.
A surface tension: 31.8 mN/m
A water solubility: less than 0.0001 g
A spreading coefficient to the liquid having surface tension of 50 mN/m: 5.4 mN/m
An interfacial tension to the liquid having surface tension of 50 mN/m: 12.8mN/m
(The above-described four numerical values were measured by the method same as in Example 1.)

(Example 17)

A nonwoven fabric sample in Example 17 was prepared in the same manner with Example 1 except for: adjusting an effective component of a liquid film cleavage agent to 3.0 mass% by using, as the liquid film cleavage agent, the material described below, and dissolving the material into ethanol; and adjusting a content proportion (OPU) of the liquid film cleavage agent to fibre mass to 5.0 mass%.

<Liquid film cleavage agent>

Polypropylene glycol (manufactured by SIGMA-ALDRICH), in which a structure Z is formed of a POP chain, the mole number of a polyoxypropylene group is 17, and a mass average molecular weight is 1000.
A surface tension: 32.4 mN/m
A water solubility: 0.0017 g
A spreading coefficient to the liquid having surface tension of 50 mN/m: 14.0 mN/m
An interfacial tension to the liquid having surface tension of 50 mN/m: 3.6 mN/m (The four numerical values were measured by the method same as in Example 1.)

(Example 18)

A nonwoven fabric sample in Example 18 was prepared in the same manner with Example 1 except for: adjusting an effective component of a liquid film cleavage agent to 3.0 mass% by using, as the liquid film cleavage agent, the material described below, and dissolving the material into hexane; and adjusting a content proportion (OPU) of the liquid film cleavage agent to fibre mass was adjusted to 5.0 mass%.

<Liquid film cleavage agent>

Liquid isoparaffin (Luvitol Lite, manufactured by BASF Japan), having a mass average molecular of 450
A surface tension: 27.0 mN/m
A water solubility: less than 0.0001 g
A spreading coefficient to the liquid having surface tension of 50 mN/m:14.5 mN/m
An interfacial tension to the liquid having surface tension of 50 mN/m: 8.5 mN/m (The four numerical values were measured by the method same as in Example 1.)

(Example 19)

A diluted solution to be applied to a nonwoven fabric was prepared in a manner similar to Example 1 with mixing polyoxypropylene (POP) alkyl ether used in Example 13 and stearyl (C18) phosphoric acid ester potassium salt (neutralised product with potassium hydroxide, GRIPPER 4131, manufactured by Kao Corporation). A content proportion of polyoxypropylene (POP) alkyl ether to stearyl (C18) phosphoric acid ester potassium salt in the diluted solution was adjusted to 3.58:1. A nonwoven fabric was prepared in the same manner with Example 13 except for: adjusting a content proportion (OPU) of polyoxypropylene (POP) alkyl ether as the liquid film cleavage agent to fibre mass to 5.0 mass%; and adjusting a content proportion (OPU) of stearyl phosphoric acid ester potassium salt to fibre mass to 1.4 mass%. The obtained nonwoven fabric was taken as a sample in Example 19. Further, as an applying method for the above-described agent, the same method as in Example 11 was adopted. {0153}

(Example 20)

A diluted solution to be applied to a nonwoven fabric was prepared in a manner similar to Example 1 with mixing caprylic/capric triglyceride used in Example 14 and stearyl (C18) phosphoric acid ester potassium salt (neutralised product with potassium hydroxide, GRIPPER 4131, manufactured by Kao Corporation). A content proportion of caprylic/capric triglyceride to stearyl (C18) phosphoric acid ester potassium salt in the diluted solution was adjusted to 3.58:1. A nonwoven fabric was prepared in the same manner with Example 14 except for: adjusting a content proportion (OPU) of caprylic/capric triglyceride as the liquid film cleavage agent to fibre mass to 5.0 mass%; and adjusting a content proportion (OPU) of stearyl phosphoric acid ester potassium salt to fibre mass to 1.4 mass%. The obtained nonwoven fabric was taken as a sample in Example 20. Further, as an applying method for the above-described agent, the same method as in Example 11 was adopted.

(Example 21)

A diluted solution to be applied to a nonwoven fabric was prepared in a manner similar to Example 1 with mixing liquid paraffin used in Example 15 and stearyl (C18) phosphoric acid ester potassium salt (neutralised product with potassium hydroxide, GRIPPER 4131, manufactured by Kao Corporation). A content proportion of liquid paraffin to stearyl (C18) phosphoric acid ester potassium salt in the diluted solution was adjusted to 3.58:1. A nonwoven fabric was prepared in the same manner with Example 15 except for: adjusting a content proportion (OPU) of liquid paraffin as the liquid film cleavage agent to fibre mass to 5.0 mass%; and adjusting a content proportion (OPU) of stearyl phosphoric

acid ester potassium salt to fibre mass to 1.4 mass%. The obtained nonwoven fabric was taken as a sample in Example 21. Further, as an applying method for the above-described agent, the same method as in Example 11 was adopted.

(Example 22)

A diluted solution to be applied to a nonwoven fabric was prepared in a manner similar to Example 1 with mixing polyoxypropylene (POP) alkyl ether used in Example 16 and stearyl (C18) phosphoric acid ester potassium salt (neutralised product with potassium hydroxide, GRIPPER 4131, manufactured by Kao Corporation). A content proportion of polyoxypropylene (POP) alkyl ether to stearyl (C18) phosphoric acid ester potassium salt in the diluted solution was adjusted to 3.58:1. A nonwoven fabric was prepared in the same manner with Example 16 except for: adjusting a content proportion (OPU) of polyoxypropylene (POP) alkyl ether as the liquid film cleavage agent to fibre mass to 5.0 mass%, and a content proportion (OPU) of stearyl phosphoric acid ester potassium salt to fibre mass to 1.4 mass%. The obtained nonwoven fabric was taken as a sample in Example 22. Further, as an applying method for the above-described agent, the same method as in Example 11 was adopted.

(Example 23)

A diluted solution to be applied to a nonwoven fabric was prepared in a manner similar to Example 1 with mixing polypropylene glycol used in Example 17 and stearyl (C18) phosphoric acid ester potassium salt (neutralised product with potassium hydroxide, GRIPPER 4131, manufactured by Kao Corporation). A content proportion of polypropylene glycol to stearyl (C18) phosphoric acid ester potassium salt in the diluted solution was adjusted to 3.58:1. A nonwoven fabric was prepared in the same manner with Example 17 except for: adjusting a content proportion (OPU) of polypropylene glycol as the liquid film cleavage agent to fibre mass to 5.0 mass%; and adjusting a content proportion (OPU) of stearyl phosphoric acid ester potassium salt to fibre mass to 1.4 mass%. The obtained nonwoven fabric was taken as a sample in Example 23. Further, as an applying method for the above-described agent, the same method as in Example 11 was adopted.

(Example 24)

A diluted solution to be applied to a nonwoven fabric was prepared in a manner similar to Example 1 with mixing liquid isoparaffin used in Example 18 and stearyl (C18) phosphoric acid ester potassium salt (neutralised product with potassium hydroxide, GRIPPER 4131, manufactured by Kao Corporation). A content proportion of liquid isoparaffin to stearyl (C18) phosphoric acid ester potassium salt in the diluted solution was adjusted to 3.58:1. A nonwoven fabric was prepared in the same manner with Example 18 except for: adjusting a content proportion (OPU) of liquid isoparaffin as the liquid film cleavage agent to fibre mass to 5.0 mass%; and adjusting a content proportion (OPU) of stearyl phosphoric acid ester potassium salt to fibre mass to 1.4 mass%. The obtained nonwoven fabric was taken as a sample in Example 24. Further, as an applying method for the above-described agent, the same method as in Example 11 was adopted.

(Example 25)

A nonwoven fabric sample in Example 25 was prepared in the same manner with Example 11 except for preparing a raw material nonwoven fabric having a concavo-convex shape shown in FIG. 10 and being formed of first fibres of thermally non-extensible and thermally fusible fibres having fineness of 3.3 dtex and second fibres of thermally extensible fibres having fineness of 4.4 dtex. Further, in the raw material nonwoven fabric, the first fibres and the second fibres were mixed at a mass ratio of 1:1. An interfibre distance at this time was adjusted to 130 $\mu$m, and a basis weight of the nonwoven fabric was adjusted to 25 g/$m^2$.

(Example 26)

A nonwoven fabric sample in Example 26 was prepared in the same manner with Example 11 except for preparing a raw material nonwoven fabric having the same concavo-convex shape as in Example 5 and being formed of first fibres of thermally non-extensible and thermally fusible fibres having fineness of 1.2 dtex and second fibres of thermally extensible fibres having fineness of 4.4 dtex. Further, in the raw material nonwoven fabric, the first fibres and the second fibres were mixed at a mass ratio of 1:1. An interfibre distance at this time was adjusted to 109 $\mu$m, and a basis weight of the nonwoven fabric was adjusted to 25 g/$m^2$.

(Example 27)

A nonwoven fabric sample in Example 27 was prepared in the same manner with Example 11 except for preparing a raw material nonwoven fabric having a concavo-convex shape shown in FIG. 5(B), in which thermally non-extensible and thermally non-shrinkable and thermally fusible fibres having fineness of 1.2 dtex were used in an upper layer, and thermally non-extensible and thermally non-shrinkable and thermally fusible fibres having fineness of 2.9 dtex were used in a lower layer. Further, an interfibre distance in the upper layer at this time was adjusted to 82 μm, an interfibre distance in the lower layer was adjusted to 104 μm, and a basis weight of the nonwoven fabric was adjusted to 30 g/m$^2$.

(Example 28)

A nonwoven fabric sample in Example 28 was prepared in the same manner with Example 11 except for preparing a raw material nonwoven fabric having a concavo-convex shape shown in FIG. 4, in which non-extensible and thermally non-shrinkable and thermally fusible fibres having fineness of 1.2 dtex were used in an upper layer, and non-extensible and thermally non-shrinkable and thermally fusible fibres having fineness of 2.3 dtex were used in a lower layer. Further, an interfibre distance in the upper layer at this time was adjusted to 86 μm, an interfibre distance in the lower layer was adjusted to 119 μm, and a basis weight of the nonwoven fabric was adjusted to 36 g/m$^2$.

(Example 29)

A raw material nonwoven fabric in the same aspect as in Example 1 was prepared. Further, an interfibre distance in an upper layer at this time was adjusted to 86 μm, and an interfibre distance in a lower layer was adjusted to 119 μm. Moreover, a liquid film cleavage agent solution (diluted solution) in which an effective component of a liquid film cleavage agent was adjusted to 2.75 mass% was prepared by diluting, with ethanol, POE3-modified silicone (polyoxyethylene (POE)-modified dimethyl silicone) which was the same as in Example 1. Next, a sample in Example 29 was prepare by applying the liquid film cleavage agent solution (diluted solution) wholly on a surface of the raw material nonwoven fabric by using Anilox rolls of 140 LPI (18 cc) in a flexographic press (Flexiproof 100, manufactured by RK Print Coat Instruments Ltd.) to be 0.4 mass% in a content proportion (OPU) to fibre mass, and then naturally drying the resultant material.

(Example 30)

A liquid film cleavage agent was attached to fibres by immersing the fibres used in Example 1 into the diluted solution used in Example 1 before the fibres were processed into a nonwoven fabric. At this time, a content proportion (OPU) of the liquid film cleavage agent to fibre mass was adjusted to 0.2 mass%. Next, in the same manner with Example 1, a concavo-convex-shaped nonwoven fabric shown in FIG. 3 formed in which thermal shrinkage occurs in part of the fibres was prepared, through a carding step and an air-through step in which the above-described fibres were used. Further, an interfibre distance in the upper layer at this time was adjusted to 86 μm, and an interfibre distance in the lower layer was adjusted to 119 μm. The obtained nonwoven fabric was taken as a sample in Example 30.

(Example 31)

A concavo-convex shaped raw material nonwoven fabric having openings as shown in FIG. 11 was prepared, and a nonwoven fabric sample in Example 31 was prepared in the same manner with Example 1. Further, in the prepared nonwoven fabric sample, thermally fusible fibres having fineness of 3.3 dtex were used in an upper layer, and thermally fusible fibres having fineness of 2.2 dtex were used in a lower layer. An interfibre distance in the upper layer at this time was adjusted to 135 μm, an interfibre distance in the lower layer was adjusted to 106 μm, and a basis weight of the nonwoven fabric was adjusted to 25 g/m$^2$.

(Comparative Example 1)

A nonwoven fabric was prepared in the same manner with Example 1 except that no liquid surface cleavage agent was incorporated to fibres. The obtained nonwoven fabric was taken as a sample in Comparative Example 1.

(Comparative Example 2)

A nonwoven fabric was prepared in the same manner with Example 1 except for: using, as an applying agent to a raw material nonwoven fabric, dimethyl silicone oil (manufactured by Shin-Etsu Chemical Co., Ltd., KF-96A-100cs);

and adjusting a content proportion (OPU) to fibre mass to 0.1 mass%. The obtained nonwoven fabric was taken as a sample in Comparative Example 2.

With regard to the dimethyl silicone oil, a surface tension was 21.0 mN/m, and a water solubility was 0.0001 g. Moreover, a spreading coefficient of the dimethyl silicone oil to the liquid having surface tension of 50 mN/m was 2.4 mN/m, and an interfacial tension thereof to the liquid having surface tension of 50 mN/m was 26.6 mN/m. The numerical values were measured by the method same as in Example 1.

(Comparative Example 3)

A nonwoven fabric was prepared in the same manner with Example 1 except for: using, as an applying agent to a raw material nonwoven fabric, water-soluble polyoxyethylene/polypropylene glycol butyl ether-modified silicone (KF6012, manufactured by Shin-Etsu Chemical Co., Ltd.) in which the addition number of moles of polyoxyethylene was 82, and a mass average molecular weight was 14400; and adjusting a content proportion (OPU) to fibre mass to 0.1 mass%. The obtained nonwoven fabric was taken as a sample in Comparative Example 3.

With regard to the water-soluble polyoxyethylene/polypropylene glycol butyl ether-modified silicone, a surface tension was 21 mN/m, and a water solubility was more than 0.025 g (> 0.025 g). However, a spreading coefficient and an interfacial tension to the liquid having surface tension of 50 mN/m were unable to be measured because the material was water-soluble, and had no extendibility. The numerical values were measured by the method same as in Example 1. Further, HLB of the water-soluble polyoxyethylene/polypropylene glycol butyl ether-modified silicone was 7.0. The HLB was at a substantially same level as "HLB of 6.4 of polyoxyethylene (POE)-modified silicone" described in Patent Literature 1.

(Comparative Example 4)

A nonwoven fabric similar to a raw material nonwoven fabric containing no liquid surface cleavage agent, which was prepared in Example 26, was taken as a nonwoven fabric sample in Comparative Example 4.

(Comparative Example 5)

A nonwoven fabric similar to a raw material nonwoven fabric containing no liquid surface cleavage agent, which was prepared in Example 27, was taken as a nonwoven fabric sample in Comparative Example 5.

(Comparative Example 6)

A nonwoven fabric similar to a raw material nonwoven fabric containing no liquid surface cleavage agent, which was prepared in Example 28, was taken as a nonwoven fabric sample in Comparative Example 6.

(Comparative Example 7)

A nonwoven fabric similar to a raw material nonwoven fabric containing no liquid surface cleavage agent, which was prepared in Example 31, was taken as a nonwoven fabric sample in Comparative Example 7.

(Evaluation)

<1> Examples 1 to 24 and 29 to 30 and Comparative Examples 1 to 3

The evaluation described below was performed by using a sanitary towel for evaluation, which was prepared by removing a topsheet from a sanitary towel (LAURIER F Shiawase Suhada, 30 cm, manufactured by Kao Corporation in 2014) as one example of an absorbent article, and laminating, in place thereof, a sample of a nonwoven fabric (hereinafter, referred to as a nonwoven fabric sample), and fixing a periphery thereof.

<2> Examples 25 and 26 and Comparative Example 4

The evaluation described below was performed by using a sanitary towel for evaluation, which was prepared by removing a topsheet from a sanitary towel (Hada-Kirei Guard, 20.5 cm, manufactured by Kao Corporation in 2014 Autumn) as one example of an absorbent article, and laminating, in place thereof, a nonwoven fabric sample, and fixing a periphery thereof.

<3> Example 27 and Comparative Example 5

The evaluation described below was performed by using a nappy for babies for evaluation, which was prepared by removing a topsheet from a nappy for babies (Merries Shun So Toki M size, tape type, manufactured by Kao Corporation in 2014) as one example of an absorbent article, and laminating, in place thereof, a nonwoven fabric sample, and fixing a periphery thereof.

<4> Example 28 and Comparative Example 6

The evaluation described below was performed by using a nappy for babies for evaluation, which was prepared by removing a topsheet from a nappy for babies (Merries Sarasara Air Through S size, tape type, manufactured by Kao Corporation in 2014) as one example of an absorbent article, and laminating, in place thereof, a nonwoven fabric sample, and fixing a periphery thereof.

<5> Example 31 and Comparative Example 7

The evaluation described below was performed by using a sanitary towel for evaluation, which was prepared by removing a topsheet from a sanitary towel (LAURIER Active Day Double Comfort, 22 cm, no wings, manufactured by Kao Corporation in 2014) as one example of an absorbent article, laminating, in place thereof, a nonwoven fabric sample, and fixing a periphery thereof.

(Amount of residual liquid in topsheet (nonwoven fabric sample))

<Examples 1 to 26 and 29 to 31, and Comparative Examples 1 to 4 and 7>

On a surface of each sanitary towel for evaluation, an acrylic plate having a permeation hole with an inner diameter of 1 cm was stacked, and a predetermined load of 100 Pa was applied to the sanitary towel. Under such a load, 6 g of defibrinated equine blood (prepared by adjusting defibrinated equine blood manufactured by NIPPON BIO-TEST LABORATORIES INC. to 8.0 cP) corresponding to menstrual blood was poured thereinto from the permeation hole of the acrylic plate. Further, the used equine blood was adjusted by TVB-10 Viscometer manufactured by Toki Sangyo Co., Ltd. under conditions of 30 rpm in advance. If the equine blood was allowed to stand, a part with high viscosity (red blood cells) precipitates, and a part with low viscosity (plasma) remains as a supernatant. A mixing ratio in the parts was adjusted to be 8.0 cP. After 60 seconds from pouring 6.0 g of defibrinated equine blood in total thereinto, the acrylic plate was removed therefrom. Next, a weight (W2) of a nonwoven fabric sample was measured, a difference (W2-W1) from a weight (W1) of the nonwoven fabric sample, a weight of which was measured in advance, before pouring the equine blood thereinto, was calculated. The operation described above was performed 3 times, and a mean value in 3 times operation was taken as an amount of residual liquid (mg). The amount of residual liquid serves as an indication as to what degree wearer's skin is wetted, and as the amount of liquid remains is lower, better results are obtained.

<Examples 27 to 28 and Comparative Examples 5 to 6>

An amount of residual liquid was tested in the same manner with the cases in <Examples 1 to 26 and 29 to 31, Comparative Examples 1 to 4 and 7> except for using, in place of the defibrinated equine blood, artificial urine (blended in a proportion of 1.94 mass% of urea, 0.795 mass% of sodium chloride, 0.11 mass% of magnesium sulphate, 0.062 mass% of calcium chloride, 0.197 mass% of potassium sulphate, 0.010 mass% of red No. 2, 96.88 mass% of water and about 0.07 mass% of POE lauryl ether, in which surface tension was adjusted to 53±1 mN/m (25°C)).

(Liquid film area proportion)

<Examples 1 to 26 and 29 to 31, and Comparative Examples 1 to 4 and 7>

A surface of a nonwoven fabric after 30 seconds from injection of the above-mentioned defibrinated equine blood thereinto was photographed by a microscope "VHX-1000" (trade name, manufactured by KEYENCE Corporation). The surface was analysed from a photographed image by using image analysis software "NewQube" (trade name, manufactured by Nexus Co., Ltd.). In the analysis, first, an RGB colour image was converted into an image of monochrome 256 tones. Then, an area of a liquid film part was calculated by performing binarisation processing to the image and extracting only a black part representing the liquid film. A value expressed in terms of a percentage of the calculated area to an area of the image was taken as a liquid film area proportion. As the liquid film area proportion is smaller, an

interfibre liquid film cleavage effect is demonstrated to be larger.

<Examples 27 to 28, and Comparative Examples 5 to 6>

An liquid film area proportion was tested in the same manner with the cases in <Examples 1 to 26 and 29 to 31, Comparative Examples 1 to 4 and 7> except for using, in place of the defibrinated equine blood, artificial urine (blended in a proportion of 1.94 mass% of urea, 0.795 mass% of sodium chloride, 0.11 mass% of magnesium sulphate, 0.062 mass% of calcium chloride, 0.197 mass% of potassium sulphate, 0.010 mass% of red No. 2, 96.88 mass% of water and about 0.07 mass% of POE lauryl ether, in which surface tension was adjusted to 53±1 mN/m (25°C)).

(L value)

<Examples 1 to 26 and 29 to 31, and Comparative Examples 1 to 4 and 7>

With regard to each nonwoven fabric sample in which the amount of residual liquid were evaluated by using the above-mentioned defibrinated equine blood, an L value in a position to which the defibrinated equine blood was charged was measured by a handy type spectrophotometer NF333 manufactured by Nippon Denshoku Industries Co., Ltd.

The L value (lightness) shows that, as the value is larger, the colour is closer to white, and redness is hard to be seen in a topsheet (nonwoven fabric sample). That is, a larger L value shows that the residual liquid between the fibres is smaller.

Further, no defibrinated equine blood was used in Examples 25 to 26 and Comparative Examples 5 to 6, and therefore the L value was not measured.

Compositions in the Examples and the Comparative Examples, and the results in each evaluation with regard to the Examples and the Comparative Examples are shown in the following Tables 1 to 6.

Table 1

| | Ex 1 | Ex 2 | Ex 3 | Ex 4 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 μm | 80 μm | 80 μm | 80 μm |
| Interfibre distance of lower layer | 60 μm | 60 μm | 60 μm | 60 μm |
| Agent applied to nonwoven fabric | POE-modified silicone (Mw: 4000) | POP-modified silicone (Mw: 4150) | POP-modified silicone (Mw: 7000) | POP-modified silicone (Mw: 5160) |
| Addition number of moles of PO-alkylene in the above agent | 3 | 3 | 10 | 10 |
| Spreading coefficient of the above agent to liquid having surface tension of 50 mN/m | 28.8 mN/m | 25.4 mN/m | 28.0 mN/m | 27.5 mN/m |
| Surface tension of the above agent | 21.0 mN/m | 21.0 mN/m | 21.5 mN/m | 21.5 mN/m |
| Interfacial tension of the above agent to liquid having surface tension of 50 mN/m | 0.2 mN/m | 3.6 mN/m | 0.5 mN/m | 1.0 mN/m |
| Water solubility of the above agent | < 0.0001 g | < 0.0001 g | < 0.0001 g | 0.0001g |
| Content proportion (OPU) of the above agent to fibre mass | 0.1 mass% | 0.1 mass% | 0.1 mass% | 0.1 mass% |
| Phosphoric acid ester type anionic surfactant | - | - | - | - |
| Content proportion (OPU) of phosphoric acid ester type anionic surfactant to fibre mass | - | - | - | - |
| Degree of liquid film formed (6 g injection, after 30 seconds) Liquid film area proportion | 1.1% | 2.9% | 1.0% | 2.1% |
| L value | 60 | 50 | 62 | 53 |
| Amount of residual liquid | 125 mg | 155 mg | 130 mg | 144 mg |

"Ex" means Example.
"Mw" means molecular weight.

Table 1 (continued-1)

| | Ex 5 | Ex 6 | Ex 7 | Ex 8 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 µm | 80 µm | 80 µm | 80 µm |
| Interfibre distance of lower layer | 60 µm | 60 µm | 60 µm | 60 µm |
| Agent applied to nonwoven fabric | POP-modified silicone (Mw: 4340) | POP-modified silicone (Mw: 12500) | PPG (Mw: 3000) | Epoxy-modified silicone (Mw: 35800) |
| Addition number of moles of PO-alkylene in the above agent | 10 | 24 | 52 | - |
| Spreading coefficient of the above agent to liquid having surface tension of 50 mN/m | 26.9 mN/m | 28.9 mN/m | 16.3 mN/m | 26.0 mN/m |
| Surface tension of the above agent | 21.5 mN/m | 20.8 mN/m | 32.7 mN/m | 21.0 mN/m |
| Interfacial tension of the above agent to liquid having surface tension of 50 mN/m | 1.6 mN/m | 0.3 mN/m | 1.0 mN/m | 3.0 mN/m |
| Water solubility of the above agent | 0.0002 g | 0.0001 g | < 0.0001 g | < 0.0001 g |
| Content proportion (OPU) of the above agent to fibre mass | 0.1 mass% | 0.1 mass% | 5.0 mass% | 0.1 mass% |
| Phosphoric acid ester type anionic surfactant | - | - | - | - |
| Content proportion (OPU) of phosphoric acid ester type anionic surfactant to fibre mass | - | - | - | - |
| Degree of liquid film formed (6 g injection, after 30 seconds) Liquid film area proportion | 2.4% | 1.0% | 1.6% | 1.9% |
| L value | 52 | 62 | 56 | 58 |
| Amount of residual liquid | 156mg | 120mg | 140mg | 129mg |

"Ex" means Example.
"Mw" means molecular weight.

Table 1 (continued-2)

| | Ex 9 | Ex 10 | Ex 11 | Ex 12 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 µm | 80 µm | 85 µm | 85 µm |
| Interfibre distance of lower layer | 60 µm | 60 µm | 60 µm | 60 µm |
| Agent applied to nonwoven fabric | Carbinol-modified silicone (Mw: 9630) | Diol-modified silicone (Mw: 6190) | POE-modified silicone (Mw: 4000) | POP-modified Silicone (Mw: 7000) |
| Addition number of moles of PO-alkylene in the above agent | - | - | 3 | 10 |
| Spreading coefficient of the above agent to liquid having surface tension of 50 mN/m | 27.3 mN/m | 27.1 mN/m | 28.8 mN/m | 28.0 mN/m |
| Surface tension of the above agent | 21.0 mN/m | 21.0 mN/m | 21.0 mN/m | 21.5 mN/m |
| Interfacial tension of the above agent to liquid having surface tension of 50 mN/m | 1.7 mN/m | 1.9 mN/m | 0.2 mN/m | 0.5 mN/m |
| Water solubility of the above agent | < 0.0001 g | < 0.0001 g | < 0.0001 g | < 0.0001 g |
| Content proportion (OPU) of the above agent to fibre mass | 0.1 mass% | 0.1 mass% | 0.1 mass% | 0.1 mass% |
| Phosphoric acid ester type anionic surfactant | - | - | Stearyl (C18) phosphoric acid ester potassium salt | Stearyl (C18) phosphoric acid ester potassium salt |
| Content proportion (OPU) of phosphoric acid ester type anionic surfactant to fibre mass | - | - | 0.06 mass% | 0.06 mass% |
| Degree of liquid film formed (6 g injection, after 30 seconds) Liquid film area proportion | 1.4% | 1.5% | 1.0% | 1.0% |
| L value | 60 | 61 | 62 | 62 |
| Amount of residual liquid | 105mg | 109mg | 89mg | 102mg |

“Ex” means Example.
“Mw” means molecular weight.

Table 2

| | Ex 13 | Ex 14 | Ex 15 | Ex 16 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 μm | 80 μm | 80 μm | 80 μm |
| Interfibre distance of lower layer | 60 μm | 60 μm | 60 μm | 60 μm |
| Agent applied to nonwoven fabric | POP alkylether (Mw: 500) | Caprylic/capric triglyceride (Mw: 550) | Liquid paraffin (Mw: 300) | POP alkylether (Mw: 1140) |
| Addition number of moles of PO-alkylene in the above agent | 5 | - | - | 15 |
| Spreading coefficient of the above agent to liquid having surface tension of 50 mN/m | 13.7 mN/m | 8.8 mN/m | 9.9 mN/m | 5.4 mN/m |
| Surface tension of the above agent | 30.4 mN/m | 28.9 mN/m | 30.6 mN/m | 31.8 mN/m |
| Interfacial tension of the above agent to liquid having surface tension of 50 mN/m | 5.9 mN/m | 12.3 mN/m | 9.5 mN/m | 12.8 mN/m |
| Water solubility of the above agent | < 0.0001 g | < 0.0001 g | < 0.0001 g | < 0.0001 g |
| Content proportion (OPU) of the above agent to fibre mass | 5.0 mass% | 5.0 mass% | 5.0 mass% | 5.0 mass% |
| Phosphoric acid ester type anionic surfactant | - | - | - | - |
| Content proportion (OPU) of phosphoric acid ester type anionic surfactant to fibre mass | - | - | - | - |
| Degree of liquid film formed (6 g injection, after 30 seconds) Liquid film area proportion | 1.2% | 2.3% | 1.4% | 1.2% |
| L value | 59 | 54 | 58 | 59 |
| Amount of residual liquid | 110 mg | 160 mg | 167 mg | 120 mg |

"Ex" means Example.
"Mw" means molecular weight.

Table 2 (continued-1)

| | Ex 17 | Ex 18 | Ex 19 | Ex 20 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 µm | 80 µm | 80 µm | 80 µm |
| Interfibre distance of lower layer | 60 µm | 60 µm | 60 µm | 60 µm |
| Agent applied to nonwoven fabric | PPG (Mw: 1000) | Liquid isoparaffin (Mw: 450) | POP alkylether (Mw: 500) | Caprylic/capric triglyceride (Mw: 550) |
| Addition number of moles of PO-alkylene in the above agent | 17 | - | 5 | - |
| Spreading coefficient of the above agent to liquid having surface tension of 50 mN/m | 14.0 mN/m | 14.5 mN/m | 13.7 mN/m | 8.8 mN/m |
| Surface tension of the above agent | 32.4 mN/m | 27.0 mN/m | 30.4 mN/m | 28.9 mN/m |
| Interfacial tension of the above agent to liquid having surface tension of 50 mN/m | 3.6 mN/m | 8.5 mN/m | 5.9 mN/m | 12.3 mN/m |
| Water solubility of the above agent | 0.0017 g | < 0.0001 g | < 0.0001 g | < 0.0001 g |
| Content proportion (OPU) of the above agent to fibre mass | 5.0 mass% | 5.0 mass% | 5.0 mass% | 5.0 mass% |
| Phosphoric acid ester type anionic surfactant | - | - | Stearyl (C18) phosphoric acid ester potassium salt | Stearyl (C18) phosphoric acid ester potassium salt |
| Content proportion (OPU) of phosphoric acid ester type anionic surfactant to fibre mass | - | - | 1.4 mass% | 1.4 mass% |
| Degree of liquid film formed (6 g injection, after 30 seconds) Liquid film area proportion | 1.5% | 1.2% | 1.2% | 1.5% |
| L value | 56 | 59 | 59 | 57 |
| Amount of residual liquid | 154 mg | 100 mg | 108 mg | 149 mg |

"Ex" means Example.
"Mw" means molecular weight.

Table 2 (continued-2)

| | Ex 21 | Ex 22 | Ex 23 | Ex 24 |
|---|---|---|---|---|
| Fineness of upper layer | 1.2 dtex | 1.2 dtex | 1.2 dtex | 1.2 dtex |
| Fineness of lower layer | 2.3 dtex | 2.3 dtex | 2.3 dtex | 2.3 dtex |
| Interfibre distance of upper layer | 80 μm | 80 μm | 80 μm | 80 μm |
| Interfibre distance of lower layer | 60 μm | 60 μm | 60 μm | 60 μm |
| Agent applied to nonwoven fabric | Liquid paraffin (Mw: 300) | POP alkylether (Mw: 1140) | PPG (Mw: 1000) | Liquid paraffin (Mw: 450) |
| Addition number of moles of PO-alkylene in the above agent | - | 15 | 17 | - |
| Spreading coefficient of the above agent to liquid having surface tension of 50 mN/m | 9.9 mN/m | 5.4 mN/m | 14.0 mN/m | 14.5 mN/m |
| Surface tension of the above agent | 30.6 mN/m | 31.8 mN/m | 32.4 mN/m | 27.0 mN/m |
| Interfacial tension of the above agent to liquid having surface tension of 50 mN/m | 9.5 mN/m | 12.8 mN/m | 3.6 mN/m | 8.5 mN/m |
| Water solubility of the above agent | < 0.0001 g | < 0.0001 g | 0.0017 g | < 0.0001 g |
| Content proportion (OPU) of the above agent to fibre mass | 5.0 mass% | 5.0 mass% | 5.0 mass% | 5.0 mass% |
| Phosphoric acid ester type anionic surfactant | Stearyl (C18) phosphoric acid ester potassium salt | Stearyl (C18) phosphoric acid ester potassium salt | Stearyl (C18) phosphoric acid ester potassium salt | Stearyl (C18) phosphoric acid ester potassium salt |
| Content proportion (OPU) of phosphoric acid ester type anionic surfactant to fibre mass | 1.4 mass% | 1.4 mass% | 1.4 mass% | 1.4 mass% |
| Degree of liquid film formed (6 g injection, after 30 seconds) Liquid film area proportion | 1.1% | 1.2% | 1.2% | 1.0% |
| L value | 61 | 59 | 59 | 62 |
| Amount of residual liquid | 135 mg | 119 mg | 150 mg | 78 mg |

"Ex" means Example.
"Mw" means molecular weight.

Table 3

| | C Ex 1 | C Ex 2 | C Ex 3 |
|---|---|---|---|
| Fineness of upper layer | 1.2 dtex | 1.2 dtex | 1.2 dtex |
| Fineness of lower layer | 2.3 dtex | 2.3 dtex | 2.3 dtex |

(continued)

| | C Ex 1 | C Ex 2 | C Ex 3 |
|---|---|---|---|
| Interfibre distance of upper layer | 80 μm | 80 μm | 80 μm |
| Interfibre distance of lower layer | 60 μm | 60 μm | 60 μm |
| Agent applied to nonwoven fabric | - | Dimethyl silicone | Water-soluble POE/PPG butyl ether-modified silicone (Mw: 14400) |
| Addition number of moles of PO-alkylene in the above agent | - | - | 82 |
| Spreading coefficient of the above agent to liquid having surface tension of 50 mN/m | - | 2.4 mN/m | - |
| Surface tension of the above agent | - | 21.0 mN/m | 21.0 mN/m |
| Interfacial tension of the above agent to liquid having surface tension of 50 mN/m | - | 26.6 mN/m | - |
| Water solubility of the above agent | - | 0.0001g | > 0.025 g |
| Content proportion (OPU) of the above agent to fibre mass | - | 0.1 mass% | 0.1 mass% |
| Phosphoric acid ester type anionic surfactant | - | - | - |
| Content proportion (OPU) of phosphoric acid ester type anionic surfactant to fibre mass | - | - | - |
| Degree of liquid film formed (6 g injection, after 30 seconds) Liquid film area proportion | 7.8% | 5.0% | 3.2% |
| L value | 41 | 45 | 49 |
| Amount of residual liquid | 280 mg | 230 mg | 234 mg |
| "C Ex" means Comparative Example.<br>"Mw" means molecular weight. | | | |

Table 4

| | Ex 25 | Ex 26 | C Ex 4 |
|---|---|---|---|
| Fineness of first fibre | 3.3dtex | 1.2 dtex | 1.2 dtex |
| Fineness of second fibre | 4.4dtex | 4.4dtex | 4.4dtex |
| Interfibre distance | 130 μm | 109 μm | 109 μm |
| Agent applied to nonwoven fabric | POE-modified silicone (Mw: 4000 | POE-modified silicone (Mw: 4000) | - |
| Addition number of moles of PO-alkylene in the above agent | 3 | 3 | - |
| Spreading coefficient of the above agent to liquid having surface tension of 50 mN/m | 28.8 mN/m | 28.8 mN/m | - |
| Surface tension of the above agent | 21.0 mN/m | 21.0 mN/m | - |
| Interfacial tension of the above agent to liquid having surface tension of 50 mN/m | 0.2 mN/m | 0.2 mN/m | - |
| Water solubility of the above agent | < 0.0001 g | < 0.0001 g | - |
| Content proportion (OPU) of the above agent to fibre mass | 0.1 mass% | 0.1 mass% | - |

(continued)

| | Ex 25 | Ex 26 | C Ex 4 |
|---|---|---|---|
| Phosphoric acid ester type anionic surfactant | Stearyl (C18) phosphoric acid ester potassium salt | Stearyl (C18) phosphoric acid ester potassium salt | - |
| Content proportion (OPU) of phosphoric acid ester type anionic surfactant to fibre mass | 0.06 mass% | 0.06 mass% | - |
| Degree of liquid film formed (6 g injection, after 30 seconds) Liquid film area proportion | 0.70% | 0.80% | 4.4% |
| L value | 63 | 63 | 46 |
| Amount of residual liquid | 30 mg | 40 mg | 195 mg |
| "Ex" means Example, and "C Ex" means Comparative Example.<br>"Mw" means molecular weight. | | | |

Table 5

| | Ex 27 | Ex 28 | C Ex 5 | C Ex 6 |
|---|---|---|---|---|
| Fineness of upper laver | 1.2 dtex | 1.2 dtex | 1.2 dtex | 1.2 dtex |
| Fineness of lower layer | 2.9dtex | 2.3 dtex | 2.9dtex | 2.3 dtex |
| Interfibre distance of upper laver | 82 µm | 86 µm | 82 µm | 86 µm |
| Interfibre distance of lower layer | 104 µm | 119 µm | 104 µm | 119 µm |
| Agent applied to nonwoven fabric | POE-modified silicone (Mw: 4000) | POE-modified silicone (Mw: 4000) | - | - |
| Addition number of moles of PO-alkylene in the above agent | 3 | 3 | - | - |
| Spreading coefficient of the above agent to liquid having surface tension of 50 mN/m | 28.8 mN/m | 28.8 mN/m | - | - |
| Surface tension of the above agent | 21.0 mN/m | 21.0 mN/m | - | - |
| Interfacial tension of the above agent to liquid having surface tension of 50 mN/m | 0.2 mN/m | 0.2 mN/m | - | - |
| Water solubility of the above agent | < 0.0001 g | < 0.0001 g | - | - |
| Content proportion (OPU) of the above agent to fibre mass | 0.1 mass% | 0.1 mass% | - | - |
| Phosphoric acid ester type anionic surfactant | Stearyl (C18) phosphoric acid ester potassium salt | Stearyl (C18) phosphoric acid ester potassium salt | - | - |
| Content proportion (OPU) of phosphoric acid ester type anionic surfactant to fibre mass | 0.06 mass% | 0.06 mass% | - | - |
| Degree of liquid film formed (6 g injection, after 30 seconds) Liquid film area proportion | 0.8% | 2.1% | 13.1% | 23.3% |

(continued)

| | Ex 27 | Ex 28 | C Ex 5 | C Ex 6 |
|---|---|---|---|---|
| Amount of residual liquid | 80 mg | 135 mg | 225 mg | 470 mq |
| "Ex" means Example, and "C Ex" means Comparative Example.<br>"Mw" means molecular weight. | | | | |

Table 6

| | Ex 29 | Ex 30 | Ex 31 | C Ex 7 |
|---|---|---|---|---|
| Fineness of upper layer | 1.2 dtex | 1.2 dtex | 3.3dtex | 3.3 dtex |
| Fineness of lower layer | 2.3 dtex | 2.3 dtex | 2.2dtex | 2.2 dtex |
| Interfibre distance of upper layer | 86 μm | 86 μm | 135 μm | 135 μm |
| Interfibre distance of lower layer | 119 μm | 119 μm | 106 μm | 106 μm |
| Agent applied to nonwoven fabric | POE-modified silicone (Mw: 4000) | POE-modified silicone (Mw: 4000) | POE-modified silicone (Mw: 4000 | - |
| Addition number of moles of PO-alkylene in the above agent | 3 | 3 | 3 | - |
| Spreading coefficient of the above agent to liquid having surface tension of 50 mN/m | 28.8 mN/m | 28.8 mN/m | 28.8 mN/m | - |
| Surface tension of the above agent | 21.0 mN/m | 21.0 mN/m | 21.0 mN/m | - |
| Interfacial tension of the above agent to liquid having surface tension of 50 mN/m | 0.2 mN/m | 0.2 mN/m | 0.2 mN/m | - |
| Water solubility of the above agent | < 0.0001 g | < 0.0001 g | < 0.0001 g | - |
| Content proportion (OPU) of the above agent to fibre mass | 0.4 mass% | 0.2 mass% | 0.1 mass% | - |
| Phosphoric acid ester type anionic surfactant | - | - | - | - |
| Content proportion (OPU) of phosphoric acid ester type anionic surfactant to fibre mass | - | - | - | - |
| Degree of liquid film formed (6 g injection, after 30 seconds) Liquid film area proportion | 0.8% | 3.5% | 0.9% | 4.7% |
| L value | 63 | 52 | 61 | 46 |
| Amount of residual liquid | 78 mg | 177 mg | 33 mg | 199 mg |
| "Ex" means Example, and "C Ex" means Comparative Example.<br>"Mw" means molecular weight. | | | | |

As shown in Tables 1 to 3 and Table 6, in Examples 1 to 24 and 29 to 31 in which the tests were conducted by using the defibrinated equine blood, an interfibre liquid film cleaved by the above-mentioned effect by the liquid film cleavage agent, and the liquid film area proportion significantly reduced in comparison with Comparative Examples 1 to 3 and 7. Further, in Examples 1 to 24 and 29 to 31, the cleavage of the interfibre liquid film results in absorbing the liquid in the absorbent body without delay, and in the nonwoven fabric in which the effect was large, the amount of residual liquid was reduced to about one third of the amount in Comparative Example, and simultaneously whiteness on the surface was significantly improved. In particular, in Example 29, the liquid film area proportion and the amount of residual liquid were the lowest, and the nonwoven fabric was excellent in a dry feeling.

In Examples 25 and 26 each shown in Table 4, the liquid film area proportion was suppressed at a level as significantly low as about 15% or about 18% of the proportion in Comparative Examples 4, and the amount of residual liquid was suppressed at a level as significantly low as about 15% or about 20% of amount in Comparative Examples 4, in which a liquid film cleavage effect was large.

As shown in Table 5, in Example 27 in which the test was conducted by using urine having smaller viscosity than the viscosity of menstrual blood, the liquid film area proportion was suppressed at a level as low as about 6% of the proportion in Comparative Examples 5, and the amount of residual liquid was suppressed at a level as low as about 35% of the amount in Comparative Examples 5. Similarly, as shown in Table 5, also in Example 28 in which the test was conducted by using urine, the liquid film area proportion was suppressed at a level as low as about 9% of the proportion in Comparative Examples 5, and the amount of residual liquid was suppressed at a level as low as about 28% of the amount in Comparative Examples 5. That is, the effect of the liquid film cleavage agent also on urine was found to be high.

Moreover, with regard to each nonwoven fabric sample, a state of attachment of the liquid film cleavage agent to the fibres was confirmed along "Confirmation method for localised state of liquid film cleavage agent" mentioned above. A photograph substituted for drawing in FIG. 12 shows a state of attachment of the liquid film cleavage agent to the fibres in the nonwoven fabric sample of Example 1. In Example 1, the raw material nonwoven fabric was immersed into the diluted solution of the liquid film cleavage agent to attach the liquid film cleavage agent to the fibres, and therefore the fibres wholly appeared somewhat white, and further as shown in a part of a circle B, a white material was partially observed in a localised manner on part of the surface of the fibres, and as shown in a part of a circle A, the white material was localised also between the fibres. That is, localisation (localisation of the liquid film cleavage agent in the vicinity of the points that fibres are entangled to each other or the points that fibres are fusion bonded to each other, for example, the part surrounded by the circle A shown in FIG. 12, and partial localisation on the surface of the fibres, for example, the part surrounded by the circle B shown in FIG. 12) was observed in a moderate manner, and as shown in each Table described above, performance was satisfactory in comparison with the Comparative Examples. The same localised states were confirmed also in Examples 2 to 28 and 31 (not shown), and the performance was satisfactory in a manner similar to Example 1.

Moreover, a photograph substituted for drawing in FIG. 13 shows a state of attachment of the liquid film cleavage agent to the fibres in the nonwoven fabric sample of Example 29. In Example 29, the cleavage agent was coated thereto by employing the flexographic press, and therefore the white material was less on the surface of the fibres, localisation was observed only in the vicinity of the points that fibres are entangled to each other or the points that fibres are fusion bonded to each other, a difference in white colour between the surface of the fibres and a localised portion was more remarkable in comparison with the case of FIG. 12 (for example, a part surrounded by a circle C shown in FIG. 13), and the performance was particularly satisfactory even in comparison with Examples 1 to 24 in which the same concavo-convex shape was formed.

On the other hand, a photograph substituted for drawing in FIG. 14 shows a state of attachment of the liquid film cleavage agent to the fibres in the nonwoven fabric sample of Example 30. In Example 30, no localisation was observed, and the surface of the fibres wholly appeared white, but the performance was further satisfactory by the effect of the liquid film cleavage agent attached to the fibres, as mentioned above, in comparison with Comparative Examples 1 to 3 and 7.

As described above, it was found that the dry feeling of the nonwoven fabric at a high level was able to be realised by containing the liquid film cleavage agent in each Example in the nonwoven fabric. Moreover, the absorbent article which is excellent in the dry feeling, is able to suppress a worry about leakage, and is conformable to wear can be provided by using the nonwoven fabric containing the liquid film cleavage agent as the topsheet.

DESCRIPTION OF SYMBOLS

1 Fibres
2 Liquid film

3 Liquid film cleavage agent
10, 20, 30, 40, 50, 60, 70 Nonwoven fabric

## Claims

1. A liquid film cleavage agent having a spreading coefficient of 15 mN/m or more to a liquid having surface tension of 50 mN/m, and a water solubility of 0 g or more and 0.025 g or less, measured according to the methods disclosed in the description, comprising a compound having at least one kind structure selected from the group consisting of the following structures X, X-Y and Y-X-Y:

   wherein the structure X designates a siloxane chain having a structure in which any of basic structures of >C(A)- (C designates a carbon atom, moreover, <, > and - each designates a bonding, hereinafter, the same applies.), $-C(A)_2-$, -C(A)(B)-, $>C(A)-C(R^1)<$, $>C(R^1)-$, $-C(R^1)(R^2)-$, $-C(R^1)_2-$, >C<, $-Si(R^1)_2O-$and $-Si(R^1)(R^2)O$ is repeated, or two or more kinds thereof are combined; or a mixed chain thereof; the structure X has, in an end of the structure X, a hydrogen atom or at least one kind of group selected from the group consisting of $-C(A)_3$, $-C(A)_2B$, $-C(A)(B)_2$, $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ and $-Si(R^1)_2(R^2)$;
   wherein $R^1$ and $R^2$ each independently designate a hydrogen atom, an alkyl group, an alkoxy group, an aryl group, or a halogen atom; A and B each independently designate a substituent having an oxygen atom or a nitrogen atom; when a plurality of $R^1$, $R^2$, A and B exist for each in the structure X, these may be identical to or different from each other; and
   wherein Y designates a hydrophilic group having hydrophilicity, the group containing an atom selected from a hydrogen atom, a carbon atom, an oxygen atom, a nitrogen atom, a phosphorus atom or a sulphur atom; and when a plurality of Y exists, these groups may be identical to or different from each other, which compound is a polyoxyalkylene modified-silicone, and wherein the addition number of moles of the polyoxyalkylene groups of the polyoxyalkylene modified-silicone is 1 or more and 10 or less.

2. The liquid film cleavage agent according to Claim 1, comprising a compound composed of a siloxane chain in which structures represented by any one of the following formulas (1) to (11) are arbitrarily combined:

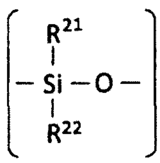 ••• (1)
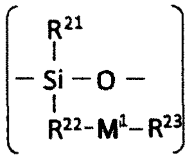 ••• (5)
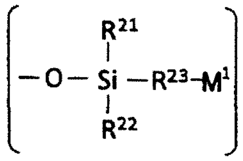 ••• (9)

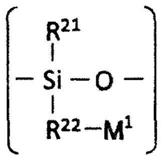 ••• (2)
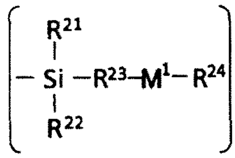 ••• (6)
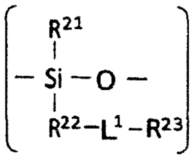 ••• (10)

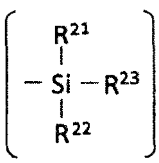 ••• (3)
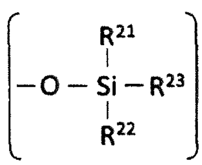 ••• (7)
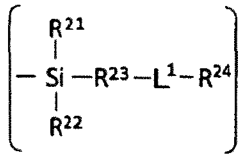 ••• (11)

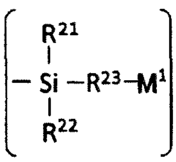 ••• (4)
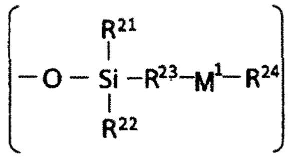 ••• (8)

wherein, in Formulas (1) to (11), $M^1$, $L^1$, $R^{21}$ and $R^{22}$ designate the following monovalent or polyvalent (divalent or more valent) group; $R^{23}$ and $R^{24}$ designate the following monovalent or polyvalent (divalent or more valent) group or a single bond;

$M^1$ designates a polyoxyethylene group, a polyoxypropylene group, a polyoxybutylene group, a group having a polyoxyalkylene group in combination therewith, an erythritol group, a xylitol group, a sorbitol group, a glycerol group or an ethylene glycol group, a hydroxyl group, a carboxylic acid group, a mercapto group, an alkoxy group, an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith; when $M^1$ is a polyvalent group, $M^1$ designates a group formed by further removing one or more hydrogen atoms from each of the groups or the functional group;

$L^1$ designates a linking group of an ether group, an amino group (the amino group adoptable as $L^1$ is represented by $>NR^C$ ($R^C$ is a hydrogen atom or a monovalent group)), an amide group, an ester group, a carbonyl group or a carbonate group; and

$R^{21}$, $R^{22}$, $R^{23}$ and $R^{24}$ each independently designate an alkyl group, an alkoxy group, an aryl group, a fluoroalkyl group, an aralkyl group, a hydrocarbon group in combination therewith, or a halogen atom.

**3.** The liquid film cleavage agent according to Claim 1 or 2, wherein the polyoxyalkylene modified-silicone is represented by any one of Formulas [I] to [IV]:

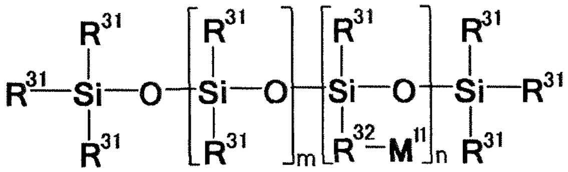
[I]

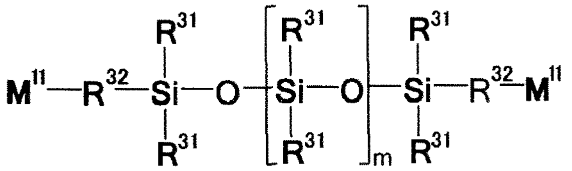
[II]

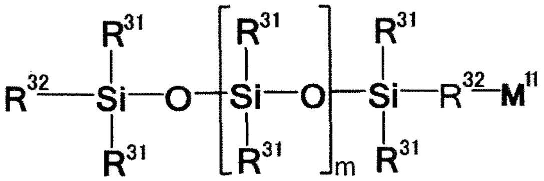
[III]

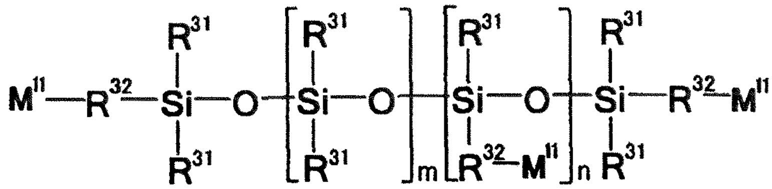
[IV]

wherein $R^{31}$ designates an alkyl group; $R^{32}$ designates a single bond or an alkylene group; a plurality of $R^{31}$ and a plurality of $R^{32}$ may be each identical to or different from each other; $M^{11}$ designates a group having a polyoxyalkylene group; as the polyoxyalkylene group, a polyoxyethylene group, a polyoxypropylene group, a polyoxybutylene group and a material in which constituent monomers thereof are copolymerised are taken; and m and n each are independently an integer of 1 or more.

**4.** A liquid film cleavage agent having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m, measured according to the methods disclosed in the description, and

comprising a compound having at least one kind structure selected from the group consisting of the following structures Z, Z-Y and Y-Z-Y:

wherein the structure Z designates a hydrocarbon chain having a structure in which any of basic structures of >C(A)- (C: carbon atom), $-C(A)_2-$, -C(A)(B)-, >C(A)-C($R^3$)<, >C($R^3$)-, -C($R^3$)($R^4$)-, $-C(R^3)_2-$ and >C< is repeated, or two or more kinds thereof are combined; the structure Z has, at an end thereof, a hydrogen atom or at least one kind of group selected from the group consisting of $-C(A)_3$, $-C(A)_2B$, $-C(A)(B)_2$, $-C(A)_2-C(R^3)_3$, $-C(R^3)_2A$ and $-C(R^3)_3$;
the $R^3$ and $R^4$ each independently designate a hydrogen atom, an alkyl group, an alkoxy group, an aryl group, a fluoroalkyl group, or an aralkyl group, or a hydrocarbon group in combination therewith, or a fluorine atom; A and B each independently designates a substituent containing an oxygen atom or a nitrogen atom;
Y designates a hydrophilic group having hydrophilicity, the hydrophilic group containing an atom selected from a hydrogen atom, a carbon atom, an oxygen atom, a nitrogen atom, a phosphorus atom and a sulphur atom; and when Y is plural, the plurality may be identical to or different from each other.

**5.** The liquid film cleavage agent according to Claim 4, comprising polyoxyalkylene alkyl ether, preferably polyoxyalkylene alkyl (POA) ether represented by any of formulas in Formula [V]; or any of polyoxyalkylene glycol, Steareth, Beheneth, PPG myristyl ether, PPG stearyl ether and PPG behenyl ether, which are represented by any of formulas in Formula [VI] and have a mass average molecular weight of 1000 or more:

$$C_mH_n-L^{21}-(C_aH_bO)_m-L^{21}-R^{51}$$

or

$$C_mH_n-L^{21}-(C_aH_bO)_m-R^{51} \qquad [V]$$

or

$$C_mH_n-L^{21}-(C_aH_bO)_m-H$$

$$R^{51}-(C_aH_bO)_m-R^{51} \qquad [VI]$$

wherein $L^{21}$ designates an ether group, an amino group, an amide group, an ester group, a carbonyl group, a carbonate group, a polyoxyethylene group, a polyoxypropylene group, a polyoxybutylene group, or a polyoxyalkylene group in combination therewith; $R^{51}$ designates a substitute of a hydrogen atom, a methyl group, an ethyl group, a propyl group, an isopropyl group, a butyl group, a pentyl group, a hexyl group, a heptyl group, 2-ethylhexyl group, a nonyl group, a decyl group, a methoxy group, an ethoxy group, a phenyl group, a fluoroalkyl group, an aralkyl group, a hydrocarbon group in combination therewith, or a fluorine atom; a, b, m and n each independently are an integer of 1 or more; $C_mH_n$ herein designates an alkyl group (n = 2m + 1), and $C_aH_b$ designates an alkylene group (a = 2b); the number of carbon atoms and the number of hydrogen atoms are each independently determined in each of Formulas (V) and (VI), and do not always represent an identical integer, and may be different from each other; "m" in $-(C_aH_bO)_m-$ is an integer of 1 or more; and values of the repeating units are each independently determined in each of Formulas (V) and (VI), and do not always represent an identical integer, and may be different from each other; or a hydrocarbon compound having 5 or more carbon atoms, and preferably 100 or less, more preferably 50 or less carbon atoms, wherein the hydrocarbon compound is preferably represented by any one of Formulas [VII] to [XV]:

$$C_mH_n-COOH \qquad [VII]$$

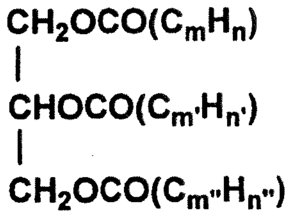

[VIII-I]

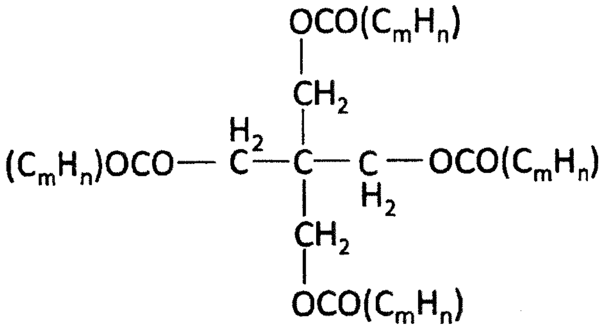

[VIII-II]

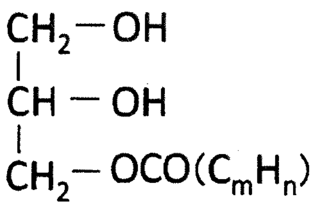

or

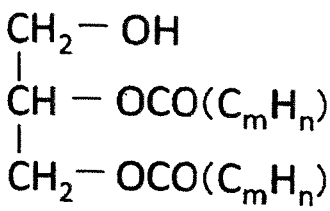

[IX]

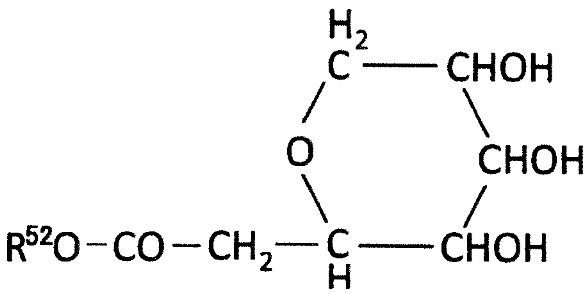

or

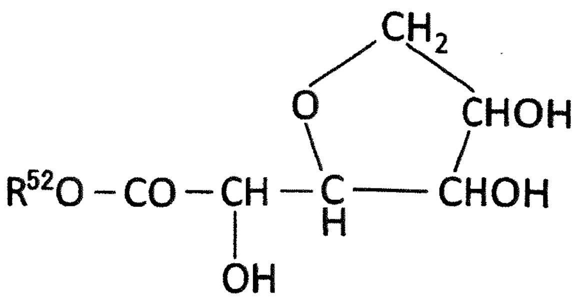

[X]

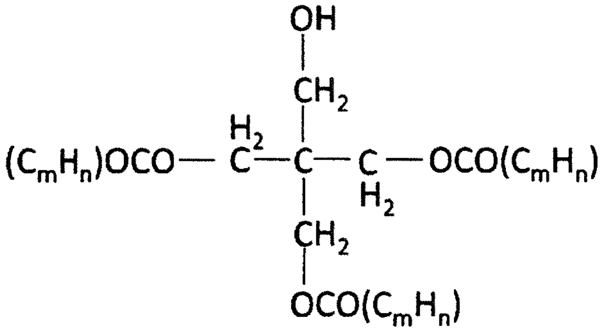

[XI]

or

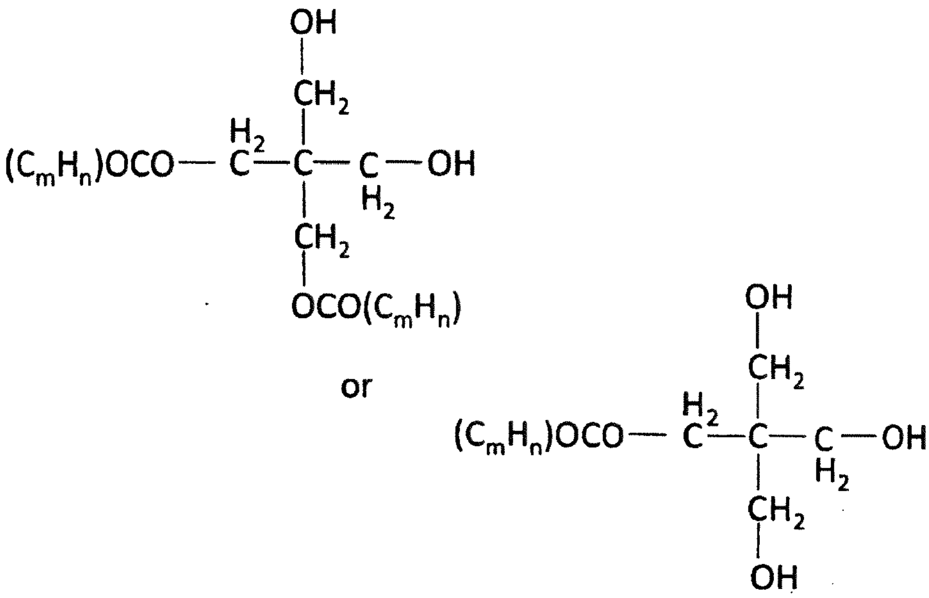

or

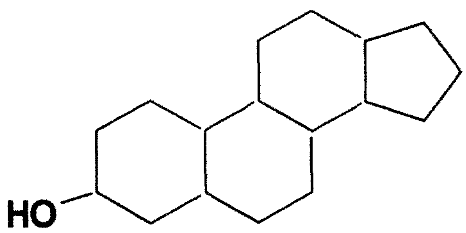

[XII]

$C_mH_n$ - OH [XIII]

$C_mH_n$ - COO - $C_mH_n$ [XIV]

$C_mH_n$ [XV]

wherein, in Formulas [VII] to [XV], m, m', m", n, n' and n" each independently are an integer of 1 or more; a plurality of m or a plurality of n each may be identical to or different from each other; and in Formula [X], $R^{52}$ designates a straight-chain or branched-chain, or saturated or unsaturated hydrocarbon group having 2 or more and 22 or less carbon atoms.

6. The liquid film cleavage agent according to any one of Claims 1 to 5, which has a mass average molecular weight of 500 or more, more preferably 1,000 or more, further preferably 1,500 or more, and particularly preferably 2,000 or more; and preferably 50,000 or less, more preferably 20,000 or less, and further preferably 10,000 or less.

7. A fibre treating agent containing the liquid film cleavage agent according to any one of Claims 1 to 6 in an amount

of 5 mass% or more and 50 mass% or less, and optionally a phosphoric acid ester type anionic surfactant.

**8.** A nonwoven fabric containing the liquid film cleavage agent according to any one of Claims 1 to 6, and optionally a phosphoric acid ester type anionic surfactant.

**9.** The nonwoven fabric according to Claim 8, wherein a content ratio of the liquid film cleavage agent to the phosphoric acid ester type anionic surfactant is preferably (1:1) to (19:1), more preferably (2:1) to (15:1), and further preferably (3:1) to (10:1) in terms of a mass ratio.

**10.** A topsheet for an absorbent article in which the nonwoven fabric according to Claim 8 or 9 is used,
wherein the topsheet has at least two layers;
the topsheet has a plurality of concave bonding portions in which the layers are bonded to each other with the compression from a skin-contact surface side in a thickness direction;
a layer on a skin non-contact surface side of the topsheet is a layer formed in which thermally shrinkable fibres are being thermally shrunk; and
a layer on the skin-contact surface side of the topsheet has thermally non-shrinkable fibres partially bonded at the bonding portion, and has convex portions projecting on the skin-contact surface side in a region among the concave bonding portions to form a concavo-convex surface of the nonwoven fabric, or
wherein the topsheet has a double-layered structure having a hollow portion and formed of a first nonwoven fabric on a skin-contact surface side and a second nonwoven fabric on a skin non-contact surface side, and both layers contain thermoplastic fibres; and
the topsheet has a bonding portion in which the first nonwoven fabric and the second nonwoven fabric are partially thermally fusion bonded, and in a non-bonding portion surrounded by the bonding portions, the first. nonwoven fabric has a large number of convex portions projecting in a direction away from the second nonwoven fabric with the hollow portions inside thereof, and the bonding portion is a concave portion positioned between the adjacent convex portions and constitutes a concavo-convex shape on the skin-contact surface side in cooperation with the convex portions, or
wherein the topsheet comprises one layer containing thermoplastic fibres and has a concavo-convex shape for both sides;
a first projecting portion projecting on a side of a first surface and a second projecting portion projecting on a side of a second surface are alternately continuously arranged in different intersecting directions in a planar view of the nonwoven fabric; and
the first projecting portion and the second projecting portion each have an internal space opened on a side of each opposite surface, which forms concave portions on the each surface, or
wherein the topsheet comprises two layers containing thermoplastic fibres;
a first projecting portion projecting on a side of a first surface and a second projecting portion projecting on a side of a second surface are alternately continuously arranged in different intersecting directions in a planar view of the nonwoven fabric, and the first projecting portion and the second projecting portion each have an internal space opened on a side of each opposite surface; and
a first fibre layer has a concave portion formed in the internal space on the each surface, and a second fibre layer is arranged along a concavo-convex shape on the side of the second surface of the first fibre layer and bonded with the first fibre layer, and a side of the first fibre layer is applied as a skin-contact surface side, or
wherein the topsheet comprises one layer containing thermoplastic fibres, and has a shape in which a plurality of hemicylindrical convex portions and a plurality of concave portions arranged along side edges of the convex portions are alternately arranged on a skin-contact surface side; and
concave bottom portions comprising fibres of the nonwoven fabric are arranged on a lower side of the concave portions, and a fibre density of the concave bottom portions are smaller than a fibre density of the convex portions.

**11.** An absorbent article, employing the nonwoven fabric according to Claim 8 or 9, or containing the topsheet according to Claim 10.

**12.** A method for producing the nonwoven fabric according to Claim 8 or 9, comprising a step of immersing, into a solution containing a liquid film cleavage agent, a raw material nonwoven fabric prepared by thermally fusing fibres to each other by air-through processing or heat embossing, or a step of coating a liquid film cleavage agent alone or a solution containing the liquid film cleavage agent to a raw material nonwoven fabric prepared by thermally fusing fibres to each other by air-through processing or heat embossing.

**13.** Use of a compound having a spreading coefficient of 15 mN/m or more to a liquid having surface tension of 50

mN/m, and a water solubility of 0 g or more and 0.025 g or less, , measured according to the methods disclosed in the description, as a liquid film cleavage agent, or having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m, measured according to the methods disclosed in the description, as a liquid film cleavage agent.

## Patentansprüche

1. Ein Flüssigkeitsfilmspaltungsmittel mit einem Spreitungskoeffizienten von 15 mN/m oder mehr zu einer Flüssigkeit mit einer Oberflächenspannung von 50 mN/m und einer Wasserlöslichkeit von 0 g oder mehr und 0,025 g oder weniger, gemessen nach den in der Beschreibung offengelegten Verfahren, umfassend eine Verbindung mit mindestens einer Art Struktur, ausgewählt aus der Gruppe bestehend aus den folgenden Strukturen X, X-Y und Y-X-Y:

   wobei die Struktur X eine Siloxankette mit einer Struktur bezeichnet, in welcher jede beliebige der Basisstrukturen von >C(A)- (C bezeichnet ein Kohlenstoffatom, ferner bezeichnen <, > und - jeweils eine Bindung, wobei im folgenden dasselbe gilt), $-C(A)_2-$, -C(A)(B)-, $>C(A)-C(R^1)<$, $>C(R^1)-$, $-C(R^1)(R^2)-$, $-C(R^1)_2-$, >C<, $-Si(R^1)_2O-$und $-Si(R^1)(R^2)O$ wiederholt wird, oder zwei oder mehrere Arten davon kombiniert werden, oder eine gemischte Kette davon; die Struktur X, an einem Ende der Struktur X, ein Wasserstoffatom oder mindestens eine Art von Gruppe, ausgewählt aus der Gruppe bestehend aus $-C(A)_3$, $-C(A)_2B$, $-C(A)(B)_2$ $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ und $-Si(R^1)_2(R^2)$ aufweist;
   wobei $R^1$ und $R^2$ jeweils unabhängig ein Wasserstoffatom, eine Alkylgruppe, eine Alkoxygruppe, eine Arylgruppe oder ein Halogenatom bezeichnen; A und B jeweils unabhängig einen Substituenten mit einem Sauerstoffatom oder einem Stickstoffatom bezeichnen; wenn eine Mehrzahl an $R^1$, $R^2$, A und B für jede Struktur X vorliegen, diese gleich oder verschieden voneinander sein können; und
   wobei Y eine hydrophile Gruppe mit hydrophiler Eigenschaft bezeichnet, wobei die Gruppe ein Atom, ausgewählt aus einem Wasserstoffatom, einem Kohlenstoffatom, einem Sauerstoffatom, einem Stickstoffatom, einem Phosphoratom oder einem Schwefelatom enthält; und wenn eine Mehrzahl an Y vorliegt, diese Gruppen gleich oder verschieden voneinander sein können, wobei die Verbindung ein Polyoxyalkylen-modifiziertes Silikon darstellt und wobei die Mol-Additionszahl der Polyoxyalkylengruppen des Polyoxyalkylen-modifizierten Silikons 1 oder mehr und 10 oder weniger beträgt.

2. Das Flüssigkeitsfilmspaltungsmittel gemäß Anspruch 1, umfassend eine Verbindung zusammengesetzt aus einer Siloxankette, in welcher Strukturen, dargestellt durch eine der folgenden Formeln (1) bis (11), beliebig kombiniert sind:

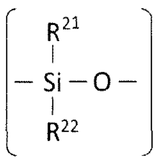 ··· (1) 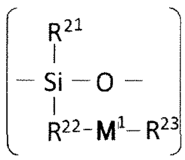 ··· (5) 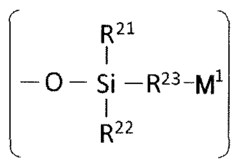 ··· (9)

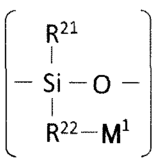 ··· (2) 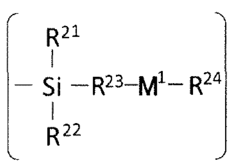 ··· (6) 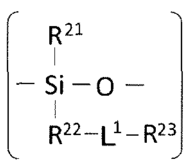 ··· (10)

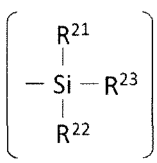 ··· (3) 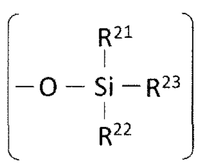 ··· (7) 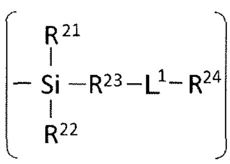 ··· (11)

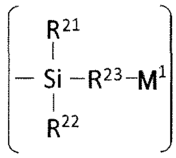 ··· (4) 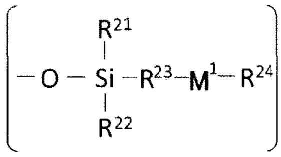 ··· (8)

wobei, in den Formeln (1) bis (11), $M^1$, $L^1$, $R^{21}$ und $R^{22}$ die folgende einwertige oder mehrwertige (zweiwertige oder höherwertige) Gruppe bezeichnen; $R^{23}$ und $R^{24}$ die folgende einwertige oder mehrwertige (zweiwertige oder höherwertige) Gruppe oder eine Einfachbindung bezeichnen;

$M^1$ eine Polyoxyethylengruppe, eine Polyoxypropylengruppe, eine Polyoxybutylengruppe, eine Gruppe mit einer Polyoxyalkylengruppe in Kombination damit, eine Erythritolgruppe, eine Xylitolgruppe, eine Sorbitolgruppe, eine Glyceringruppe oder eine Ethylenglykolgruppe, eine Hydroxylgruppe, eine Carbonsäuregruppe, eine Mercaptogruppe, eine Alkoxygruppe, eine Aminogruppe, eine Amidgruppe, eine Iminogruppe, eine Phenolgruppe, eine Sulfonsäuregruppe, eine quaternäre Ammoniumgruppe, eine Sulfobetaingruppe, eine Hydroxysulfobetaingruppe, eine Phosphobetaingruppe, eine Imidazoliumbetaingruppe, eine Carbobetaingruppe, eine Epoxidgruppe, eine Carbinolgruppe, eine (Meth)acrylgruppe oder eine funktionelle Gruppe in Kombination damit; wenn $M^1$ eine mehrwertige Gruppe ist, $M^1$ eine Gruppe bezeichnet, welche durch weiteres Entfernen eines oder mehrerer Wasserstoffatome aus jeder der Gruppen oder der funktionellen Gruppe gebildet ist;

$L^1$ eine Verbindungsgruppe einer Ethergruppe, einer Aminogruppe (die als $L^1$ annehmbare Aminogruppe wird durch $>NR^C$ dargestellt ($R^C$ stellt ein Wasserstoffatom oder eine einwertige Gruppe dar)), einer Amidgruppe, einer Estergruppe, einer Carbonylgruppe oder einer Carbonatgruppe bezeichnet; und

$R^{21}$, $R^{22}$, $R^{23}$ und $R^{24}$ jeweils unabhängig eine Alkylgruppe, eine Alkoxygruppe, eine Arylgruppe, eine Fluoralkylgruppe, eine Aralkylgruppe, eine Kohlenwasserstoffgruppe in Kombination damit oder ein Halogenatom bezeichnen.

**3.** Das Flüssigkeitsfilmspaltungsmittel gemäß Anspruch 1 oder 2, wobei das Polyoxyalkylen-modifizierte Silikon durch eine der Formeln [1] bis [IV] dargestellt ist:

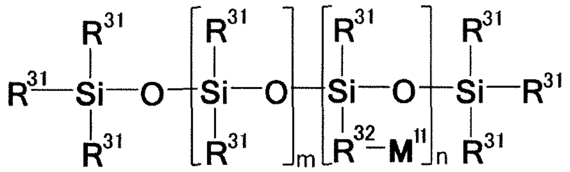 [I]

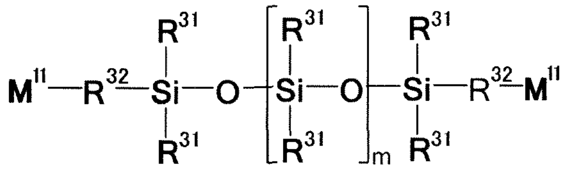 [II]

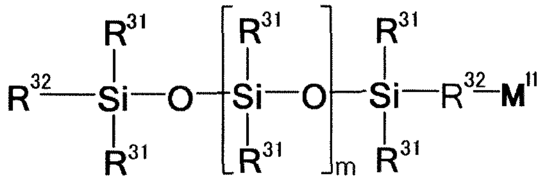 [III]

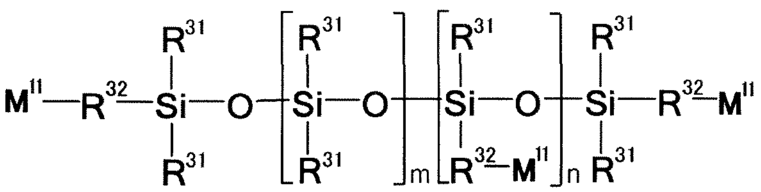 [IV]

wobei $R^{31}$ eine Alkylgruppe bezeichnet; $R^{32}$ eine Einfachbindung oder eine Alkylengruppe bezeichnet; eine Mehrzahl an $R^{31}$ und eine Mehrzahl an $R^{32}$ gleich oder verschieden voneinander sein können; $M^{11}$ eine Gruppe bezeichnet, welche eine Polyoxyalkylengruppe aufweist; als die Polyoxyalkylengruppe werden eine Polyoxyethylengruppe, eine Polyoxypropylengruppe, eine Polyoxybutylengruppe und ein Material, in welchem konstituierende Monomere davon copolymerisiert sind, genommen; und m und n jeweils unabhängig eine ganze Zahl von 1 oder mehr darstellen.

**4.** Ein Flüssigkeitsfilmspaltungsmittel mit einem Spreitungskoeffizienten von mehr als 0 mN/m zu einer Flüssigkeit mit einer Oberflächenspannung von 50 mN/m und einer Wasserlöslichkeit von 0 g oder mehr und 0,025 g oder weniger, und einer Grenzflächenspannung von 20 mN/m oder weniger zu der Flüssigkeit mit einer Oberflächenspannung von 50 mN/m, gemessen nach den in der Beschreibung offengelegten Verfahren, und umfassend eine Verbindung mit mindestens einer Art Struktur, ausgewählt aus der Gruppe bestehend aus den folgenden Strukturen Z, Z-Y und Y-Z-Y:

wobei die Struktur Z eine Kohlenwasserstoffkette mit einer Struktur bezeichnet, in welcher jede beliebige der Basisstrukturen von >C(A)- (C: Kohlenstoffatom), $-C(A)_2-$, -C(A)(B)-, >C(A)-C($R^3$)<, >C($R^3$)-, -C($R^3$)($R^4$)-, $-C(R^3)_2-$ und >C<, wiederholt wird, oder zwei oder mehrere Arten davon kombiniert werden; die Struktur Z, an einem Ende davon, ein Wasserstoffatom oder mindestens eine Art von Gruppe, ausgewählt aus der Gruppe bestehend aus $-C(A)_3$, $-C(A)_2B$, $-C(A)(B)_2$, $-C(A)_2-C(R^3)_3$, $-C(R^3)_2A$ und $-C(R^3)_3$, aufweist;
$R^3$ und $R^4$ jeweils unabhängig ein Wasserstoffatom, eine Alkylgruppe, eine Alkoxygruppe, eine Arylgruppe, eine Fluoralkylgruppe oder eine Aralkylgruppe oder eine Kohlenwasserstoffgruppe in Kombination damit oder ein Fluoratom bezeichnen; A und B jeweils unabhängig einen Substituenten, welcher ein Sauerstoffatom oder ein Stickstoffatom enthält, bezeichnen;
Y eine hydrophile Gruppe mit hydrophiler Eigenschaft bezeichnet, wobei die hydrophile Gruppe ein Atom, ausgewählt aus einem Wasserstoffatom, einem Kohlenstoffatom, einem Sauerstoffatom, einem Stickstoffatom, einem Phosphoratom und einem Schwefelatom enthält; und wenn Y mehrfach vorliegt, diese Mehrzahl gleich oder verschieden voneinander sein kann.

**5.** Das Flüssigkeitsfilmspaltungsmittel gemäß Anspruch 4, umfassend Polyoxyalkylenalkylether, bevorzugt Polyoxyalkylenalkyl (POA)-Ether, dargestellt durch jede beliebige der Formeln in Formel [V]; oder jedes beliebige von Polyoxyalkylenglykol, Steareth, Beheneth, PPG-Myristylether, PPG-Stearylether und PPG-Behenylether, welche durch jede beliebige der Formeln in Formel [VI] dargestellt sind und ein massegemitteltes Molekulargewicht von 1000 oder mehr aufweisen:

$$C_mH_n-L^{21}-\left(C_aH_bO\right)_m-L^{21}-R^{51}$$

or

$$C_mH_n-L^{21}-\left(C_aH_bO\right)_m-R^{51} \qquad [V]$$

or

$$C_mH_n-L^{21}-\left(C_aH_bO\right)_m-H$$

$$R^{51}-\left(C_aH_bO\right)_m-R^{51} \qquad [VI]$$

wobei $L^{21}$ eine Ethergruppe, eine Aminogruppe, eine Amidgruppe, eine Estergruppe, eine Carbonylgruppe, eine Carbonatgruppe, eine Polyoxyethylengruppe, eine Polyoxypropylengruppe, eine Polyoxybutylengruppe oder eine Polyoxyalkylengruppe in Kombination damit bezeichnet; $R^{51}$ ein Substitut eines Wasserstoffatoms, eine Methylgruppe, eine Ethylgruppe, eine Propylgruppe, eine Isopropylgruppe, eine Butylgruppe, eine Pentylgruppe, eine

Hexylgruppe, eine Heptylgruppe, 2-Ethylhexylgruppe, eine Nonylgruppe, eine Decylgruppe, eine Methoxygruppe, eine Ethoxygruppe, eine Phenylgruppe, eine Fluoralkylgruppe, eine Aralkylgruppe, eine Kohlenwasserstoffgruppe in Kombination damit oder ein Fluoratom bezeichnet; a, b, m und n jeweils unabhängig eine ganze Zahl von 1 oder mehr darstellen; $C_mH_n$ eine Alkylgruppe (n = 2m + 1) bezeichnet und $C_aH_b$ eine Alkylengruppe (a = 2b) bezeichnet; die Anzahl der Kohlenstoffatome und die Anzahl der Wasserstoffatome jeweils unabhängig in jeder der Formeln (V) und (VI) bestimmt sind und nicht immer eine identische ganze Zahl darstellen und unterschiedlich voneinander sein können; "m" in $-(C_aH_bO)_m-$ eine ganze Zahl von 1 oder mehr darstellt; und Werte der Wiederholungseinheiten jeweils unabhängig in jeder der Formeln (V) und (VI) bestimmt sind und nicht immer eine identische ganze Zahl darstellen und unterschiedlich voneinander sein können; oder eine Kohlenwasserstoffverbindung mit 5 oder mehr Kohlenstoffatomen, und bevorzugt 100 oder weniger, stärker bevorzugt 50 oder weniger Kohlenstoffatomen, wobei die Kohlenwasserstoffverbindung vorzugsweise durch eine beliebige der Formeln [VII] bis [XV] dargestellt ist:

$C_mH_n$ - COOH [VII]

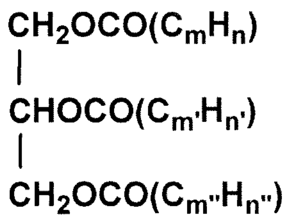

[VIII-I]

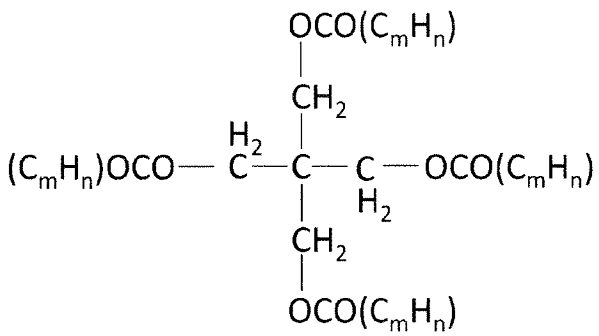

[VIII-II]

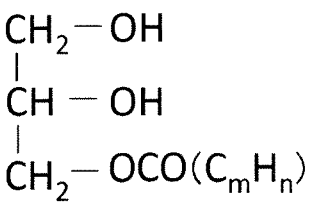

or

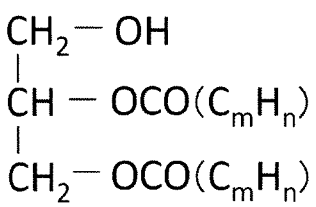

[IX]

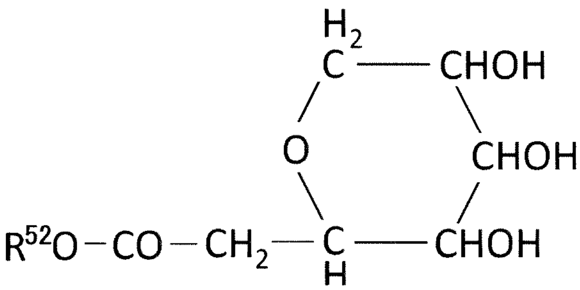

or

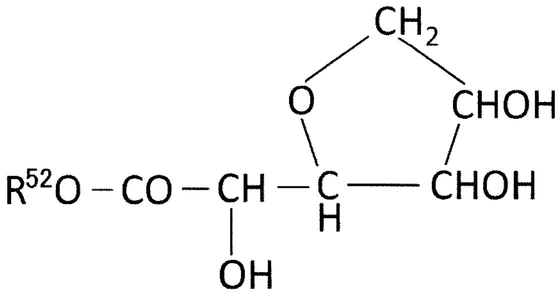

[X]

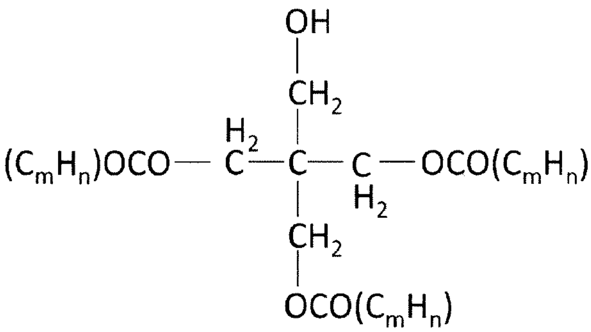

[XI]

or

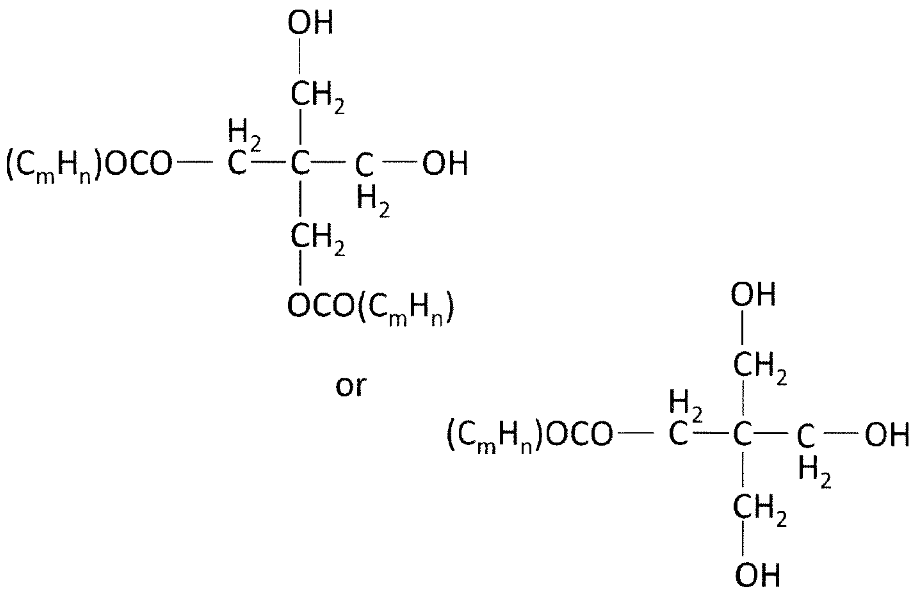

or

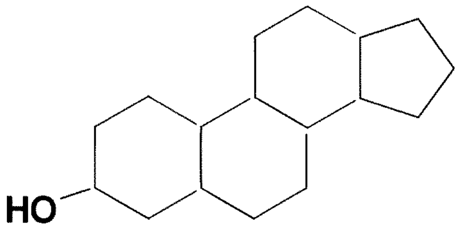

[XII]

$C_mH_n$ - OH [XIII]

$C_mH_n$ - COO - $C_mH_n$ [XIV]

$C_mH_n$ [XV]

wobei in den Formeln [VII] bis [XV], m, m', m", n, n' und n" jeweils unabhängig eine ganze Zahl von 1 oder mehr darstellen; eine Mehrzahl an m oder eine Mehrzahl an n jeweils gleich oder unterschiedlich voneinander sein kann; und $R^{52}$ in Formel [X] eine geradkettige oder verzweigtkettige oder gesättigte oder ungesättigte Kohlenwasserstoffgruppe mit 2 oder mehr und 22 oder weniger Kohlenstoffatomen bezeichnet.

**6.** Das Flüssigkeitsfilmspaltungsmittel gemäß einem der Ansprüche 1 bis 5, welches ein massegemitteltes Molekulargewicht von 500 oder mehr, stärker bevorzugt 1000 oder mehr, noch stärker bevorzugt 1500 oder mehr und am stärksten bevorzugt 2000 oder mehr; und bevorzugt 50,000 oder weniger, stärker bevorzugt 20,000 oder weniger und noch stärker bevorzugt 10,000 oder weniger aufweist.

**7.** Ein Faserbehandlungsmittel enthaltend das Flüssigkeitsfilmspaltungsmittel gemäß einem der Ansprüche 1 bis 6 in einer Menge von 5 Massen-% oder mehr und 50 Massen-% oder weniger und gegebenenfalls ein anionisches grenzflächenaktives Mittel vom Phosphorsäureester-Typ.

**8.** Ein Vliesstoff, enthaltend das Flüssigkeitsfilmspaltungsmittel gemäß einem der Ansprüche 1 bis 6 und gegebenenfalls ein anionisches grenzflächenaktives Mittel vom Phosphorsäureester-Typ.

**9.** Der Vliesstoff gemäß Anspruch 8, wobei ein Gehaltsverhältnis des Flüssigkeitsfilmspaltungsmittels zu dem anionischen grenzflächenaktiven Mittel vom Phosphorsäureester-Typ vorzugsweise (1:1) bis (19:1), stärker bevorzugt (2:1) bis (15:1) und noch stärker bevorzugt (3:1) bis (10:1), bezogen auf ein Massenverhältnis, beträgt.

**10.** Eine Deckschicht für einen absorbierenden Gegenstand, in welchem der Vliesstoff gemäß Anspruch 8 oder 9 verwendet wird,
wobei die Deckschicht mindestens zwei Schichten aufweist;
die Deckschicht eine Mehrzahl an konkaven Verbindungsabschnitten aufweist, in welchen die Schichten mit dem Druck von einer mit der Haut in Berührung stehenden Oberflächenseite aus in Dickenrichtung miteinander verbunden werden;
eine Schicht auf einer nicht mit der Haut in Berührung stehenden Oberflächenseite der Deckschicht eine Schicht ist, in welcher thermisch schrumpfbare Fasern thermisch geschrumpft werden; und
eine Schicht auf der mit der Haut in Berührung stehenden Oberflächenseite der Deckschicht thermisch nichtschrumpfbare Fasern, welche teilweise an den Verbindungsabschnitten gebunden sind, aufweist und konvexe Abschnitte aufweist, die auf der mit der Haut in Berührung stehenden Oberflächenseite in einem Bereich zwischen den konkaven Verbindungsabschnitten vorstehen, um eine konkav-konvexe Oberfläche des Vliesstoffs zu bilden oder
wobei die Deckschicht eine doppellagige Struktur mit einem hohlen Abschnitt aufweist und aus einem ersten Vliesstoff auf einer mit der Haut in Berührung stehenden Oberflächenseite und einem zweiten Vliesstoff auf einer nicht mit der Haut in Berührung stehenden Oberflächenseite gebildet ist und beide Schichten thermoplastische Fasern enthalten; und
die Deckschicht einen Verbindungsabschnitt aufweist, in welchem der erste Vliesstoff und der zweite Vliesstoff teilweise thermisch schmelzverbunden sind und in einem von den Verbindungsabschnitten umgebenen nicht verbindenden Abschnitt der erste Vliesstoff eine große Anzahl von konvexen Abschnitten aufweist, die in einer Richtung weg vom zweiten Vliesstoff mit den hohlen Abschnitten im Inneren desselben vorstehen, und der Verbindungsabschnitt ein konkaver Abschnitt ist, der zwischen den benachbarten konvexen Abschnitten angeordnet ist und im

Zusammenwirken mit den konvexen Abschnitten eine konkav-konvexe Form auf der mit der Haut in Berührung stehenden Oberflächenseite bildet, oder

wobei die Deckschicht eine Schicht umfasst, welche thermoplastische Fasern enthält und eine konkav-konvexe Form für beide Seiten aufweist;

ein erster vorstehender Abschnitt, der auf einer Seite einer ersten Oberfläche vorsteht, und ein zweiter vorstehender Abschnitt, der auf einer Seite einer zweiten Oberfläche vorsteht, in einer ebenen Ansicht des Vliesstoffs abwechselnd kontinuierlich in verschiedenen sich kreuzenden Richtungen angeordnet sind; und

der erste vorstehende Abschnitt und der zweite vorstehende Abschnitt jeweils einen Innenraum aufweisen, der auf einer Seite jeder gegenüberliegenden Oberfläche geöffnet ist, wodurch konkave Abschnitte auf jeder Oberfläche gebildet werden, oder

wobei die Deckschicht zwei Schichten umfasst, welche die thermoplastischen Fasern aufweisen;

ein erster vorstehender Abschnitt, der auf einer Seite einer ersten Oberfläche vorsteht, und ein zweiter vorstehender Abschnitt, der auf einer Seite einer zweiten Oberfläche vorsteht, in einer ebenen Ansicht des Vliesstoffs abwechselnd kontinuierlich in verschiedenen sich kreuzenden Richtungen angeordnet sind und der erste vorstehende Abschnitt und der zweite vorstehende Abschnitt jeweils einen Innenraum aufweisen, welcher auf einer Seite jeder der gegenüberliegenden Oberfläche geöffnet ist; und

eine erste Faserschicht einen konkaven Abschnitt aufweist, welcher in dem Innenraum auf jeder Oberfläche gebildet ist, und eine zweite Faserschicht entlang einer konkav-konvexen Form auf der Seite der zweiten Oberfläche der ersten Faserschicht angeordnet ist und mit der ersten Faserschicht verbunden ist, und eine Seite der ersten Faserschicht als eine mit der Haut in Berührung stehenden Oberflächenseite aufgebracht wird, oder

wobei die Deckschicht eine Schicht umfasst, welche thermoplastische Fasern umfasst und eine Form aufweist, in welcher eine Mehrzahl an halbzylindrischen konvexen Abschnitten und eine Mehrzahl an konkaven Abschnitten, welche entlang seitlicher Kanten der konvexen Abschnitte angeordnet sind, abwechselnd auf einer mit der Haut in Berührung stehenden Oberflächenseite angeordnet sind; und

konkave untere Abschnitte, welche Fasern des Vliesstoffs umfassen, auf einer Unterseite der konkaven Teile angeordnet sind und eine Faserdichte der konkaven unteren Abschnitte kleiner als eine Faserdichte der konvexen Abschnitte ist.

**11.** Ein absorbierender Gegenstand, welcher den Vliesstoff gemäß Anspruch 8 oder 9 verwendet oder die Deckschicht gemäß Anspruch 10 enthält.

**12.** Ein Verfahren zur Herstellung des Vliesstoffs gemäß Anspruch 8 oder 9, umfassend einen Schritt des Eintauchens eines Rohmaterial-Vliesstoffs, der durch thermisches Verschmelzen von Fasern miteinander durch Luftdurchgangsverarbeitung oder Wärmeprägung hergestellt wird, in eine Lösung, die ein Flüssigkeitsfilmspaltungsmittel enthält, oder einen Schritt des Beschichtens eines Rohmaterial-Vliesstoffs, der durch thermisches Verschmelzen von Fasern miteinander durch Luftdurchgangsverarbeitung oder Wärmeprägung hergestellt wird mit einem Flüssigkeitsfilmspaltungsmittel allein oder einer Lösung, die das Flüssigkeitsfilmspaltungsmittel enthält.

**13.** Verwendung einer Verbindung mit einem Spreitungskoeffizienten von 15 mN/m oder mehr zu einer Flüssigkeit mit einer Oberflächenspannung von 50 mN/m und einer Wasserlöslichkeit von 0 g oder mehr und 0,025 g oder weniger, gemessen nach den in der Beschreibung offengelegten Verfahren, als ein Flüssigkeitsfilmspaltungsmittel, oder mit einem Spreitungskoeffizienten von mehr als 0 mN/m zu einer Flüssigkeit mit einer Oberflächenspannung von 50 mN/m, einer Wasserlöslichkeit von 0 g oder mehr und 0,025 g oder weniger und einer Grenzflächenspannung von 20 mN/m oder weniger zu der Flüssigkeit mit einer Oberflächenspannung von 50 mN/m, gemessen nach den in der Beschreibung offengelegten Verfahren, als ein Flüssigkeitsfilmspaltungsmittel.

## Revendications

**1.** Agent de clivage de film liquide ayant un coefficient d'étalement de 15 mN/m ou plus vis-à-vis d'un liquide ayant une tension de surface de 50 mN/m, et une solubilité dans l'eau de 0 g ou plus et de 0,025 g ou moins, mesurés conformément aux méthodes divulguées dans la description, comprenant un composé ayant au moins un type de structure choisi dans le groupe constitué par les structures suivantes X, X-Y et Y-X-Y :

dans lequel la structure X désigne une chaîne siloxane ayant une structure dans laquelle n'importe laquelle des structures basiques de >C(A)- (C désigne un atome de carbone, de plus, chacun de <, > et - désigne une liaison, la même chose s'applique ci-après), $-C(A)_2-$, -C(A)(B)-, $>C(A)-C(R^1)<$, $>C(R^1)-$, $-C(R^1)(R^2)-$, $-C(R^1)_2-$, >C<, $-Si(R^1)_2O-$ et $-Si(R^1)(R^2)O$ est répétée, ou deux ou plus de deux types de celles-ci sont combinés ; ou

une chaîne mixte de celles-ci ; la structure X a, à une extrémité de la structure X, un atome d'hydrogène ou au moins un type de groupe choisi dans le groupe constitué par $-C(A)_3$, $-C(A)_2B$, $-C(A)(B)_2$, $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ et $-Si(R^1)_2(R^2)$ ;

dans lequel chacun de $R^1$ et $R^2$ désigne indépendamment un atome d'hydrogène, un groupe alkyle, un groupe alcoxy, un groupe aryle, ou un atome d'halogène ; chacun de A et B désigne indépendamment un substituant ayant un atome d'oxygène ou un atome d'azote ; quand plusieurs $R^1$ , $R^2$, A et B existent pour chaque dans la structure X, ceux-ci peuvent être identiques ou différents ; et

dans lequel Y désigne un groupe hydrophile ayant un caractère hydrophile, le groupe contenant un atome choisi parmi un atome d'hydrogène, un atome de carbone, un atome d'oxygène, un atome d'azote, un atome de phosphore ou un atome de soufre ; et quand plusieurs Y existent, ces groupes peuvent être identiques ou différents l'un de l'autre,

dans lequel le composé est une silicone à modification polyoxyalkylène, et dans lequel le nombre de moles d'adduits polyoxyalkylène de la silicone à modification polyoxyalkylène est de 1 ou plus et 10 ou moins.

2. Agent de clivage de film liquide selon la revendication 1, comprenant un composé qui est composé d'une chaîne siloxane dans laquelle les structures représentées par n'importe lesquelles des formules (1) à (11) suivantes sont combinées arbitrairement :

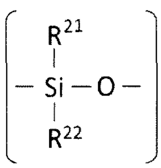 ••• (1)

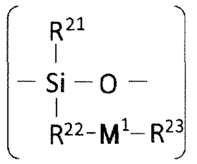 ••• (5)

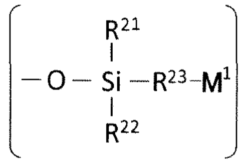 ••• (9)

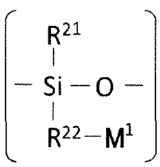 ••• (2)

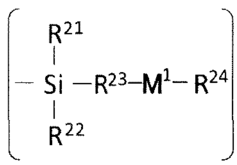 ••• (6)

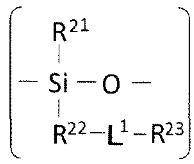 ••• (10)

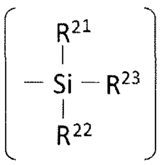 ••• (3)

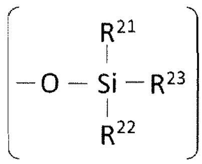 ••• (7)

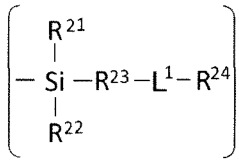 ••• (11)

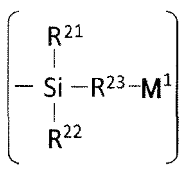 ••• (4)

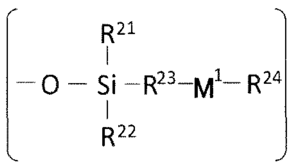 ••• (8)

où, dans les formules (1) à (11), $M^1$, $L^1$, $R^{21}$ et $R^{22}$ désigne le groupe monovalent ou polyvalent (divalent ou de valence supérieure) qui suit ; $R^{23}$ et $R^{24}$ désignent le groupe monovalent ou polyvalent (divalent ou de valence supérieure) qui suit ou une liaison simple ;

$M^1$ désigne un groupe polyoxyéthylène, un groupe polyoxypropylène, un groupe polyoxybutylène, un groupe ayant un groupe polyoxyalkylène en combinaison avec celui-ci, un groupe érythritol, un groupe xylitol, un groupe sorbitol, un groupe glycérol ou un groupe éthylèneglycol, un groupe hydroxyle, un groupe acide carboxylique, un groupe mercapto, un groupe alcoxy, un groupe amino, un groupe amide, un groupe imino, un groupe phénol, un groupe acide sulfonique, un groupe ammonium quaternaire, un groupe sulfobétaïne, un groupe hydroxysulfobétaïne, un groupe phosphobétaïne, un groupe imidazolium-bétaïne, un groupe carbobétaïne, un groupe époxy, un groupe carbinol, un groupe (méth)acrylique ou un groupe fonctionnel en combinaison avec celui-ci ; quand $M^1$ est un groupe polyvalent, $M^1$ désigne un groupe formé par élimination en outre d'un ou plusieurs atomes d'hydrogène sur chacun des groupes ou le groupe fonctionnel ;

$L^1$ désigne un groupe de liaison d'un groupe éther, d'un groupe amino (le groupe amino utilisable en tant que $L^1$ est représenté par >$NR^C$ ($R^C$ est un atome d'hydrogène ou un groupe monovalent)), d'un groupe amide, d'un groupe ester, d'un groupe carbonyle ou d'un groupe carbonate ; et

chacun de $R^{21}$, $R^{22}$, $R^{23}$ et $R^{24}$ désigne indépendamment un groupe alkyle, un groupe alcoxy, un groupe aryle, un groupe fluoroalkyle, un groupe aralkyle, un groupe hydrocarboné en combinaison avec ceux-ci, ou un atome d'halogène.

**3.** Agent de clivage de film liquide selon la revendication 1 ou 2, dans lequel la silicone à modification polyoxyalkylène est représentée par l'une quelconque des formules [I] à [IV] :

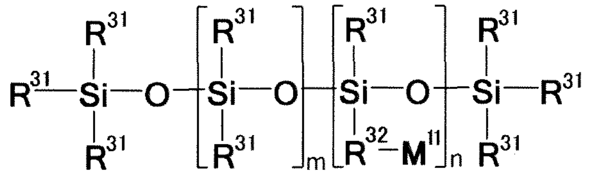
[I]

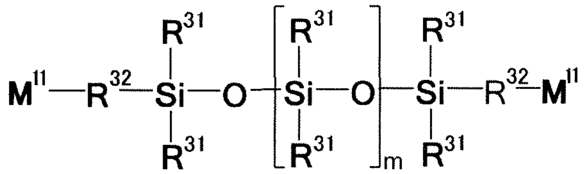
[II]

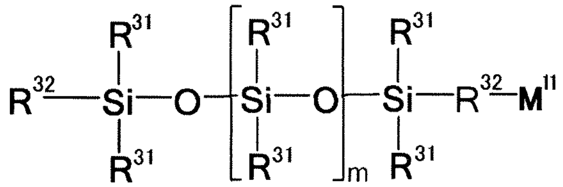
[III]

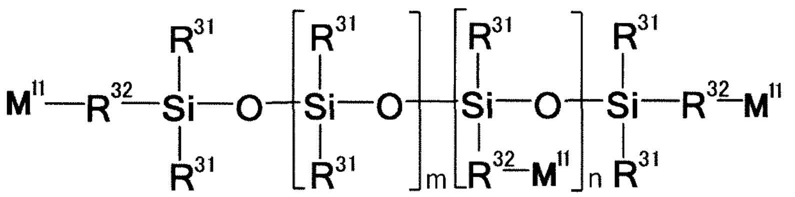
[IV]

dans lesquelles $R^{31}$ désigne un groupe alkyle ; $R^{32}$ désigne une liaison simple ou un groupe alkylène ; plusieurs $R^{31}$ et plusieurs $R^{32}$ peuvent pour chacun être identiques ou différents ; $M^{11}$ désigne un groupe ayant un groupe polyoxyalkylène ; en tant que groupe polyoxyalkylène, sont utilisés un groupe polyoxyéthylène, un groupe polyoxypropylène, un groupe polyoxybutylène et un matériau dans lequel des monomères constitutifs de celui-ci sont copolymérisés ; et chacun de m et n est indépendamment un entier valant 1 ou plus.

**4.** Agent de clivage de film liquide ayant un coefficient d'étalement supérieur à 0 mN/m vis-à-vis d'un liquide ayant une tension de surface de 50 mN/m, une solubilité dans l'eau de 0 g ou plus et de 0,025 g ou moins, et une tension interfaciale de 20 mN/m ou moins vis-à-vis du liquide ayant une tension de surface de 50 mN/m, mesurés conformément aux méthodes divulguées dans la description, et comprenant un composé ayant au moins un type de structure choisi dans le groupe constitué par les structures suivantes Z, Z-Y et Y-Z-Y :

dans lequel la structure Z désigne une chaîne hydrocarbonée ayant une structure dans laquelle n'importe laquelle des structures basiques de >C(A)- (C: atome de carbone), $-C(A)_2-$, -C(A)(B)-, >C(A)-$C(R^3)$<, >$C(R^3)$-, $-C(R^3)(R^4)-$, $-C(R^3)_2-$ et >C< est répétée, ou deux ou plus de deux types de celles-ci sont combinés ; la structure Z a, à une extrémité de celle-ci, un atome d'hydrogène ou au moins un type de groupe choisi dans le groupe constitué par $-C(A)_3$, $-C(A)_2B$, $-C(A)(B)_2$, $-C(A)_2-C(R^3)_3$, $-C(R^3)_2A$ et $-C(R^3)_3$ ;

chacun de $R^3$ et $R^4$ désigne indépendamment un atome d'hydrogène, un groupe alkyle, un groupe alcoxy, un

groupe aryle, un groupe fluoroalkyle, ou un groupe aralkyle, ou un groupe hydrocarboné en combinaison avec ceux-ci, ou un atome de fluor ; chacun de A et B désigne indépendamment un substituant ayant un atome d'oxygène ou un atome d'azote ;
Y désigne un groupe hydrophile ayant un caractère hydrophile, le groupe hydrophile contenant un atome choisi parmi un atome d'hydrogène, un atome de carbone, un atome d'oxygène, un atome d'azote, un atome de phosphore et un atome de soufre ; et quand plusieurs Y existent, ces plusieurs groupes peuvent être identiques ou différents les uns des autres.

**5.** Agent de clivage de film liquide selon la revendication 4, comprenant un alkyléther polyoxyalkyléné, de préférence un alkyléther polyoxyalkyléné (POA) représenté par l'une quelconque des formules dans la formule [V] ; ou l'un quelconque parmi un polyoxyalkylèneglycol, un Steareth, un Beheneth, un éther myristylique de PPG, un éther stéarylique de PPG et un éther béhénylique de PPG, qui sont représentés par l'une quelconque des formules dans la formule [VI] et ont une masse moléculaire moyenne en poids de 1 000 ou plus :

$$C_mH_n-L^{21}-(C_aH_bO)_m-L^{21}-R^{51}$$

ou

$$C_mH_n-L^{21}-(C_aH_bO)_m-R^{51} \qquad [V]$$

ou

$$C_mH_n-L^{21}-(C_aH_bO)_m-H$$

$$R^{51}-(C_aH_bO)_m-R^{51} \qquad [VI]$$

dans lesquelles $L^{21}$ désigne un groupe éther, un groupe amino, un groupe amide, un groupe ester, un groupe carbonyle, un groupe carbonate, un groupe polyoxyéthylène, un groupe polyoxypropylène, un groupe polyoxybutylène, ou un groupe polyoxyalkylène en combinaison avec ceux-ci ; $R^{51}$ désigne un substitut d'un atome d'hydrogène, un groupe méthyle, un groupe éthyle, un groupe propyle, un groupe isopropyle, un groupe butyle, un groupe pentyle, un groupe hexyle, un groupe heptyle, un groupe 2-éthylhexyle, un groupe nonyle, un groupe décyle, un groupe méthoxy, un groupe éthoxy, un groupe phényle, un groupe fluoroalkyle, un groupe aralkyle, un groupe hydrocarboné en combinaison avec ceux-ci, ou un atome de fluor ; chacun de a, b, m et n est indépendamment un entier valant 1 ou plus ; $C_mH_n$ désigne ici un groupe alkyle (n = 2m+1), et $C_aH_b$ désigne un groupe alkylène (a = 2b) ; le nombre d'atomes de carbone et le nombre d'atomes d'hydrogène sont chacun indépendamment déterminés dans chacune des formules (V) et (VI), et ne représentent pas toujours un entier identique, et ils peuvent être différents ; "m" dans $-(C_aH_bO)_m-$ est un entier valant 1 ou plus ; et les valeurs des motifs répétitifs sont chacune indépendamment déterminées dans chacune des formules (V) et (VI), et ne représentent pas toujours un entier identique, et elles peuvent être différentes ; ou un composé hydrocarboné ayant 5 atomes de carbone ou plus, et de préférence 100 atomes de carbone ou moins, mieux encore 50 ou moins, lequel composé hydrocarboné est de préférence représenté par l'une quelconque des formules [VII] à [XV] :

$C_nH_n$-COOH [VII]

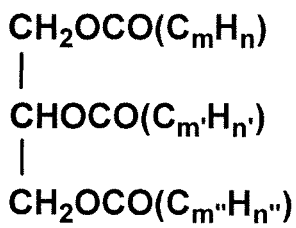

[VIII-I]

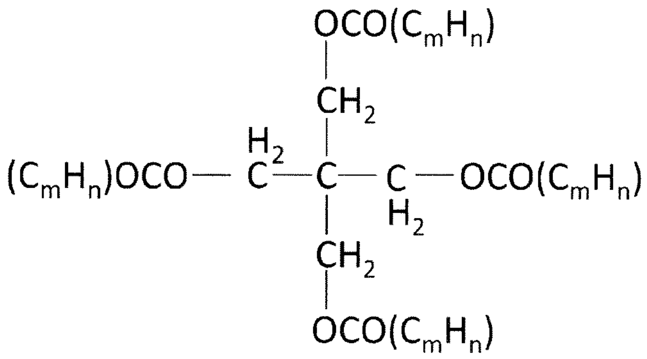

[VIII-II]

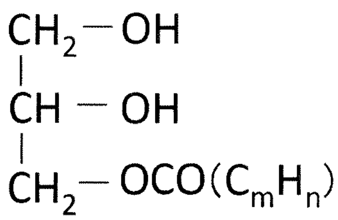

ou

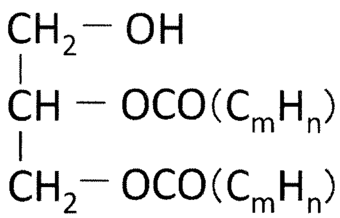

[IX]

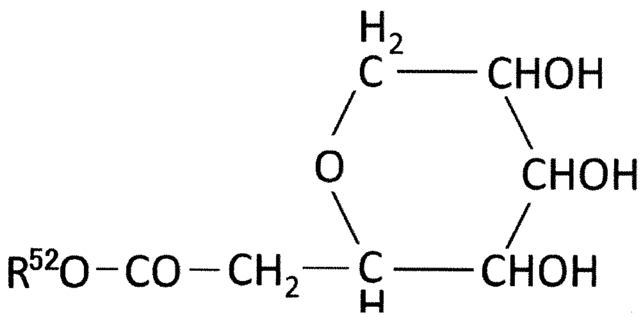

ou

[X]

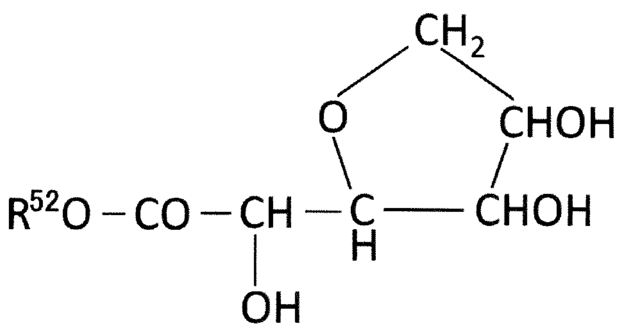

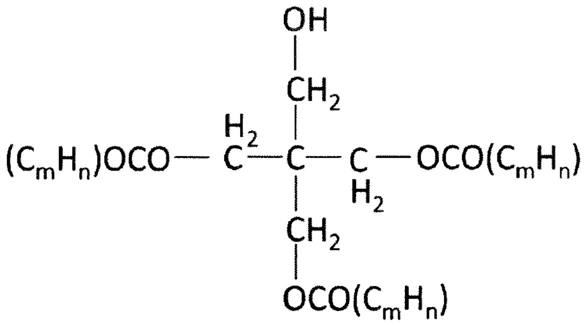

[XI]

ou

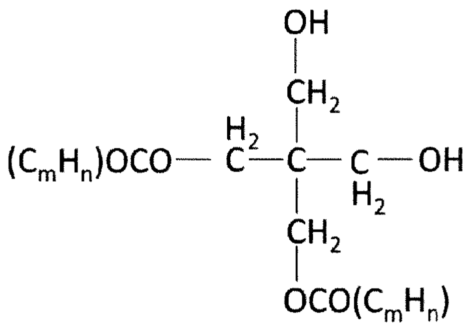

ou

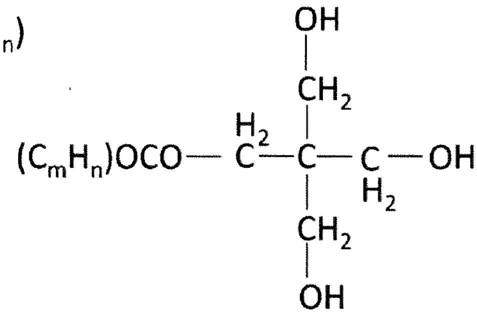

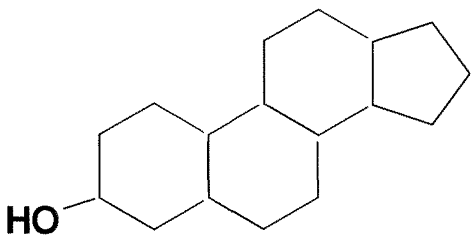

[XII]

$C_mH_n$-OH [XIII]

$C_mH_n$-COO-$C_mH_n$ [XIV]

$C_mH_n$ [XV]

où, dans les formules [VII] à [XV], chacun de m, m', m", n, n' et n" est indépendamment un entier valant 1 ou plus ; plusieurs m ou plusieurs n peuvent pour chaque cas être identiques ou différents ; et, dans la formule [X], $R^{52}$ désigne un groupe hydrocarboné à chaîne linéaire ou ramifiée, ou saturé ou insaturé, ayant 2 atomes de carbone ou plus et 22 atomes de carbone ou moins.

**6.** Agent de clivage de film liquide selon l'une quelconque des revendications 1 à 5, qui a une masse moléculaire moyenne en poids de 500 ou plus, mieux encore de 1 000 ou plus, plus particulièrement de 1 500 ou plus, et tout spécialement de 2 000 ou plus ; et de préférence de 50 000 ou moins, mieux encore de 20 000 ou moins, et plus particulièrement de 10 000 ou moins.

**7.** Agent de traitement de fibres contenant l'agent de clivage de film liquide de l'une quelconque des revendications

1 à 6 en une quantité de 5 % en masse ou plus et de 50 % en masse ou moins, et éventuellement un tensioactif anionique de type ester d'acide phosphorique.

**8.** Etoffe non tissée contenant l'agent de clivage de film liquide selon l'une quelconque des revendications 1 à 6 et éventuellement un tensioactif anionique de type ester d'acide phosphorique.

**9.** Etoffe non tissée selon la revendication 8, dans lequel le rapport de la teneur en agent de clivage de film liquide par rapport au tensioactif anionique de type ester d'acide phosphorique est de préférence de (1/1) à (19/1), mieux encore de (2/1) à (15/1), et plus particulièrement de (3/1) à (10/1), exprimé en poids.

**10.** Feuille supérieure pour un article absorbant dans lequel l'étoffe non tissée de la revendication 8 ou 9 est utilisée, dans laquelle
la feuille supérieure a au moins deux couches ;
la feuille supérieure a une pluralité de parties de collage concaves dans lesquelles les couches sont collées entre elles par compression depuis un côté de surface de contact avec la peau dans la direction de l'épaisseur ;
une couche sur un côté de surface sans contact avec la peau de la feuille supérieure est une couche façonnée dans laquelle des fibres thermiquement rétractables sont thermiquement rétractées ; et
une couche sur le côté de surface de contact avec la peau de la feuille supérieure a des fibres non thermiquement rétractables partiellement collées au niveau de la partie de collage, et a des parties convexes faisant saillie sur le côté de surface de contact avec la peau dans une région parmi les parties de collage concaves pour former une surface concavo-convexe de l'étoffe non tissée, ou
dans laquelle la feuille supérieure a une structure double couche ayant une partie creuse et formée d'une première étoffe non tissée sur un côté de surface de contact avec la peau et une deuxième étoffe non tissée sur un côté de surface sans contact avec la peau, et les deux couches contiennent des fibres thermoplastiques ; et
la feuille supérieure a une partie de collage dans laquelle la première étoffe non tissée et la deuxième étoffe non tissée sont partiellement collées par fusion thermique, et, dans une partie sans collage entourée par les parties de collage, la première étoffe non tissée a un grand nombre de parties convexes faisant saillie dans une direction éloignée de la deuxième étoffe non tissée avec les parties creuses à l'intérieur de celle-ci, et la partie de collage est une partie concave positionnée entre les parties convexes adjacentes et constitue une forme concavo-convexe sur le côté de surface en contact avec la peau en coopération avec les parties convexes, ou
dans laquelle la feuille supérieure comprend une couche contenant des fibres thermoplastiques et a une forme concavo-convexe pour les deux côtés ;
une première partie saillante faisant saillie sur un côté d'une première surface et une deuxième partie saillante faisant saillie sur un côté d'une deuxième surface sont disposées en continu et en alternance dans des directions différentes qui se croisent en vue en plan de l'étoffe non tissée ; et
la première partie saillante et la deuxième partie saillante ont chacune un espace interne ouvert sur un côté de chaque surface opposée, qui forme des parties concaves sur chaque surface, ou
dans laquelle la feuille supérieure comprend deux couches contenant des fibres thermoplastiques ;
une première partie saillante faisant saillie sur un côté d'une première surface et une deuxième partie saillante faisant saillie sur un côté d'une deuxième surface sont disposées en continu et en alternance dans des directions différentes qui se croisent en vue en plan de l'étoffe non tissée, et la première partie saillante et la deuxième partie saillante ont chacune un espace interne ouvert sur un côté de chaque surface opposée ; et
une première couche de fibres a une partie concave formée dans l'espace interne sur chaque surface, et une deuxième couche de fibres est disposée le long d'une forme concavo-convexe sur le côté de la deuxième surface de la première couche de fibres et collée avec la première couche de fibres, et un côté de la première couche de fibres est appliqué en tant que côté de surface en contact avec la peau, ou
dans laquelle la feuille supérieure comprend une seule couche contenant des fibres thermoplastiques, et a une forme dans laquelle une pluralité de parties convexes hémicyclindriques et une pluralité de parties concaves disposées le long de bords latéraux des parties convexes sont disposées en alternance sur un côté de surface en contact avec la peau ; et
des parties de fond concaves comprenant des fibres de l'étoffe non tissée sont disposées sur un côté inférieur des parties concaves, et la densité de fibres des parties de fond concaves est inférieure à la densité de fibres des parties convexes.

**11.** Article absorbant employant l'étoffe non tissée de la revendication 8 ou 9, ou contenant la feuille supérieure de la revendication 10.

**12.** Méthode de production de l'étoffe non tissée de la revendication 8 ou 9, comprenant une étape d'immersion, dans

une solution contenant un agent de clivage de film liquide, une étoffe non tissée servant de matière première préparée par fusion thermique de fibres entre elles par traitement par air traversant ou emboutissage à la chaleur, ou une étape d'enduction d'un agent de clivage de film liquide seul ou d'une solution contenant l'agent de clivage de film liquide sur une étoffe non tissée servant de matière première préparée par fusion thermique de fibres entre elles par traitement par air traversant ou emboutissage à la chaleur.

**13.** Utilisation d'un composé ayant un coefficient d'étalement de 15 mN/m ou plus vis-à-vis d'un liquide ayant une tension de surface de 50 mN/m, et une solubilité dans l'eau de 0 g ou plus et de 0,025 g ou moins, mesurés conformément aux méthodes divulguées dans la description, en tant qu'agent de clivage de film liquide, ou ayant un coefficient d'étalement supérieur à 0 mN/m ou plus vis-à-vis d'un liquide ayant une tension de surface de 50 mN/m, une solubilité dans l'eau de 0 g ou plus et de 0,025 g ou moins, et une tension interfaciale de 20 mN/m vis-à-vis du liquide ayant une tension de surface de 50 mN/m, mesurés conformément aux méthodes divulguées dans la description, en tant qu'agent de clivage de film liquide.

{FIG. 1}

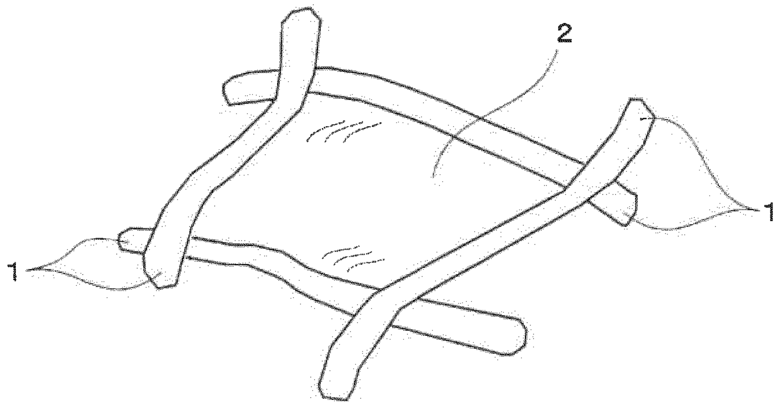


{FIG. 2}

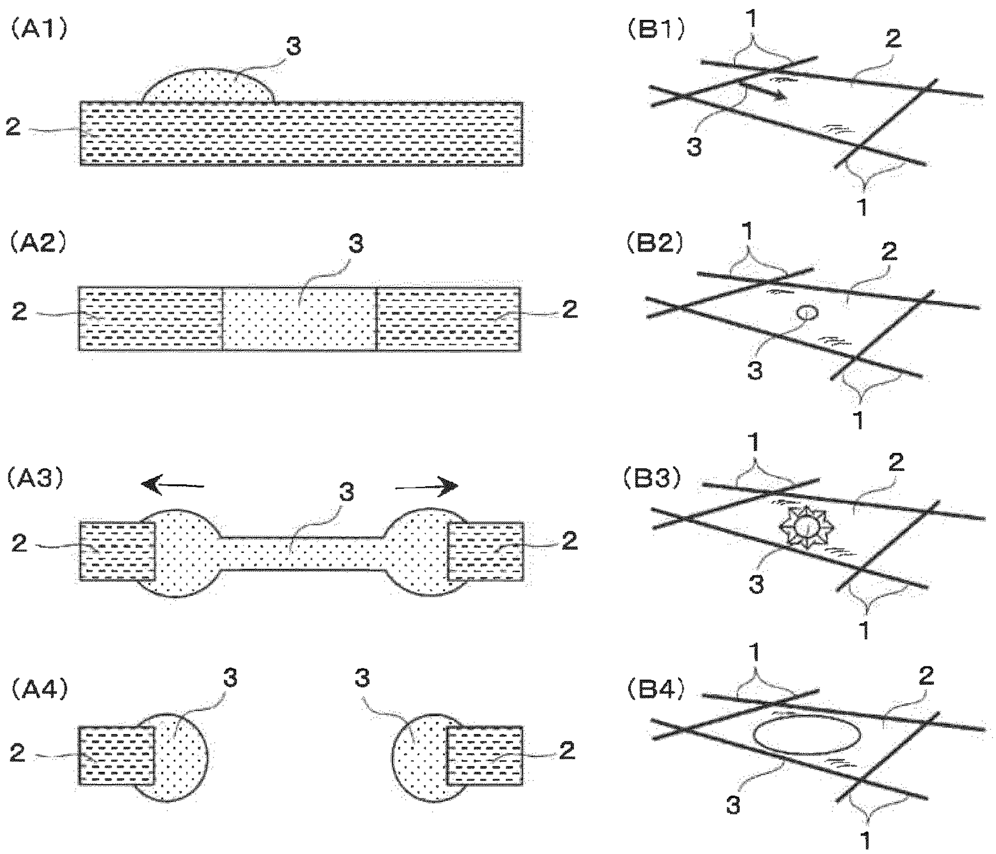

{FIG. 3}

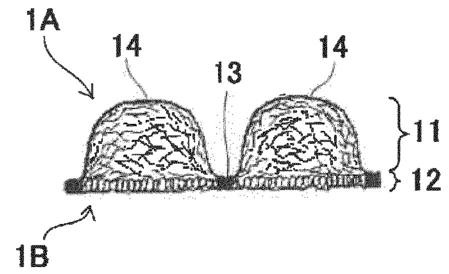


{FIG. 4}

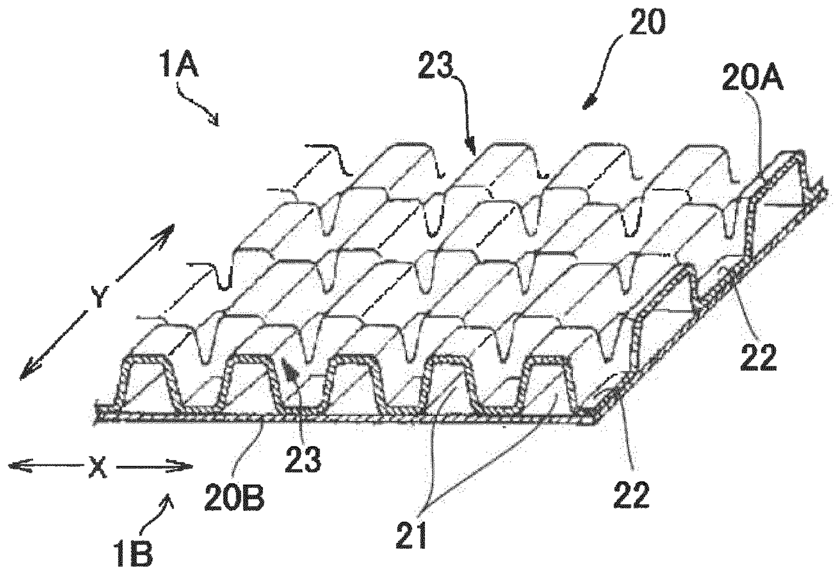

{FIG. 5}

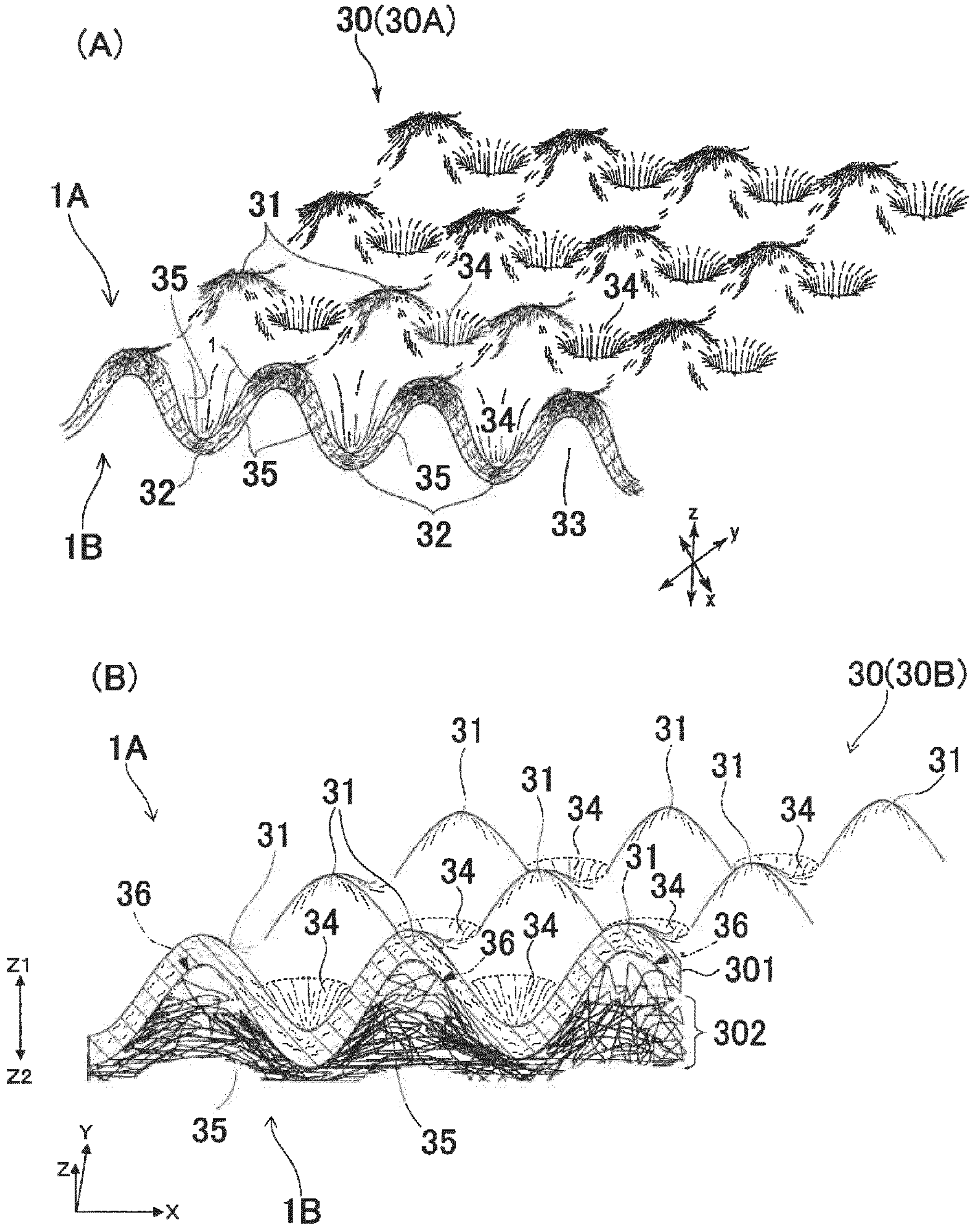
(A)
30(30A)
1A
31
35
34
34
1
34
32
35
35
33
32
1B
z
y
x
(B)
30(30B)
1A
31
31
31
31
31
31
31
34
34
34
34
31
34
36
34
36
34
36
301
302
Z1
Z2
35
35
Y
Z
X
1B

{FIG. 6}

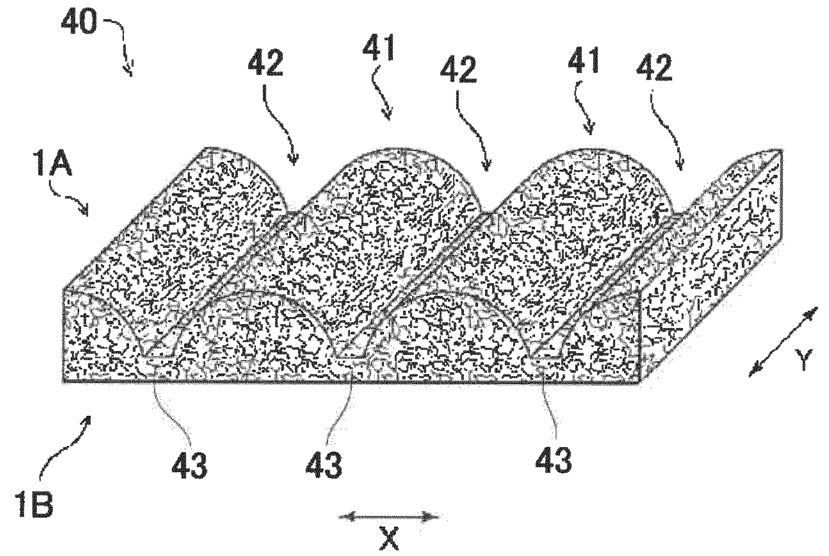
40
42
41
42
41
42
1A
Y
43
43
43
1B
X

{FIG. 7}

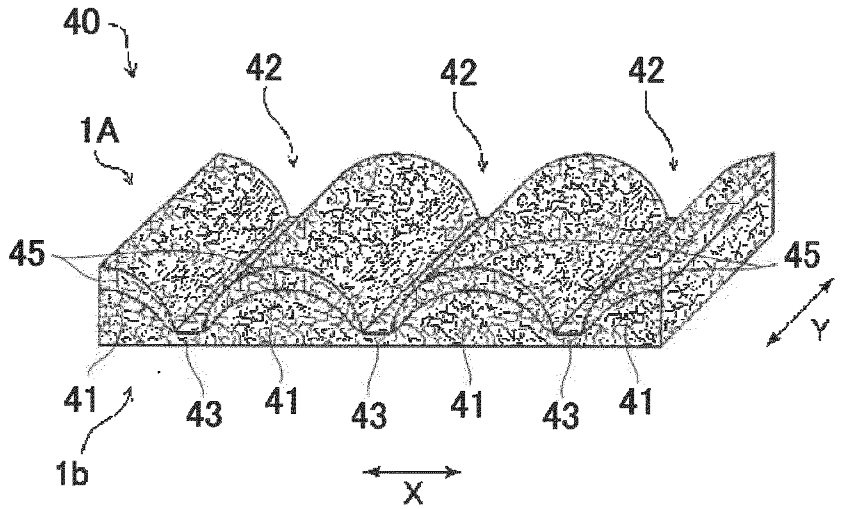
40
42
42
42
1A
45
45
Y
41
43
41
43
41
43
41
1b
X

{FIG. 8}

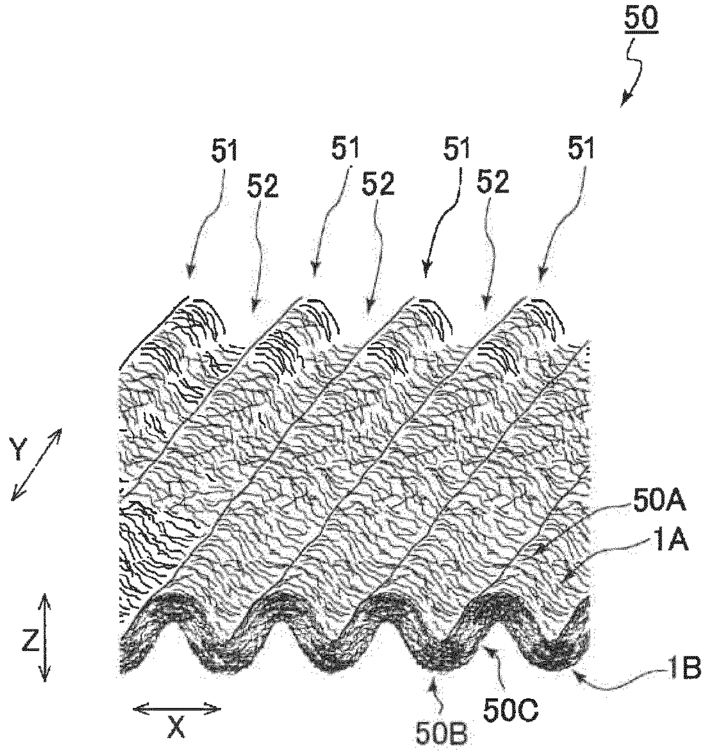
50
51
52
51
52
51
52
51
Y
50A
1A
Z
1B
X
50B
50C

{FIG. 9}

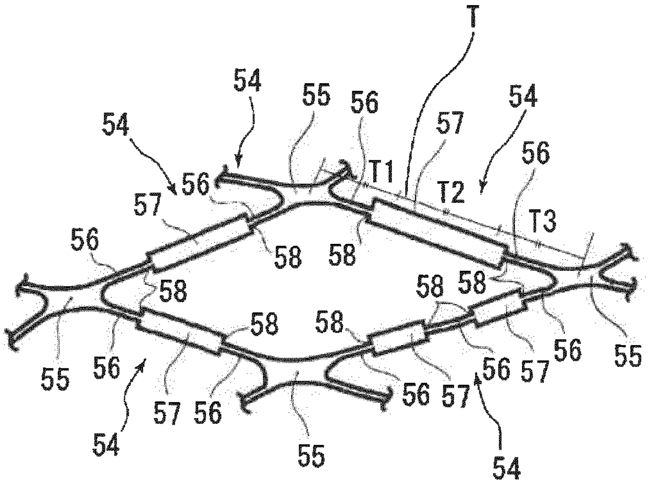
T
54
55
56
57
54
56
54
56
T1
T2
57
T3
58
58
56
58
58
58
58
58
58
56
55
56
57
56
55
55
56
57
56
56
57
54
55
54

{FIG. 10}

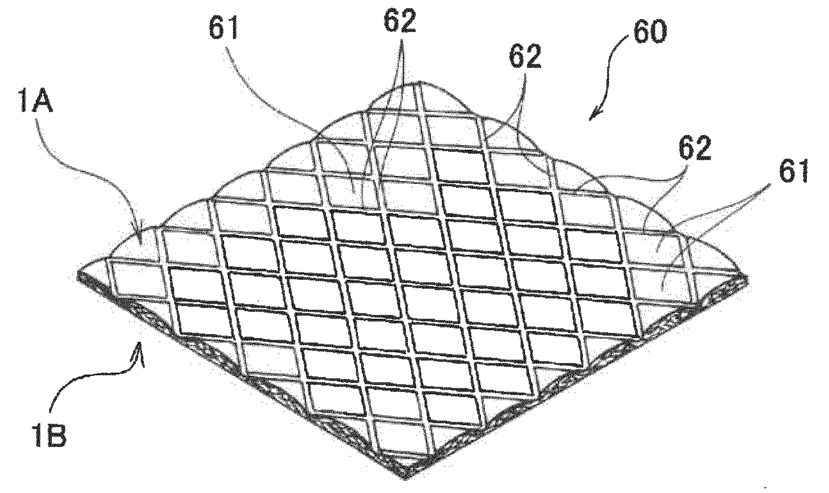


{FIG. 11}

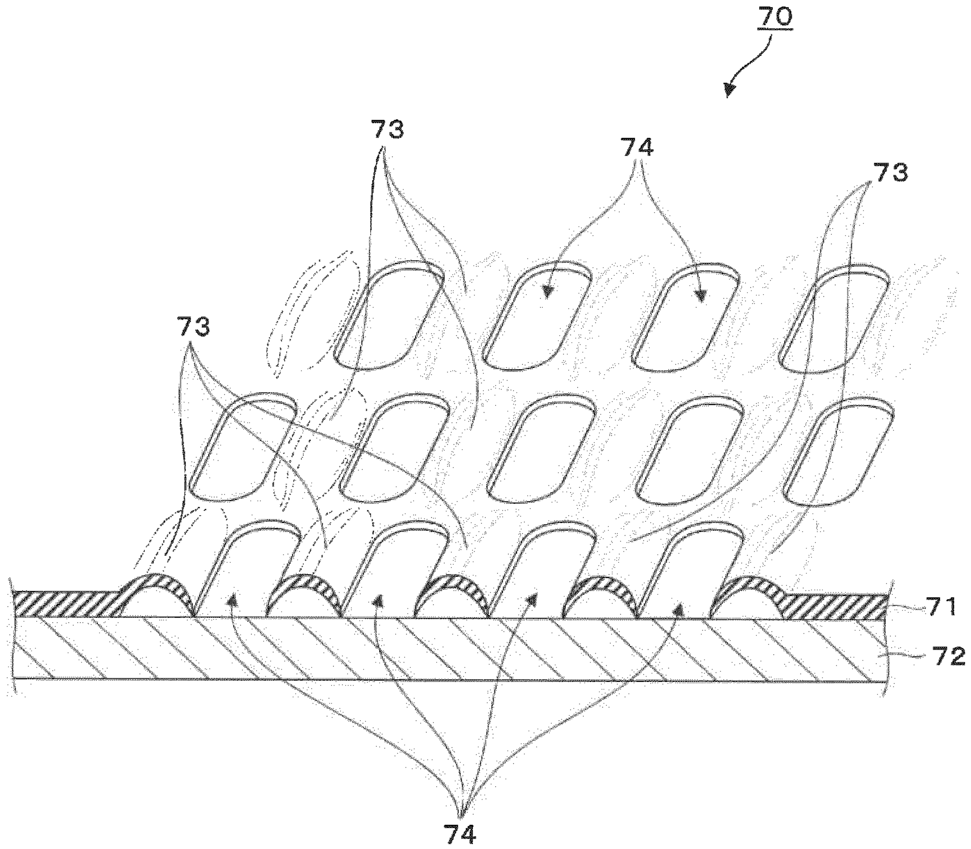

{FIG. 12}

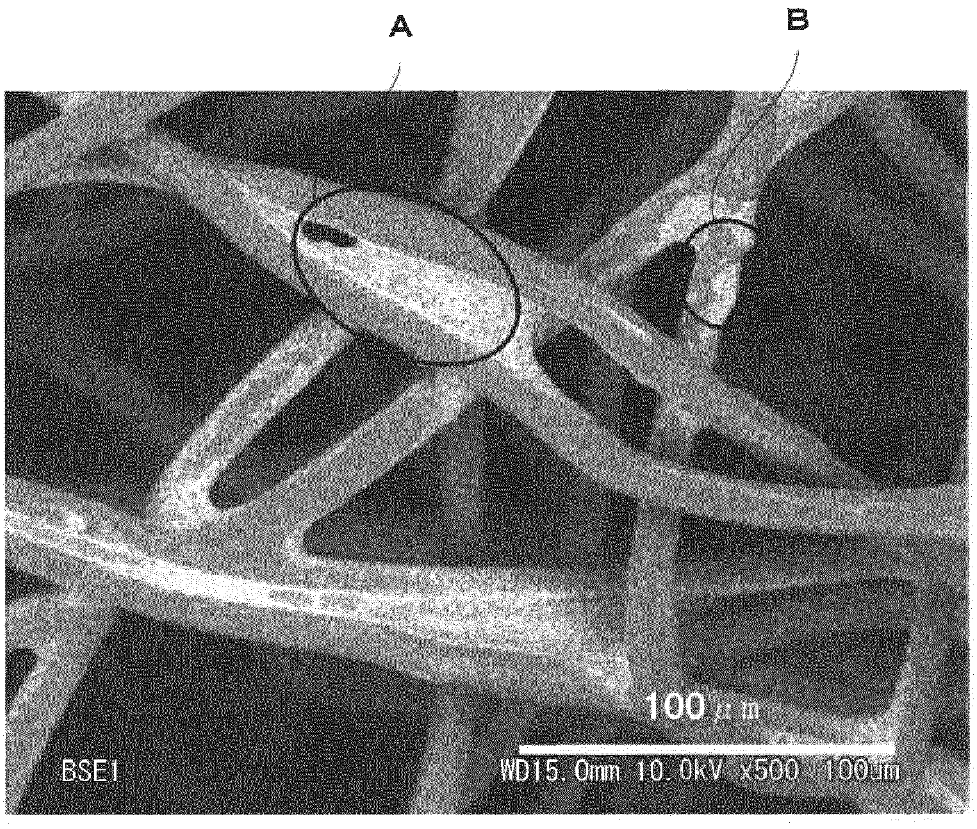

{FIG. 13}

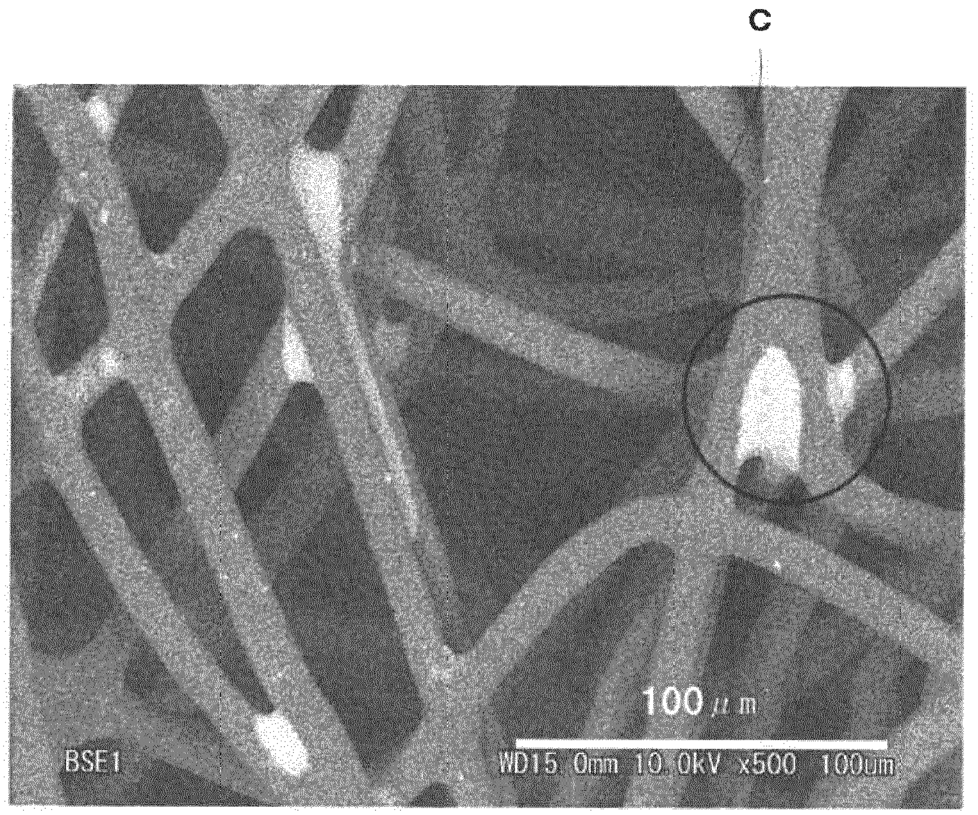

{FIG. 14}

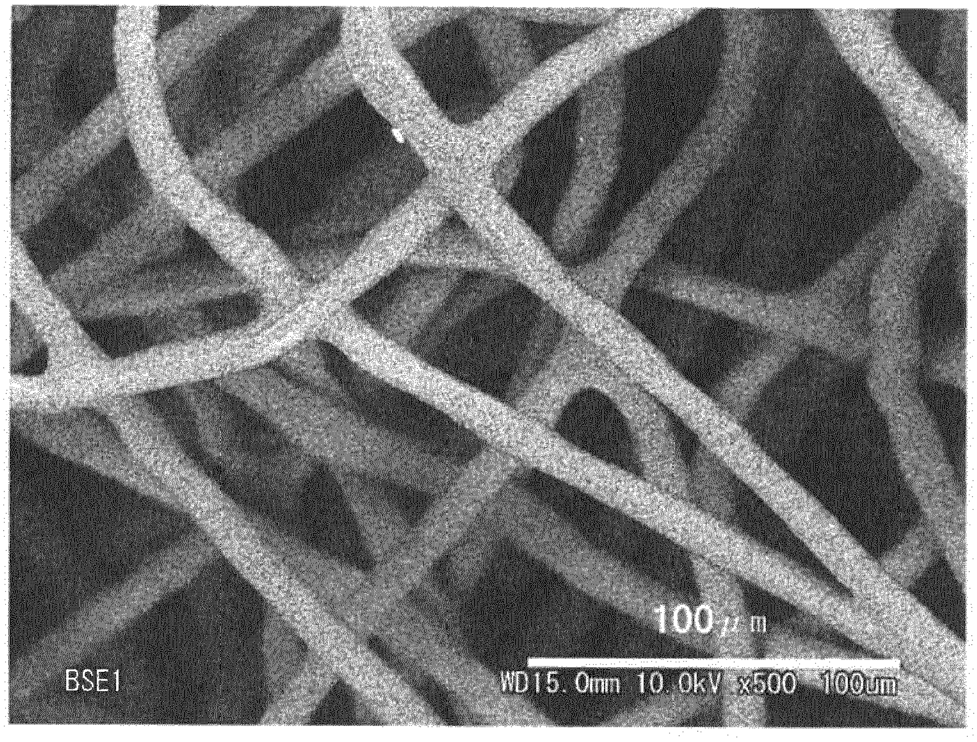

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004256935 A
- JP 2013179969 A
- JP 2014068942 A
- JP H01148879 A
- JP H09215706 A
- JP H1053955 A
- JP 2002161474 A
- JP 2002187228 A
- JP 2012 A
- JP 136790 A
- JP 2012149370 A
- JP 2012149371 A
- JP 2013124428 A
- JP 2010168715 A
- JP 2005350836 A
- JP 20111277258 A
- JP H424263 A